(19)

Europäisches Patentamt

European Patent Office

Office européen des brevets

(11) **EP 4 546 346 A1**

(12) **EUROPEAN PATENT APPLICATION**
published in accordance with Art. 153(4) EPC

(43) Date of publication:
**30.04.2025 Bulletin 2025/18**

(21) Application number: **23827299.1**

(22) Date of filing: **23.06.2023**

(51) International Patent Classification (IPC):
**G16B 15/00** (2019.01)      **G06N 99/00** (2019.01)

(52) Cooperative Patent Classification (CPC):
**G06N 99/00; G16B 15/00**

(86) International application number:
**PCT/JP2023/023405**

(87) International publication number:
**WO 2023/249117 (28.12.2023 Gazette 2023/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.06.2022   JP 2022102155**

(71) Applicants:
• **Toshiba Digital Solutions Corporation**
  **Kawasaki-shi, Kanagawa 212-0013 (JP)**
• **Revorf Co., Ltd.**
  **Tokyo 103-0027 (JP)**

(72) Inventors:
• **KIMURA, Keiichi**
  **Tokyo 103-0027 (JP)**
• **IBUKI, Masato**
  **Tokyo 103-0027 (JP)**
• **AKIYAMA, Yutaka**
  **Kawasaki-shi, Kanagawa 210-0007 (JP)**
• **TAKABATAKE, Kazuki**
  **Kawasaki-shi, Kanagawa 212-0013 (JP)**
• **IZUMI, Yasuichiro**
  **Kawasaki-shi, Kanagawa 212-0013 (JP)**
• **IWASAKI, Motokazu**
  **Kawasaki-shi, Kanagawa 212-0013 (JP)**

(74) Representative: **Marks & Clerk LLP**
  **15 Fetter Lane**
  **London EC4A 1BW (GB)**

(54) **ALLOSTERIC PATH PREDICTION DEVICE, ALLOSTERIC PATH PREDICTION RESULT ACQUISITION METHOD, ALLOSTERIC PATH PREDICTION METHOD, AND PROGRAM**

(57)     An allosteric path prediction device includes a network graph generating unit and a path calculating unit. The network graph generating unit generates, on the basis of three-dimensional structure information of a protein, a network graph which includes vertices corresponding to at least amino acid residues constituting the protein out of the amino acid residues and arbitrary binding substances bound to the protein and in which weights based on interactions between at least the amino acid residues out of the amino acid residues and the arbitrary binding substances are assigned to edges on the basis of three-dimensional structure information of the protein. The path calculating unit calculates a path connecting the vertices on the network graph generated by the network graph generating unit on the basis of an evaluation function based on the weights.

*FIG. 6*

EP 4 546 346 A1

**Description**

TECHNICAL FIELD

The present invention relates to an allosteric path prediction device, an allosteric path prediction result acquisition method, an allosteric path prediction method, and a program.
Priority is claimed on Japanese Patent Application No. 2022-102155, filed June 24, 2022, the content of which is incorporated herein by reference.

BACKGROUND ART

Recently, there has been a trend of research and development investment in the drug development market expanding, but the depletion of drug development targets has become evident. Therefore, it is expected to derive new drug development targets using IT technology. For example, in the related art, proteins in which it was difficult to develop a drug can be used as drug development targets through allosteric drug development. Allosteric drug development is an approach with an allosteric control mechanism of a protein as a target. Allosteric control means that a signal to an allosteric control site of a protein controls a structure, an activity, or a reaction of an active site. In allosteric drug development, an allosteric control site of a protein or a mechanism (an allosteric path) by which a signal to an allosteric control site is transmitted to an active site is used as a target.
Prediction of an allosteric control site or how a signal to the allosteric control site is transmitted to an active site (referred to as an allosteric path) is very important in realization of allosteric drug development.

Citation List

Patent Document

Patent Document 1: Chinese Patent No. 108830043

Non Patent Document

Non Patent Document 1: "Biophysical Journal," February 9, 2007, vol. 92, p. 3052-3062
Non Patent Document 2: "NATURE COMMUNICATIONS," July 31, 2020, vol. 11, p. 3862
Non Patent Document 3: "Scientific Reports," February 22, 2016, vol. 6, p. 21686

SUMMARY OF INVENTION

Technical Problem

A problem to be solved by the present invention is to provide an allosteric path prediction device, an allosteric path prediction result acquisition method, an allosteric path prediction method, and a program that can predict amino acid residues contributing to allosteric control from three-dimensional structure information of proteins.

Solution to Problem

An allosteric path prediction device according to an embodiment includes a network graph generating unit and a path calculating unit. The network graph generating unit generates, on the basis of three-dimensional structure information of a protein, a network graph which includes vertices corresponding to at least amino acid residues constituting the protein out of the amino acid residues and arbitrary binding substances bound to the protein and in which weights based on interactions between at least the amino acid residues out of the amino acid residues and the arbitrary binding substances are assigned to edges. The path calculating unit calculates a path connecting the vertices on the network graph generated by the network graph generating unit on the basis of an evaluation function based on the weights.

BRIEF DESCRIPTION OF DRAWINGS

[FIG. 1] A diagram illustrating an example of a three-dimensional structure of a protein according to a first embodiment of the present invention.

[FIG. 2] A diagram illustrating an example of a network graph representing a three-dimensional structure of a protein according to the first embodiment of the present invention.

[FIG. 3] A diagram illustrating an example of a path according to the first embodiment of the present invention.

[FIG. 4] A diagram illustrating a signal transmission probability according to the first embodiment of the present invention.

[FIG. 5] A diagram illustrating a signal transmission probability from a start point to an end point of a path according to the first embodiment of the present invention.

[FIG. 6] A diagram illustrating an example of the functional configuration of a allosteric path prediction device according to the first embodiment of the present invention.

[FIG. 7] A diagram illustrating an example of an allosteric path predicting process according to the first embodiment of the present invention.

[FIG. 8] A diagram illustrating an example of a network graph generating process according to the first embodiment of the present invention.

[FIG. 9] A diagram illustrating an Ising calculation process according to the first embodiment of the present invention.

[FIG. 10] A diagram illustrating oriented sides according to the first embodiment of the present invention.

[FIG. 11] A diagram illustrating an example of Hamiltonian of a single path according to the first embodiment of the present invention.

[FIG. 12] A diagram illustrating an example of calculation results of a path according to the first embodiment of the present invention.

[FIG. 13] A diagram illustrating an example of the outline of a method of searching for an amino acid residue contributing to allosteric control according to a second embodiment of the present invention.

[FIG. 14] A diagram illustrating an example of values of residue scores according to the second embodiment of the present invention.

[FIG. 15] A diagram illustrating an example of the functional configuration of a allosteric path prediction device according to the second embodiment of the present invention.

[FIG. 16] A diagram illustrating an example of an allosteric path predicting process according to the second embodiment of the present invention.

[FIG. 17] A diagram illustrating an example of an important amino acid residue search process according to the second embodiment of the present invention.

[FIG. 18] A diagram illustrating a network graph representing search results of an important I amino acid residue according to a first example of the second embodiment of the present invention.

[FIG. 19] A diagram illustrating the search results of an important amino acid residue according to the first example of the second embodiment of the present invention.

[FIG. 20] A diagram illustrating a network graph representing search results of an important amino acid residue according to a second example of the second embodiment of the present invention.

[FIG. 21] A diagram illustrating the search results of an important amino acid residue according to the second example of the second embodiment of the present invention.

[FIG. 22] A diagram illustrating a network graph representing search results of an important amino acid residue according to the second example of the second embodiment of the present invention.

[FIG. 23] A diagram illustrating the search results of an important amino acid residue according to the second example of the second embodiment of the present invention.

[FIG. 24] A diagram illustrating an example of the outline of an allosteric region search method according to a third embodiment of the present invention.

[FIG. 25] A diagram illustrating an example of evaluation results of an influence of each of a plurality of start points on an end point according to the third embodiment of the present invention.

[FIG. 26] A diagram illustrating an example of the functional configuration of a allosteric path prediction device according to the third embodiment of the present invention.

[FIG. 27] A diagram illustrating an example of an allosteric path predicting process according to the third embodiment of the present invention.

[FIG. 28] A diagram illustrating an Ising calculation process according to the third embodiment of the present invention.

[FIG. 29] A diagram illustrating an example of a region search process according to the third embodiment of the present invention.

[FIG. 30] A diagram illustrating search results of an allosteric control region with respect to amino acid regions of which the amino acid number of KRAS ranges from 1 to 67 according to an example of the third embodiment of the present invention.

[FIG. 31] A diagram illustrating search results of an allosteric control region with respect to amino acid regions of which the amino acid number of KRAS ranges from 114 to 169 according to an example of the third embodiment of the present invention.

[FIG. 32] A diagram illustrating search results of an allosteric control region with respect to the whole KRAS along with a position of an α helix chain according to an example of the third embodiment of the present invention.

[FIG. 33] A diagram illustrating an example of the outline of an allosteric region search method according to a modified example of the third embodiment of the present invention.

[FIG. 34] A diagram illustrating an example of the outline of an allosteric region search method according to a modified example of the third embodiment of the present invention.

[FIG. 35] A diagram illustrating an example of the functional configuration of a allosteric path prediction device according to a modified example of the third embodiment of the present invention.

[FIG. 36] A diagram illustrating an example of an allosteric path predicting process according to a modified example of the third embodiment of the present invention.

[FIG. 37] A diagram illustrating an example of a residue set search process according to a modified example of the third embodiment of the present invention.

[FIG. 38] A diagram illustrating an example of a residue set search and Ising calculation process according to a modified example of the third embodiment of the present invention.

[FIG. 39] A diagram illustrating an example of the outline of a residue set score calculating process according to a modified example of the third embodiment of the present invention.

[FIG. 40] A diagram illustrating an example of the distance between residues according to a modified example of the third embodiment of the present invention.

[FIG. 41] A diagram illustrating an example of the outline of search results of a residue set according to a modified example of the third embodiment of the present invention.

[FIG. 42] A diagram illustrating search results of an allosteric control region according to a modified example of the third embodiment of the present invention.

[FIG. 43] A diagram illustrating a ribbon model of a KRAS/RAF composite according to a modified example of the third embodiment of the present invention.

[FIG. 44] A diagram illustrating a ribbon model of a KRAS/RAF composite according to a modified example of the third embodiment of the present invention.

[FIG. 45] A diagram illustrating a ribbon model of a KRAS/RAF composite according to a modified example of the third embodiment of the present invention.

[FIG. 46] A diagram illustrating an example of a multiplex path according to a fourth embodiment of the present invention.

[FIG. 47] A diagram illustrating an example of the functional configuration of a allosteric path prediction device according to the fourth embodiment of the present invention.

[FIG. 48] A diagram illustrating an example of Hamiltonians for a multiplex path according to the fourth embodiment of the present invention.

[FIG. 49] A diagram illustrating search results of an important amino acid residue according to an example of the fourth embodiment of the present invention.

[FIG. 50] A diagram illustrating an example of an allosteric path which is calculated using Hamiltonians for a multiplex path according to an example of the fourth embodiment of the present invention.

[FIG. 51] A diagram illustrating search results of an allosteric control region according to a second example of the fourth embodiment of the present invention.

[FIG. 52] A diagram illustrating search results of an allosteric control region according to the second example of the fourth embodiment of the present invention.

[FIG. 53] A diagram illustrating an example of a configuration of an allosteric path prediction system according to a fifth embodiment of the present invention.

[FIG. 54] A diagram illustrating an example of an allosteric path predicting process according to the fifth embodiment of the present invention.

[FIG. 55] A diagram illustrating an example of a network graph generating process according to the fifth embodiment of the present invention.

[FIG. 56] A diagram illustrating an example of a QUBO and constraint expression list setting process according to the fifth embodiment of the present invention.

[FIG. 57] A diagram illustrating an example of a configuration of a system according to a first modified example of the fifth embodiment of the present invention.

[FIG. 58] A diagram illustrating an example of a configuration of a system according to a second modified example of the fifth embodiment of the present invention.

DESCRIPTION OF EMBODIMENTS

In this specification and the appended claims, a "protein" means a molecule having a polypeptide chain in which a plurality of amino acids are connected through peptide binding. The number of amino acids constituting a protein is not particularly limited, and may be equal to or less than 100, may be equal to or less than 1000, may be equal to or less than 2000, may be equal to or less than 3000, or may be equal to or less than 5000. The upper limit thereof is not particularly limited, and, for example, equal to or less than 5000 may be set as a criterion. Amino acids constituting a protein are not limited to natural amino acids, and may be non-natural amino acids which are artificially synthesized. Non-natural amino acids include amino acid residues which are arbitrarily chemically modified. An amino acid sequence of a protein is not limited to natural amino acid sequences, and may be non-natural amino acid sequences which are artificially designed. In general, proteins are roughly classified into water-soluble proteins and water-insoluble proteins. A protein in the present invention may be any protein and may be a water-insoluble membrane protein. A protein in the present invention has only to be a protein of which a three-dimensional structure can be acquired. The protein in the present invention may be a protein of which a three-dimensional structure is difficult to experimentally acquire but of which a three-dimensional structure can be predicted by a three-dimensional structure prediction system such as AlphaFold2. When a three-dimensional structure is experimentally acquired, it means that a three-dimensional structure can be acquired through X-ray structure analysis, analysis of imaging results using cryo-electron microscopy, NMR analysis, or the like.

(First embodiment)

Hereinafter, an embodiment of the present invention will be described in detail with reference to the accompanying drawings.

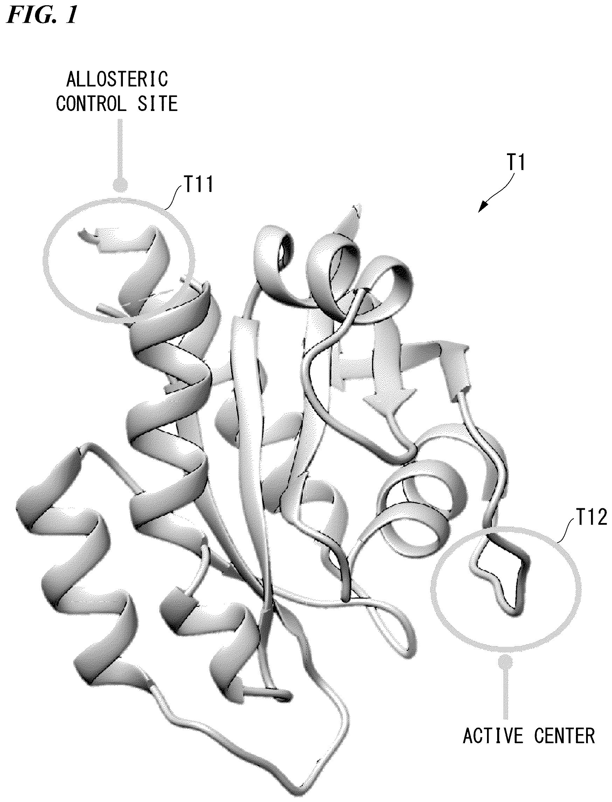

The outline of modeling of allosteric control in an allosteric path prediction method according to the present embodiment will be first described below with reference to FIGS. 1 to 3. In the present embodiment, allosteric control means that a signal to an allosteric control site of a protein controls one or more of a three-dimensional structure or reactivity (also referred to as activity) of an active site.

An example of positive control for making a reaction of interest is as follows. That is, in a state in which allosteric control has not been performed, an active site is not a three-dimensional structure that can be bound to a binding partner such as a substrate to be specifically bound to the active site or is not in a state suitable for a reaction. On the other hand, in a state in which allosteric control has been performed by applying exogenous allosteric control molecules such as chemicals to an allosteric control site which is far from an active site of a target protein, one or more of a three-dimensional structure and a reactivity of the active site change. The changed active site is bound to a binding partner such as a specific substrate and reacts therewith.

In an example of negative control for hindering a reaction of interest, one or more of a three-dimensional structure and a reactivity of an active site are changed to prevent binding or reaction which occurs normally by applying exogenous allosteric control molecules such as chemicals to an allosteric control site of a target protein.

A three-dimensional structure T1 illustrated in FIG. 1 is an example of a three-dimensional structure of a protein. The three-dimensional structure T1 includes an allosteric control site T11 and an active site T12. In allosteric control in the three-dimensional structure T1, a signal to the allosteric control site T11 changes one or more of a three-dimensional structure and a reactivity of the active site T12 which is located at a position far from the allosteric control site T11.

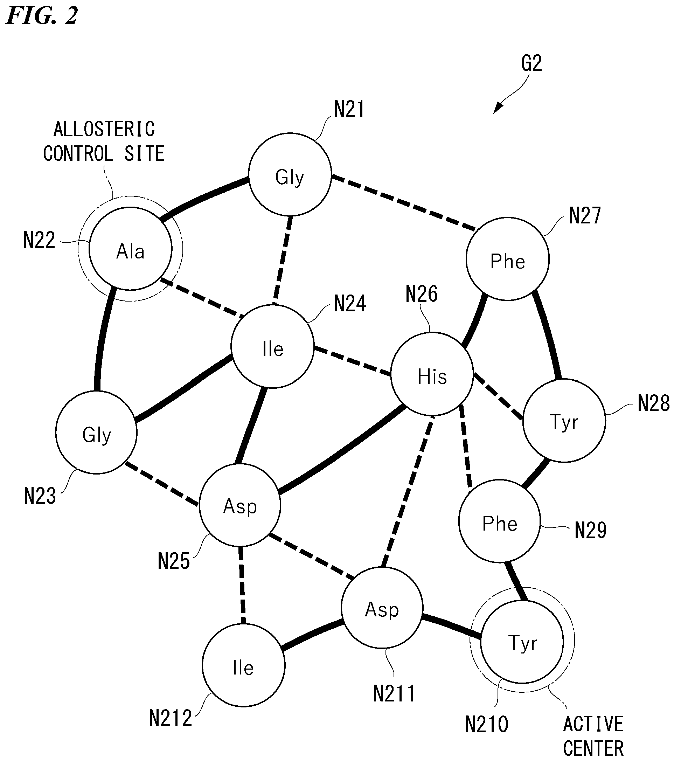

A network graph G2 illustrated in FIG. 2 is a network graph representing a three-dimensional structure of a protein. The network graph G2 includes a plurality of vertices and edges connecting the plurality of vertices. Each of the plurality of vertices corresponds to an amino acid residue. A vertex N22 corresponds to an allosteric control site. A vertex N210 corresponds to the active site T12. Weights based on interactions between the amino acid residues are assigned to the edges connecting the plurality of vertices. The edges connecting the plurality of vertices include edges representing a main chain and edges representing interactions between side chains. In FIG. 2, the edges representing a main chain are indicated by solid lines, and the edges representing an interaction between side chains are indicted by dotted lines.

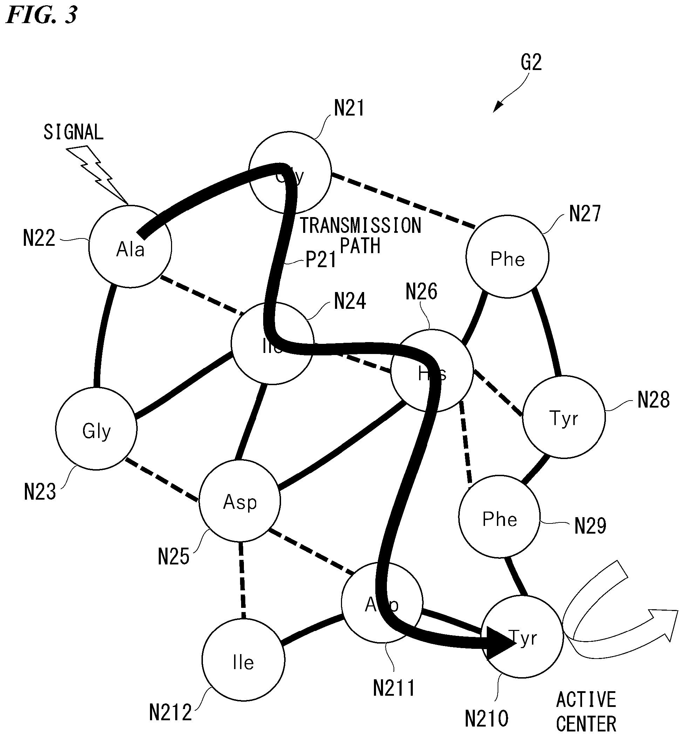

In an allosteric path prediction method according to the present embodiment, the allosteric control is modeled as signal transmission. The signal transmission is expressed as a path between two vertices in a network graph. This path is referred to as a signal transmission path or an allosteric path. Allosteric control in a three-dimensional structure of a protein is modeled as, for example, signal transmission from the vertex N22 to the vertex N210 using the network graph G2. A path P21 illustrated in FIG. 3 is a signal transmission path corresponding to signal transmission from the vertex N22 to the vertex N210.

In the allosteric path prediction method according to the present embodiment, a signal transmission path is predicted on the basis of an Ising model. In the allosteric path prediction method according to the present embodiment, a region important to allosteric control in a three-dimensional structure of a protein is identified on the basis of the predicted signal transmission path.

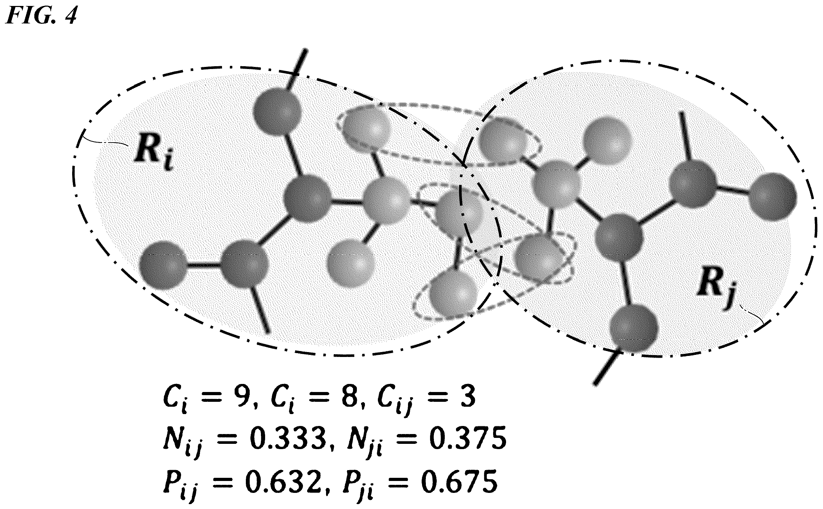

Weights which are assigned to edges connecting a plurality of vertices in a network graph and are based on interactions between amino acid residues will be described below with reference to FIG. 4. In the allosteric path prediction method according to the present embodiment, signal transmission probabilities between amino acid residues are used as the weights. FIG. 4 is a diagram illustrating a signal transmission probability according to the present embodiment. In FIG. 4, an i-th amino acid residue in a protein is defined as an amino acid residue $R_i$, and a j-th amino acid residue is defined as an amino acid residue $R_j$. The number of atoms $C_i$ constituting the amino acid residue $R_i$ is 9, and the number of atoms $C_j$ constituting the amino acid residue $R_j$ is 8.

A position vector indicating a position of an a-th atom constituting the amino acid residue $R_i$ is defined as a vector $r_a^i$. A position vector indicating a position of a b-th atom constituting the amino acid residue $R_j$ is defined as a vector $r_b^j$. The number of pairs in which a distance between two atoms in a pair of one atom constituting the amino acid residue $R_i$ and one atom constituting the amino acid residue $R_j$ is less than a cut-off value (also referred to as a threshold value) $r_0$ is defined as the number of pairs $C_{ij}$. The number of pairs $C_{ij}$ is expressed by Expression (1) using a heaviside step function H.
[Math. 1]

$$C_{ij} = \sum_{ab} H(r_0 - |r_a^i - r_b^j|) \qquad \cdots (1)$$

In the example illustrated in FIG. 4, the number of pairs $C_{ij}$ is 3.

In the present embodiment, in calculating the number of pairs $C_{ij}$, an example in which a distance between atoms in a side chain out of a main chain and a side chain is used as the distance between two atoms will be described below, but the present invention is not limited thereto. The distance between two atoms may be calculated to include an atom in the main chain along with an atom in the side chain.

A numerical value obtained by deviding the number of pairs $C_{ij}$ of one amino acid residue $R_i$ with respect to the other amino acid residue $R_j$ by the number of atoms $C_i$ constituting the one amino acid residue $R_i$ is defined as the number of contacts $N_{ij}$. In other words, the number of contacts $N_{ij}$ is the number of pairs $C_{ij}$ in which the size of one amino acid residue $R_i$ is considered. The number of contacts $N_{ij}$ is expressed by Expression (2).
[Math. 2]

$$N_{ij} = \frac{C_{ij}}{C_i} \qquad \cdots (2)$$

The number of contacts $N_{ij}$ can be considered as elements in i rows and j columns of a matrix, and the number of contacts $N_{ij}$ is also referred to as an interaction matrix.

A signal transmission probability $P_{ij}$ from the amino acid residue $R_i$ to the amino acid residue $R_j$ is expressed by Expression (3) using the number of contacts $N_{ij}$. Parameter $\alpha$ in Expression (3) is a constant.
[Math. 3]

$$P_{ij} = 1 - \exp(-\alpha \times N_{ij}) \qquad \cdots (3)$$

Accordingly, the signal transmission probability $P_{ij}$ from the amino acid residue $R_i$ to the amino acid residue $R_j$ becomes closer to 1 as the number of contacts $N_{ij}$ becomes larger, becomes closer to 0 as the number of contacts $N_{ij}$ becomes smaller, and is 0 particularly when the number of contacts $N_{ij}$ is 0. The signal transmission probability $P_{ij}$ can be considered as elements in i rows and j columns of a matrix, and the signal transmission probability $P_{ij}$ is also referred to as a transmission probability matrix.

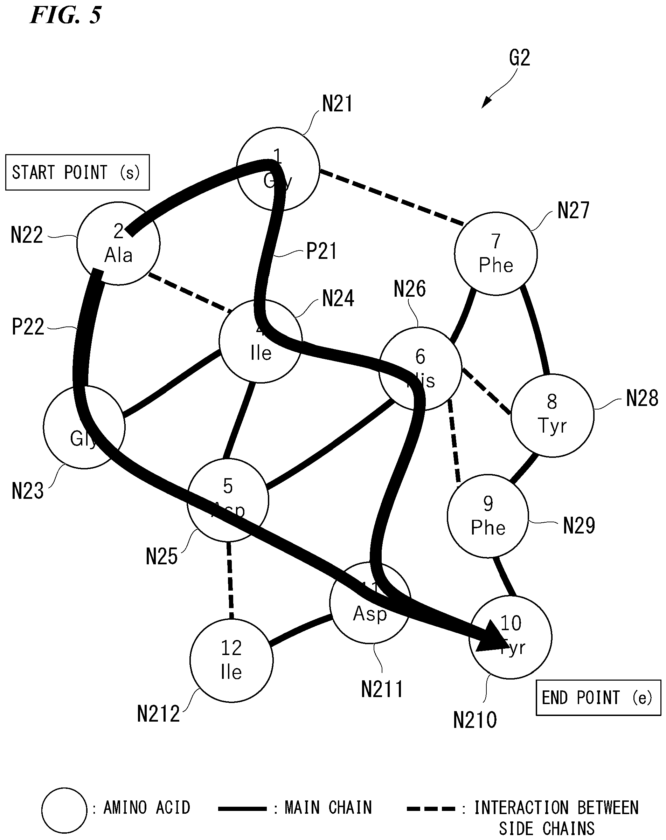

A signal transmission probability from a start point to an end point in a path connecting vertices in a network graph will be described below with reference to FIG. 5. FIG. 5 is a diagram illustrating a signal transmission probability from a start point to an end point of a path according to the present embodiment. When an amino acid residue $R_i$ and an amino acid residue $R_j$ are adjacent to each other, the signal transmission probability from the amino acid residue $R_i$ to the amino acid residue $R_j$ is expressed by the signal transmission probability $P_{ij}$, and the signal transmission probability from the amino acid residue $R_j$ to the amino acid residue $R_i$ is expressed by a signal transmission probability $P_{ji}$. The vertex N22 is used as the start point s of the path, and the vertex N210 is used as the end point e of the path.

A signal transmission probability from a start point s to an end point e is expressed by a product of the signal transmission probabilities between each vertex in a path. The product of the signal transmission probabilities between each vertex in a path is defined as an allosteric path score $APS_{s,e}^n$ of the path.

A path P21 is a first path from the start point s to the end point e and passes through the vertex N22 (start point s), the vertex N21, the vertex N24, the vertex N26, the vertex N211, and the vertex N210 (end point e) in this order. The allosteric path score $APS_{s,e}^1$ of the path P21 is expressed by Expression (4).
[Math. 4]

$$APS_{s,e}^1 = P_{2,1} \times P_{1,4} \times P_{4,6} \times P_{6,11} \times P_{11,10} \qquad \cdots (4)$$

A path P22 is a second path from the start point s to the end point e and passes through the vertex N22 (start point s), the vertex N23, the vertex N25, the vertex N211, and the vertex N210 (end point e) in this order. The allosteric path score $APS_{s,e}^2$ of the path P22 is expressed by Expression (5).
[Math. 5]

$$APS_{s,e}^2 = P_{2,3} \times P_{3,5} \times P_{5,11} \times P_{11,10} \qquad \cdots (5)$$

The allosteric paths from the start point s to the end point e are ranked by the allosteric path scores $APS_{s,e}^n$. The allosteric paths are evaluated to be important in allosteric control because the signal transmission probability from the start point s to the end point e increases as the allosteric path score $APS_{s,e}^n$ increases.

Here, each allosteric path is a combination of directed edges connecting vertices in a network graph. Accordingly, a problem for searching for allosteric paths with a high allosteric path score $APS_{s,e}^n$ is a combination optimization problem for searching for allosteric paths which are a combination of directed edges connecting vertices in a network graph.

In the present embodiment, it is assumed that an allosteric path is a single path. A single path does not include a bifurcation or a junction and is a path in which the number of start points is 1, and the number of end points is 1. In other words, a single path is a path which does not bifurcate and which does not have an interruption in the middle.

In the allosteric path prediction method according to the present embodiment, the combination optimization problem is solved on the basis of an Ising model. In the allosteric path prediction method according to the present embodiment, a simulated bifurcation machine (SBM) is used to calculate the Ising model. In the allosteric path prediction method according to the present embodiment, an optimal path with a highest allosteric path score $APS_{s,e}^n$ and suboptimal paths with high-ranked allosteric path scores $APS_{s,e}^n$ are predicted using a simulated bifurcation machine.

Here, a problem for calculating an approximate solution in which energy is as small as possible using an Ising model (that is, an approximate solution in which a value of an objective function approaches an optimal value as much as possible) is referred to as an Ising problem. On the other hand, a combination optimization problem for calculating a solution for minimizing a quadratic objective function with a discrete variable (bit) having one of 0 and 1 as a variable is referred to as a quadratic unconstrained binary optimization problem (QUBO). Here, a discrete variable in a QUBO problem can be converted to a spin in an Ising model through variable conversion, and the QUBO problem can be converted to an Ising problem.

An information processing device that calculates energy in the ground state of the Ising model is referred to as an Ising machine. A quantum annealer, a coherent machine, a quantum bifurcation machine, or the like is proposed as hardware mounting of the Ising machine. However, such hardware mounting may be likely to greatly shorten a calculation time, but has a problem in that an increase in scale or a stable operation is difficult.

Therefore, it is conceivable that a solution to the Ising problem be calculated using a digital computer which has spread widely. It is possible to more easily achieve an increase in scale and a stable operation of the digital computer in comparison with the hardware mounting. Simulated annealing is known as an example of an algorithm for calculating a solution to an Ising problem using a digital computer. Since the simulated annealing is a sequentially update algorithm in which a plurality of variables are sequentially updated, it is difficult to achieve an increase in processing speed of calculation based on parallelism.

A simulated bifurcation algorithm that can fast calculate a solution to a combination optimization problem with a large scale through parallel calculation in a digital computer has been proposed. A simulated bifurcation machine is a combination optimization algorithm that calculates a solution by numerically solving a motion equation of a classical model of a quantum bifurcation machine (for example, Non Patent Document 3).

As described above, in the allosteric path prediction method according to the present embodiment, the simulated bifurcation machine is used to calculate a solution to an Ising problem using a digital computer.

[Functional configuration of allosteric path prediction device 1]

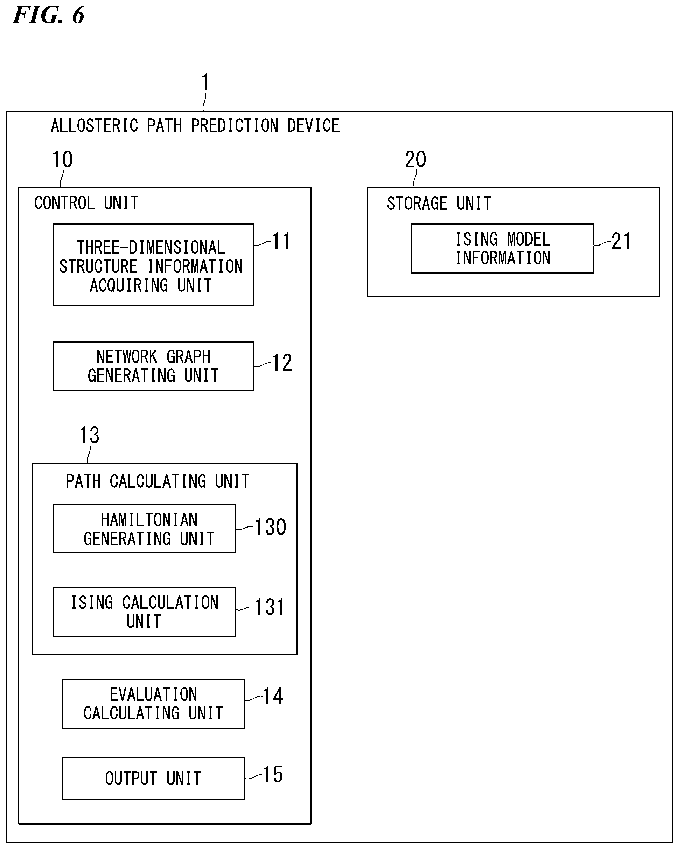

The functional configuration of a allosteric path prediction device 1 that performs an allosteric path predicting process which is a process of predicting an allosteric path will be described below with reference to FIG. 6. FIG. 6 is a diagram illustrating an example of the functional configuration of the allosteric path prediction device 1 according to the present embodiment. The allosteric path prediction device 1 is, for example, a computer such as a personal computer (PC), a workstation, or a server.

The allosteric path prediction device 1 includes a control unit 10 and a storage unit 20. The control unit 10 includes, for example, a central processing unit (CPU), and functional units of the control unit 10 are realized by causing the CPU to read and execute a program from a read only memory (ROM). The control unit 10 may include a graphics processing unit (GPU) or a field-programmable gate array (FPGA). The control unit 10 includes a three-dimensional structure information acquiring unit 11, a network graph generating unit 12, a path calculating unit 13, an evaluation unit 14, and an output unit 15.

The three-dimensional structure information acquiring unit 11 acquires three-dimensional structure information of a protein (also referred to as protein three-dimensional structure information). The protein three-dimensional structure information is information indicating a three-dimensional structure of a protein. The protein three-dimensional structure information can be acquired, for example, from a known database such as a protein data bank (PDB). In the PDB, coordinates of atoms constituting a protein which are analyzed using various techniques such as X-ray crystal structure analysis, NMR, and an electron microscope are registered. The protein three-dimensional structure information may be predicted by a three-dimensional structure prediction system such as AlphaFold2. The protein three-dimensional structure information may include coordinates of an arbitrary atom or an arbitrary group of atoms (hereinafter referred to as an arbitrary group of atoms together) analyzed at the same time as the protein in addition to atoms constituting proteins. Examples of the arbitrary group of atoms include proteins (which include antibodies), peptides (which include artificial peptides), nucleic acids (which includes an artificial nucleic acid), glycans, chemical/natural ligand/organic solvents, metals, various ions, and water molecules included in solvents, and middle/high molecules such as antibodiesm, peptides, and nucleic acids (which include artificial nucleic acids).

The protein three-dimensional structure information which is used may include all of atoms constituting proteins or may include only arbitrary atoms of interest. Atom coordinates of proteins registered in the PDB do not often include hydrogen atoms which are originally present. The reason is that it is difficult to identify coordinates of hydrogen atoms due to an influence of characteristics, resolutions, or the like of three-dimensional structure analysis techniques. Accordingly, only atom coordinates of a carbon atoms, oxygen atoms, nitrogen atoms, sulfur atoms, and the like other than hydrogen atoms may be used.

The number of proteins included in the protein three-dimensional structure information may be one or two or more. When the number of proteins is two or more, two or more proteins may be (covalently) bound to form one polypeptide by a linker or the like. For example, when proteins form one polypeptide, three-dimensional structure information including two or more proteins can be used. When two or more proteins are included, the types of the proteins may be the same or may be different from each other.

The network graph generating unit 12 generates a network graph on the basis of the three-dimensional structure information of a protein. The network graph generated by the network graph generating unit 12 is a graph which includes amino acid residues as vertices and in which weights based on an interaction between amino acid residues are assigned to edges thereof.

The path calculating unit 13 calculates a path connecting the vertices of the network graph generated by the network graph generating unit 12 on the basis of an evaluation function based on the weights assigned to the edges of the network graph. The evaluation function based on the weights is a Hamiltonian based on an Ising model. The path calculated by the path calculating unit 13 includes a suboptimal path which is a suboptimal solution along with an optimal path which is an optimal solution. An optimal solution is a local optimal solution.

As described above, in the present embodiment, the path is a single path. Accordingly, the path calculated by the path calculating unit 13 does not include a bifurcation or a junction, and the path is a path without an interruption in the middle which connects one start point and one end point. In the present embodiment, a start point and an end point of a path are designated in advance. Accordingly, in the present embodiment, a start point and an end point of a path are predetermined vertices in the network graph.

The path calculating unit 13 includes a Hamiltonian generating unit 130 and an Ising calculation unit 131.

The Hamiltonian generating unit 130 generates a Hamiltonian on the basis of the network graph. The Hamiltonian generated by the Hamiltonian generating unit 130 is expressed in the form of QUBO. QUBO is a problem to which a solution can be calculated using an Ising machine. In the following description, the Hamiltonian generated by the Hamiltonian generating unit 130 is also referred to as QUBO.

The Ising calculation unit 131 performs Ising calculation based on a simulated bifurcation machine on the Hamiltonian generated by the Hamiltonian generating unit 130. The simulated bifurcation machine is a parallel update algorithm, and parallel calculation is appropriately used for calculation in the Ising calculation unit 131.

The evaluation unit 14 evaluates the path calculated by the path calculating unit 13. The evaluation unit 14 extracts a set of paths with K (where K is a natural number equal to or greater than 1) highest evaluation values out of the paths calculated by the path calculating unit 13.

The output unit 15 outputs a prediction result of an allosteric path. The prediction result includes information indicating the set of paths with K (where K is a natural number equal to or greater than 1) highest evaluation values evaluated by the evaluation unit 14 and the evaluation result from the evaluation unit 14.

The storage unit 20 stores various types of information. The information stored in the storage unit 20 includes Ising model information 21. The Ising model information 21 indicates information on an objective function and constraint conditions which are used for the Hamiltonian generating unit 130 to generate a Hamiltonian. The storage unit 20 is constituted by a storage device such as a magnetic hard disk device or a semiconductor storage device.

[Allosteric path predicting process]

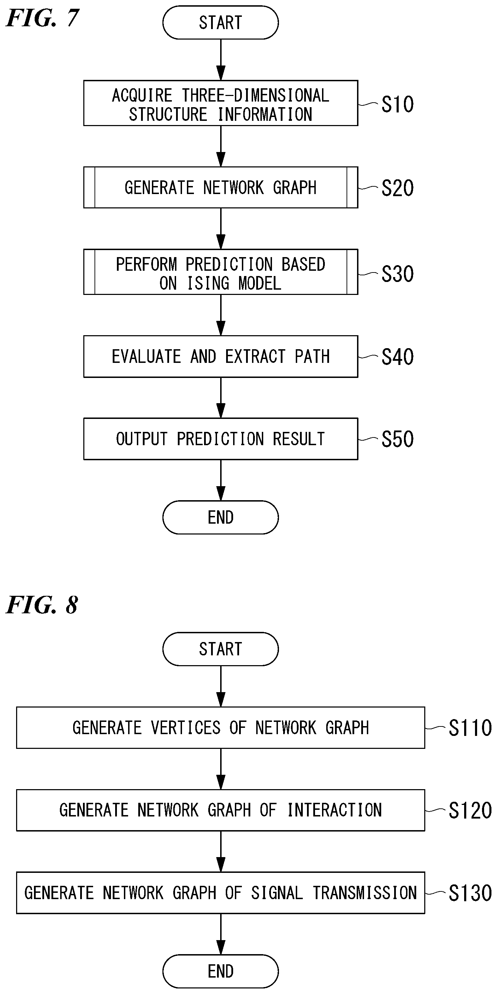

An allosteric path predicting process will be described below with reference to FIGS. 7 to 9. FIG. 7 is a diagram illustrating an example of the allosteric path predicting process according to the present embodiment. The allosteric path predicting process is performed by the control unit 10.

Step S10: The three-dimensional structure information acquiring unit 11 acquires three-dimensional structure information of proteins. The three-dimensional structure information acquiring unit 11 acquires three-dimensional structure information which is input by a user of the allosteric path prediction device 1. Thereafter, the control unit 10 performs the process of Step S20.

Step S20: The network graph generating unit 12 generates a network graph on the basis of the three-dimensional structure information acquired by the three-dimensional structure information acquiring unit 11. Thereafter, the control unit 10 performs the process of Step S30.

Details of a network graph generating process which is a process in which the network graph generating unit 12 generates a network graph will be described below with reference to FIG. 8. FIG. 8 is a diagram illustrating an example of the network graph generating process according to the present embodiment. The network graph generating process illustrated in FIG. 8 is performed in Step S20 illustrated in FIG. 7.

Step S110: The network graph generating unit 12 generates vertices of the network graph. The network graph generating unit 12 generates vertices for amino acid residues on the basis of the three-dimensional structure information. The network graph generating unit 12 generates the vertices such that the vertices correspond to the amino acid residues in a one-to-one manner. Thereafter, the network graph generating unit 12 performs the process of Step S120.

Step S120: The network graph generating unit 12 generates a network graph of interactions. Generation of a network graph of interactions means that an interaction matrix is calculated. Thereafter, the network graph generating unit 12 performs the process of Step S130.

Step S130: The network graph generating unit 12 generates a signal transmission network graph. Generation of a signal transmission network graph means that a signal transmission matrix is calculated.

Then, the network graph generating unit 12 ends the network graph generating process.

Description of the allosteric path predicting process will be continued with reference back to FIG. 7.

Step S30: The path calculating unit 13 performs a process of predicting an allosteric path on the basis of an Ising model. Thereafter, the control unit 10 performs the process of Step S40.

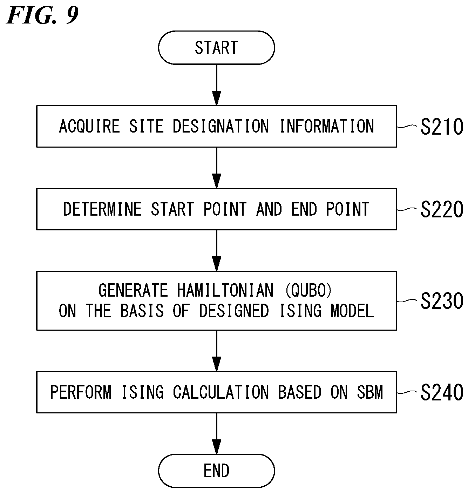

An Ising calculation process which is a process in which the path calculating unit 13 predicts an allosteric path on the basis of an Ising model will be described below with reference to FIG. 9. FIG. 9 is a diagram illustrating an example of the Ising calculation process according to the present embodiment. The Ising calculation process illustrated in FIG. 9 is performed in Step S30 illustrated in FIG. 7.

Step S210: The path calculating unit 13 acquires site designation information. The site designation information is information indicating an allosteric control site and an allosteric control target site. The site designation information is input, for example, by a user of the allosteric path prediction device 1. Thereafter, the path calculating unit 13 performs the process of Step S220.

Step S220: The path calculating unit 13 determines a start point and an end point of a path on the basis of the site designation information. The path calculating unit 13 determines a vertex corresponding to the allosteric control site indicated by the site designation information as a start point of the path. The path calculating unit 13 determines a vertex corresponding to the allosteric control target site indicated by the site designation information as an end point of the path. Thereafter, the path calculating unit 13 performs the process of Step S230.

Step S230: The Hamiltonian generating unit 130 generates a Hamiltonian on the basis of a designed mathematical model. The mathematical model is designed on the basis of the network graph generated by the network graph generating unit 12, designated objective function and constraint conditions, and the site designation information. The objective function and the constraint conditions are stored as Ising model information 21 in the storage unit 20 in advance. The objective function and the constraint conditions may be input by a user of the allosteric path prediction device 1. The mathematical model may be designed using an Ising model. The mathematical model is a model obtained by extending the Ising model and may be designed using a model including a high-powered expression, a rational expression, or a general real-valued function.

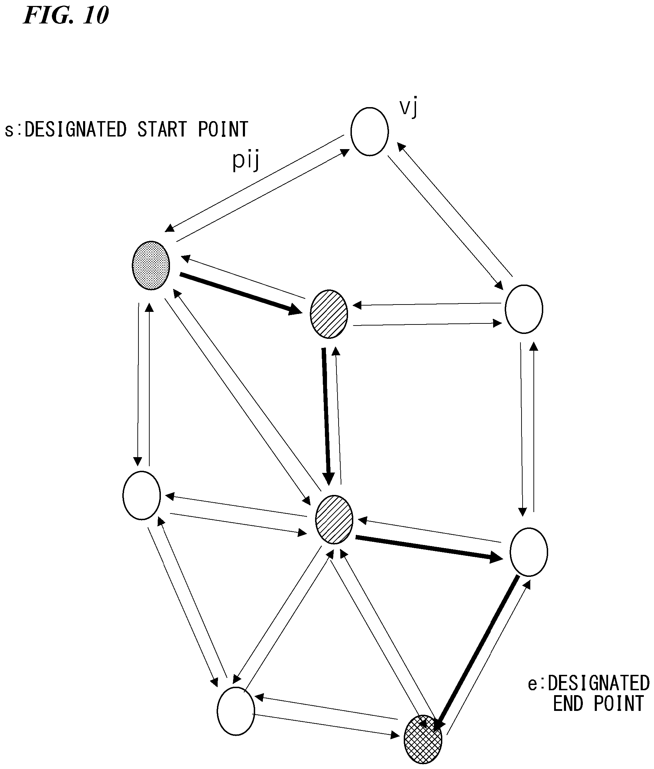

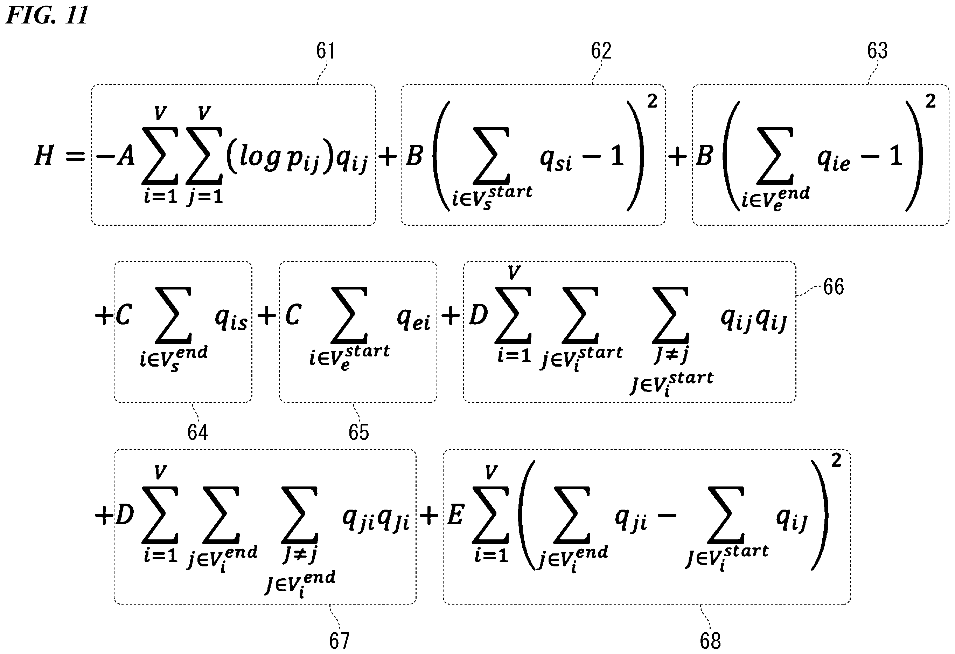

A Hamiltonian which is generated by the Hamiltonian generating unit 130 will be described below with reference to FIGS. 10 and 11. An example of the Hamiltonian generated by the Hamiltonian generating unit 130 is expressed as Expression (6). A, B, C, D, and E in Expression (6) are constants.
[Math. 6]

$$H = -A \sum_{i=1}^{V} \sum_{j=1}^{V} (\log p_{ij}) q_{ij} + B \left( \sum_{i \in V_s^{start}} q_{si} - 1 \right)^2 + B \left( \sum_{i \in V_e^{end}} q_{ie} - 1 \right)^2$$

$$+ C \sum_{i \in V_s^{end}} q_{is} + C \sum_{i \in V_e^{start}} q_{ei} + D \sum_{i=1}^{V} \sum_{\substack{j \in V_i^{start}}} \sum_{\substack{J \neq j \\ J \in V_i^{start}}} q_{ij} q_{iJ}$$

$$+ D \sum_{i=1}^{V} \sum_{\substack{j \in V_i^{end}}} \sum_{\substack{J \neq j \\ J \in V_i^{end}}} q_{ji} q_{Ji} + E \sum_{i=1}^{V} \left( \sum_{j \in V_i^{end}} q_{ji} - \sum_{J \in V_i^{start}} q_{iJ} \right)^2$$

$$\cdots (6)$$

In Expression (6), V denotes the number of vertices, and $p_{ij}$ indicates an interaction between a vertex $V_i$ and a vertex $V_j$.

$q_{ij}$ indicates a spin corresponding to a edge connecting a vertex $V_i$ and a vertex $V_j$. A value of the spin is 1 when the side corresponding to the spin is employed as a part of the path and is 0 when the side corresponding to the spin is not employed as a part of the path. The edge connecting a vertex $V_i$ and a vertex $V_j$ is a edge which is oriented as illustrated in FIG. 10. Accordingly, the edge connecting the vertex $V_i$ and the vertex $V_j$ includes a edge with the vertex $V_i$ as a start point and the vertex $V_j$ as an end point and a edge with the vertex $V_j$ as a start point and the vertex $V_i$ as an end point. Here when the edge with the vertex $V_i$ as a start point and the vertex $V_j$ as an end point is employed as the path, the vertex $V_i$ is expressed to have an outflow to the vertex $V_j$, or the vertex $V_j$ is expressed to have an inflow from the vertex $V_i$.

$V_i^{start}$ denotes a set of vertices which are an end point of a edge with the vertex $V_i$ as a start point. $V_i^{end}$ denotes a set of vertices which are a start point of a edge with the vertex $V_i$ as an end point. In Expression (6), a start point s denotes a designated start point, and an end point e denotes a designated end point.

The Hamiltonian generated by the Hamiltonian generating unit 130 includes an objective function and constraint conditions. As illustrated in FIG. 11, term 61 is an objective function. Term 62, term 63, term 64, term 65, term 66, term 67, and term 68 are constraint conditions. The constraint conditions are constraint conditions required in graph theory.

The objective function corresponds to a product of scores of edges which are employed for the path. As described above, $p_{ij}$ indicates an interaction between the vertex $V_i$ and the vertex $V_j$, and the interaction is the signal transmission probability $P_{ij}$. In Expression (6), for example, a logarithm of $p_{ij}$ is used as the weight assigned to the edge connecting the vertex $V_i$ and the vertex $V_j$.

Term 62 represents constraint conditions in which there is an outflow from the start point s to only one vertex (the number of outflows is 1). Term 63 represents constraint conditions in which there is an inflow to the end point e from only one vertex (the number of inflows is 1). Term 64 represents constraint conditions in which there is no inflow to the start point s from any vertices (the number of inflows is 0). Term 65 represents constraint conditions in which there is no outflow from the end point e to any vertices (the number of outflows is 0).

Term 66 represents constraint conditions in which there is an outflow from a vertex in the middle of a path to only one or less vertex (the number of outflows is 1 or less). Term 67 represents constraint conditions in which there is an inflow to a vertex in the middle of a path from only one or less vertex (the number of inflows is 1 or less). Term 68 represents constraint conditions in which the number of inflows to a vertex in the middle of a path and the number of outflows from the vertex are equal to each other. Term 66, term 67, and term 68 correspond to a path which is a single path in the present embodiment.

Thereafter, the path calculating unit 13 performs the process of Step S240.

Step S240: The Ising calculation unit 131 performs Ising calculation based on a simulated bifurcation machine on the Hamiltonian generated by the Hamiltonian generating unit 130.

In this way, the path calculating unit 13 ends the Ising calculation process.

Description of the allosteric path predicting process will be continued with reference back to FIG. 7.

Step S40: The evaluation unit 14 calculates an evaluation value of the path calculated by the path calculating unit 13. The evaluation value corresponds to energy indicated by the Hamiltonian, and a low evaluation value means that the path is evaluated to be a more optimal path. The evaluation unit 14 acquires energy indicated by the Hamiltonian for each path calculated by the Ising calculation unit 131 as an evaluation value of the corresponding path. The evaluation unit 14 determines rankings of the paths on the basis of the evaluation values. The evaluation unit 14 extracts a path of the first ranking as an optimal path. The evaluation unit 14 extracts paths of the second ranking to a predetermined ranking as suboptimal paths.

The evaluation unit 14 may determine whether the path calculated by the path calculating unit 13 is a single path for each path before calculating the evaluation value. In this case, the evaluation unit 14 calculates the evaluation value of each path determined to be a single path out of the paths calculated by the path calculating unit 13. The evaluation unit 14 does not calculate the evaluation value of a path determined not to be a single path out of the paths calculated by the path calculating unit 13.

The number of paths extracted by the evaluation unit 14 has only to be equal to or greater than one and may be two or more, three or more, five or more, ten or more, twenty or more, thirty or more, forty or more, fifty or more, 100 or more, 500 or more, or 1000 or more.

Thereafter, the control unit 10 performs the process of Step S50.

Step S50: The output unit 15 outputs a prediction result of an allosteric path. The output unit 15 outputs information indicating the optimal path and one or more suboptimal paths extracted by the evaluation unit 14 and the evaluation result from the evaluation unit 14 as a prediction result. The output unit 15 outputs the prediction result to, for example, an external server such as a database server, a display device (not illustrated in FIG. 6), or the storage unit 20.

In this way, the control unit 10 ends the allosteric path predicting process.

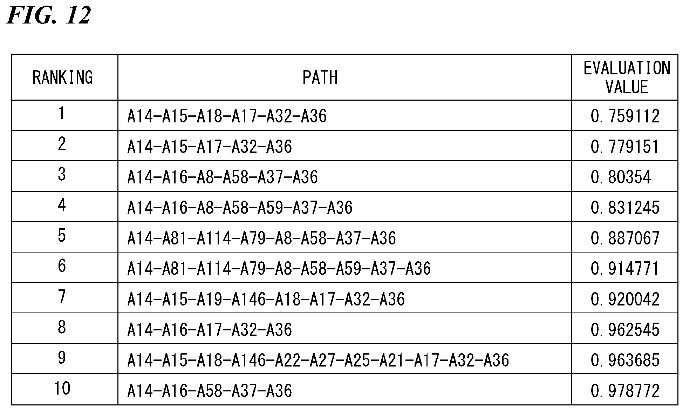

In FIG. 12, paths, rankings thereof, and evaluation values thereof are illustrated as a calculation result of the paths from the path calculating unit 13. In FIG. 12, high-ranked 10 paths as paths from the 14-th amino acid residue to the 36-th amino acid residue in KRAS are illustrated as examples.

In the present embodiment, an example in which amino acid residues constituting a protein are correlated with vertices of a network graph to generate the network graph has been described above, the present invention is not limited thereto. The network graph may be generated to include vertices of the network graph correlated with substances bound to a protein (referred to as binding substances). The network graph may be generated by assigning weights based on an interaction between a binding substance and an amino acid residue or an interaction between a binding substance and a binding substance as weights of sides of the network graph.

Examples of the binding substance include an arbitrary atom, an arbitrary ion, an arbitrary molecule, an arbitrary group of atoms, and an arbitrary compound. More specifically, the binding substance may include a protein (which includes an antibody), a peptide (which includes an artificial peptide), a nucleic acid (which includes an artificial nucleic acid), a glycan, a chemical/organic compound, a natural ligand, a metal, water, a solvent, and an ion. When atom coordinates of atoms are included in the protein three-dimensional structure information, vertices corresponding to the atoms may be included as the vertices of the network graph.

When a vertex corresponding to a binding substance or an atom is included as a vertex of the network graph, the vertex corresponding to the binding substance or the atom may be designated as one or more of a start point and an end point of a path in the allosteric path predicting process.

When the number of proteins included in the protein three-dimensional structure information is two or more, a vertex corresponding to an amino acid residue of a first protein molecule (or a binding substance bound to the first protein molecule) may be designated as a start point of a path, and a vertex corresponding to an amino acid residue constituting a second protein molecule (or a binding substance bound to the second protein molecule) bound to the first protein molecule may be designated as an end point of a path.

(Second embodiment)

A second embodiment of the present invention will be described below in detail with reference to the drawings.

In the first embodiment, suboptimal paths along with an optimal path have been calculated, and they have been calculated as candidates of one allosteric path. That is, in the first embodiment, allosteric control is converted to a model as one signal transmission path from a predetermined start point to a predetermined end point. It is thought that transmission of a signal is performed in combination of a plurality of paths in the allosteric control. Amino acid residues included in a plurality of paths with a high signal transmission probability play an important role in the allosteric control. In the present embodiment, it is assumed that an amino acid residue (that is, a vertex) important in allosteric control is extracted on the basis of the number of times the vertex is included in a plurality of predicted allosteric paths.

The same elements as in the first embodiment will be referred to by the same reference signs, and description of the same elements and operations may be omitted.

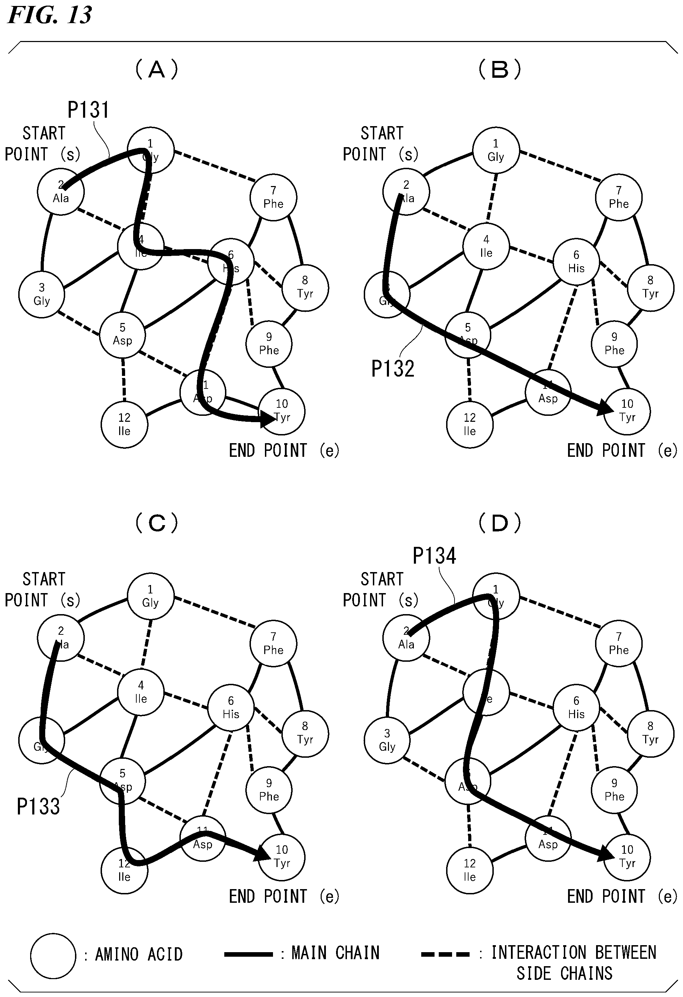

FIG. 13 is a diagram illustrating an example of the outline of a search method of an amino acid residue contributing to allosteric control according to the present embodiment. In the present embodiment, contribution of an amino acid residue to allosteric control is defined by the number of times the amino acid residue is included in paths with highest n evaluation values. A score for evaluating contribution of an amino acid residue to allosteric control is determined on the basis of the number of times a vertex corresponding to the amino acid residue is included in allosteric paths from a start point s to an end point e. When the score is defined as a residue score $RS_{s,e}^{i}$, the residue score $RS_{s,e}^{i}$ is expressed by Expression (7).

[Math. 7]

$$RS_{s,e}^{i} = \sum_{k=1}^{n} w_{(k)} \times q_i^k \qquad \cdots (7)$$

In Expression (7), $w_{(k)}$ is a weighting function corresponding to an allosteric path of a k-th ranking out of allosteric paths from the start point s to the end point e. In Expression (7), $q_{ik}$ is a numerical value which is 0 when an i-th amino acid residue is not included in the k-th path and which is 1 when the i-th amino acid residue is included in the k-th path as expressed by Expression (8).

[Math. 8]

$$q_i^k = \begin{cases} 0 & : \text{Residue i is not included in k-th path} \\ 1 & : \text{Residue i is included in k-th path} \end{cases} \qquad \cdots (8)$$

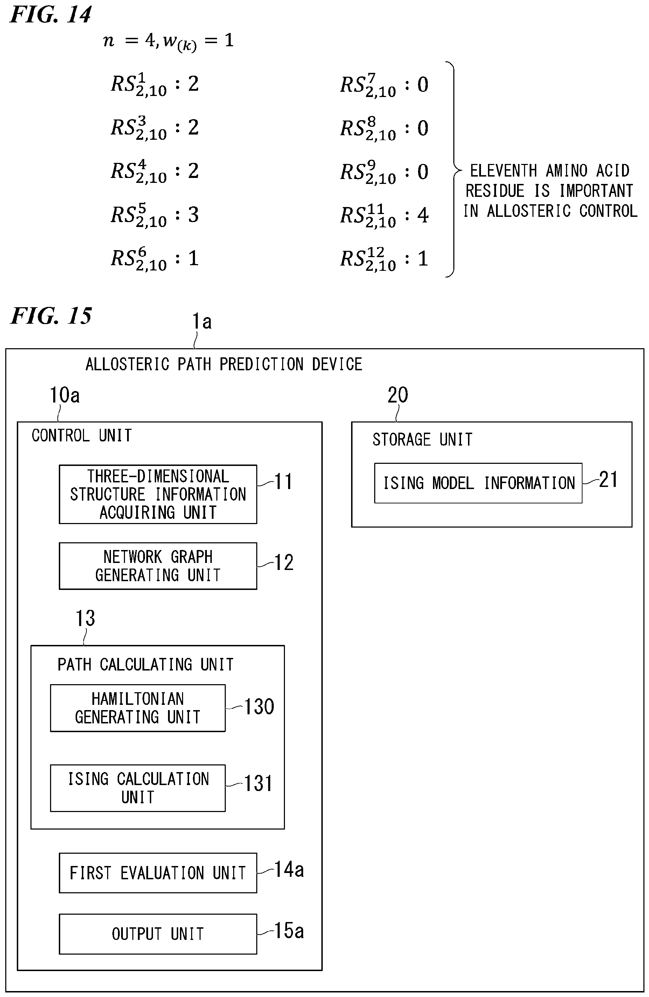

An allosteric path P131 illustrated in FIG. 13(A), an allosteric path P132 illustrated in FIG. 13(B), an allosteric path P133 illustrated in FIG. 13(C), and an allosteric path P134 illustrated in FIG. 13(D) are examples of allosteric paths of highest four rankings from the start point s to the end point e. The values of the residue scores $RS_{s,e}^{i}$ for the amino acid residues included in the four allosteric paths are illustrated in FIG. 14. As illustrated in FIG. 14, an eleventh amino acid residue out of ten amino acid residues except a second amino acid residue corresponding to the start point s and a tenth amino acid residue corresponding to the end point e has the highest residue score $RS_{s,e}^{i}$. Accordingly, the eleventh amino acid residue out of the ten amino acid residues is predicted to most contribute to allosteric control.

[Functional configuration of allosteric path prediction device 1a]

FIG. 15 is a diagram illustrating an example of the functional configuration of a allosteric path prediction device 1a according to the present embodiment. The allosteric path prediction device 1a includes a control unit 10a and a storage unit 20. The allosteric path prediction device 1a according to the present embodiment (FIG. 15) and the allosteric path prediction device 1 according to the first embodiment (FIG. 6) are different from each other in the control unit 10a. A description of the same functions as in the first embodiment will be omitted, and differences from the first embodiment will be mainly described in the second embodiment.

The control unit 10a includes a three-dimensional structure information acquiring unit 11, a network graph generating unit 12, a path calculating unit 13, a first evaluation unit 14a, and an output unit 15a. The control unit 10a (FIG. 15) and the control unit 10 (FIG. 6) are different in the first evaluation unit 14a and the output unit 15a. Here, the functions of the other elements (the three-dimensional structure information acquiring unit 11, the network graph generating unit 12, and the path calculating unit 13) are the same as those in the first embodiment.

In the present embodiment, the path calculating unit 13 calculates a plurality of paths. The first evaluation unit 14a evaluates a vertex included in a network graph on the basis of the number of times the vertex is included in the optimal path and the one or more suboptimal paths calculated by the path calculating unit 13. The first evaluation unit 14a performs the evaluation by calculating the residue score $RS_{s,e}^{i}$.

The output unit 15a outputs a search result of an important amino acid residue. The search result includes the evaluation result of the vertex from the first evaluation unit 14a.

[Important amino acid residue search process]

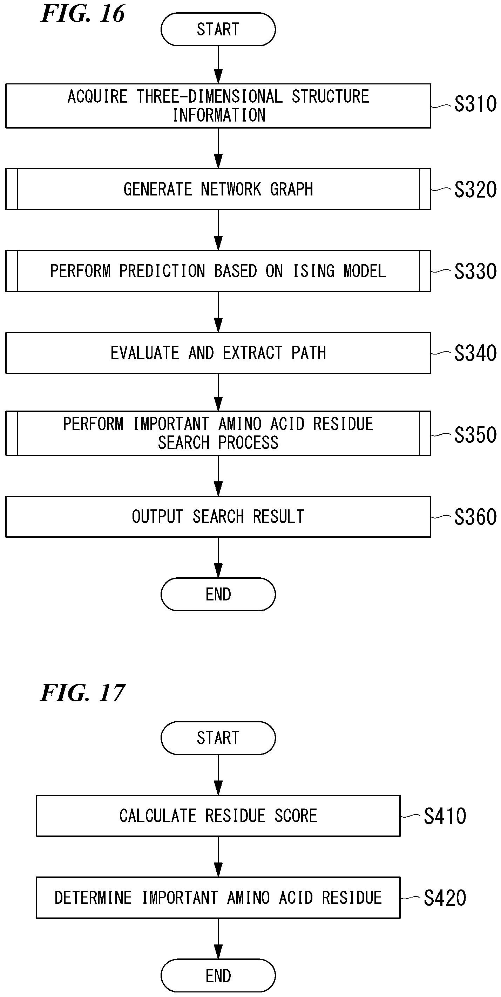

An important amino acid residue search process which is a process of searching for an amino acid residue contributing to allosteric control will be described below with reference to FIGS. 16 and 17. The important amino acid residue search process is performed as a part of the allosteric path predicting process. FIG. 16 is a diagram illustrating an example of the allosteric path predicting process according to the present embodiment. The processes of Steps S310 to S340 are the same as the processes of Steps S10 to S40 in FIG. 7, and thus description thereof will be omitted. In the process of Step S340 illustrated in FIG. 16, the rankings of the paths have only to be evaluated, and extraction of the optimal path and the one or more suboptimal paths may be omitted.

Step S350: The first evaluation unit 14a performs the important amino acid residue search process. Thereafter, the control unit 10a performs the process of Step S360.

Details of the important amino acid residue search process will be described below with reference to FIG. 17. FIG. 17 is a diagram illustrating an example of the important amino acid residue search process according to the present embodiment. The important amino acid residue search process illustrated in FIG. 17 is performed in Step S350 illustrated in FIG. 16.

Step S410: The first evaluation unit 14a calculates the residue scores $RS_{s,e}^i$ of the vertices included in the network graph. Thereafter, the first evaluation unit 14a performs the process of Step S420.

Step S420: The first evaluation unit 14a determines an important amino acid residue on the basis of the calculated residue scores $RS_{s,e}^i$. The first evaluation unit 14a determines, for example, vertices of predetermined highest rankings of the residue scores $RS_{s,e}^i$ as the important amino acid residues on the vertices other than the start point and the end point of a path out of the vertices included in the network graph.

In this way, the first evaluation unit 14a ends the important amino acid residue search process.

Description of the allosteric path predicting process will be continued with reference back to FIG. 16.

Step S360: The output unit 15a outputs an evaluation result of the vertices from the first evaluation unit 14a as an important amino acid residue search result. The output unit 15a may output the allosteric path prediction result along with the important amino acid residue search result.

In this way, the control unit 10a ends the allosteric path predicting process.

(First example of second embodiment)

In the present example, search for an important amino acid residue in allosteric control of an HRAS has been performed. It is known that proteins of an RAS family are activated by replacing a GDP bound to an RAS with a GTP and replacement of GDP/GTP in a normal RAS is caused through reaction of a GEF with the RAS. That is, replacement of GDP/GTP is allosterically controlled. In the allosteric control, a GEF binding region is an allosteric control region, and a GDP/GTP binding region is an allosteric control target region. Therefore, in the present example, it has been confirmed through a GDP/GTP replacement reaction in an HRAS that an amino acid residue contributing to allosteric control can be extracted using the important amino acid residue search process according to the second embodiment.

An allosteric path predicting process with a start point set to A719 and with an end point set to A528 is performed on HRAS structure information (PDB ID: 3 k8y). A719 is ACT which is a compound located in a GEF binding region in a PDB file, and A528 is GNP which is a GTP analogue. Here, notation Ai (where i is a natural number) refers to an i-th "amino acid" from an N-terminal in principle. The same is true of the following description. Here, notation Aj (where j is a natural number other than natural numbers assigned to known amino acids) is conveniently used for an atom or a compound other than amino acids constituting a protein.

The residue scores $RS_{s,e}^i$ of the amino acid residues are calculated using 10 paths with highest evaluation values (allosteric path scores $APS_{s,e}^n$) out of the paths acquired through the allosteric path predicting process. The allosteric path score $APS_{s,e}^k$ is used as a weighting function $w_{(k)}$ corresponding to an allosteric path as expressed by Expression (9).

[Math. 9]

$$w_{(k)} = APS_{s,e}^k \qquad \cdots (9)$$

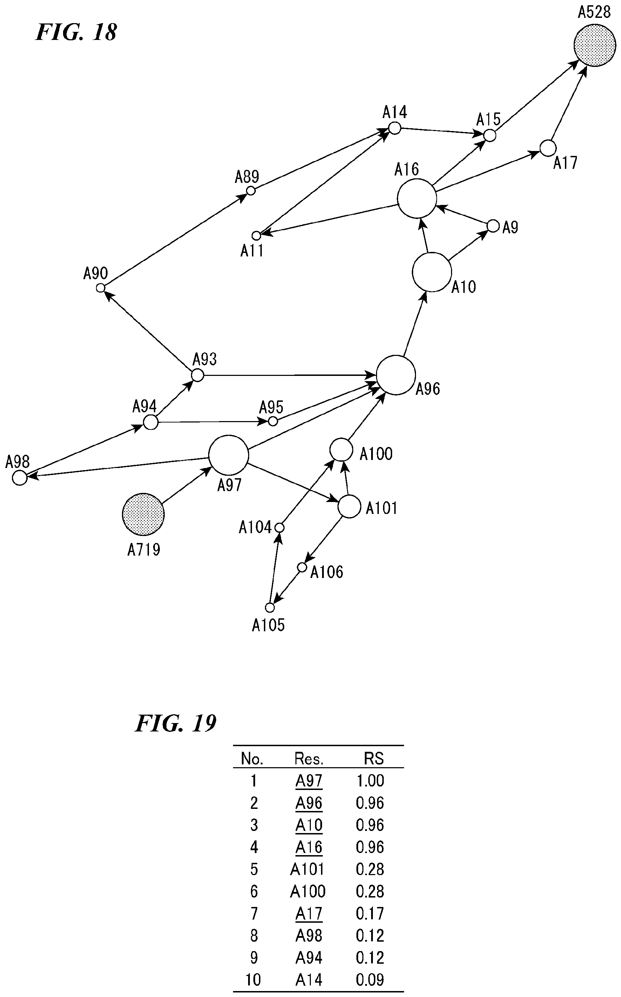

Search results of an important amino acid residue according to the present example are illustrated in FIGS. 18 and 19. In FIG. 18, 10 paths with highest evaluation values are illustrated along with amino acid residues. In FIG. 19, amino acid residues with 10 highest residue scores $RS_{s,e}^i$ are illustrated along with the residue scores $RS_{s,e}^i$.

The search result of an important amino acid residue according to the present example is compared with a result based on Ohm which is a technique according to the related art (see Non Patent Document 2). A97, A96, A10, A16, and A17 out of 10 amino acid residues of highest rankings in the search result according to the present example are also extracted as important amino acid residues in the result based on Ohm. That is, the result based on Ohm according to the related art can be reproduced using the important amino acid residue search process according to the second embodiment. In the search result according to the present example, amino acid residues such as A101, A100, A98, A94, and A14 which are not extracted through Ohm are extracted as important amino acid residues.

The fact that A101, A100, A98, A94, and A14 are extracted as important amino acid residues suggests that a combination of more bifurcating paths in comparison with the result based on Ohm contributes to allosteric control. This result is thought because path search is sequentially performed with whether there is signal transmission between each vertex as an indicator in the Ohm, but the path search is performed with signal transmission probabilities of all the paths as an indicator in the important amino acid residue search process according to the second embodiment.

(Second example of second embodiment)

In the present example, search for an important amino acid residue in allosteric control of a KRAS has been performed. A normal KRAS does not react with an effector protein in a state in which it is bound to GDP, but the normal KRAS can react with an effector protein by replacing GDP to GTP. In this way, a reaction with a KRAS with an effector protein is allosterically controlled using GDP/GTP. In the allosteric control, a GDP binding region is an allosteric control region, and an effector binding region is an allosteric control target region. On the other hand, a KRAS including G12C mutation cannot be allosterically controlled through GDP/GTP, but can always react with an effector protein (activating mutation)

In activating mutation, there is a likelihood that a larger change will occur an allosteric path from a GDP binding region to an effector binding region in comparison with a wild type. Therefore, it has been confirmed that activation of a protein based on the activating mutation can be evaluated using the allosteric path predicting process and the important amino acid residue search process according to the second embodiment with change of the allosteric path and change of the important amino acid residue as indicators.

The allosteric path predicting process with a start point set to an amino acid residue A14 and with an end point set to an amino acid residue A36 is performed on two pieces of KRAS structure information (PDB ID: 4obe: wild-type and PDB ID: 4ldj: G12C mutation). In each of two pieces of KRAS structure information, the residue scores $RS_{s,e}^{i}$ of the amino acid residues are calculated using 10 paths with highest evaluation values (allosteric path scores $APS_{s,e}^{n}$) out of the paths acquired through the allosteric path predicting process. The allosteric path score $APS_{s,e}^{k}$ is used as a weighting function $w_{(k)}$ corresponding to an allosteric path similarly to the first example.

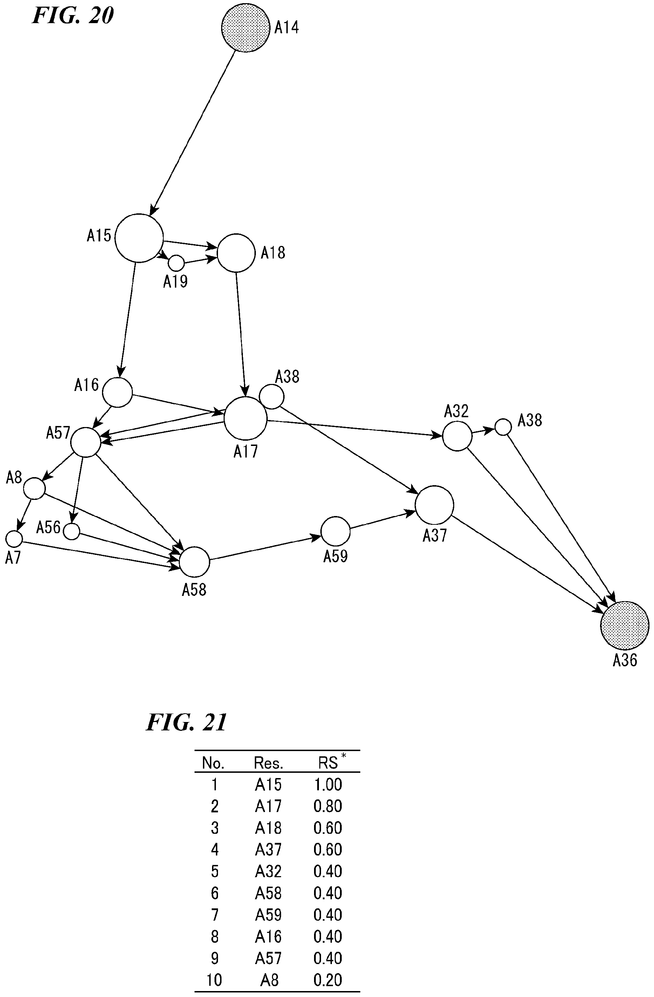

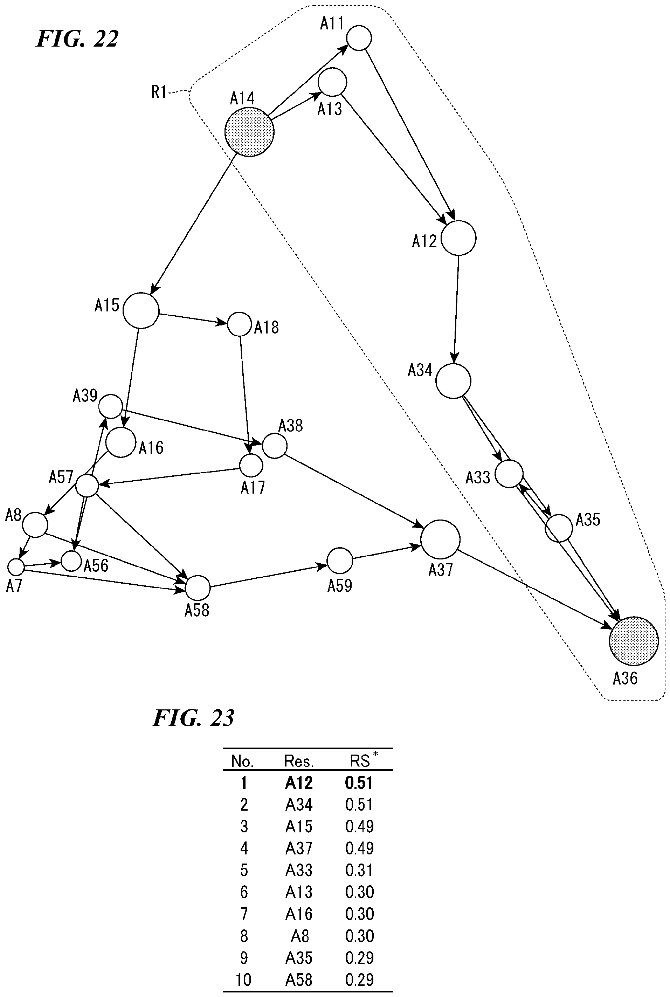

Search results of important amino acid residues according to the present example are illustrated in FIGS. 20 to 23. In FIG. 20, 10 paths with highest evaluation values in the wild type are illustrated along with amino acid residues. In FIG. 21, amino acid residues with 10 highest residue scores $RS_{s,e}^{i}$ in the wild type are illustrated along with the residue scores $RS_{s,e}^{i}$. In FIG. 22, 10 amino acid residues with highest evaluation values in the G12C mutation type are illustrated along with the residue scores $RS_{s,e}^{i}$. In FIG. 23, 10 amino acid residues with highest residue scores $RS_{s,e}^{i}$ in the G12C mutation type are illustrated along with the residue scores $RS_{s,e}^{i}$.

When FIG. 21 and FIG. 23 are compared in the search results, it can be seen that allosteric paths passing through A12 (an amino acid residue in which G12C mutation occurs) has been detected in the G12C mutation type. The allosteric paths passing through A12 are new paths which have not been detected in the wild type. The allosteric paths passing through A12 include an amino acid residue included in a region R1 as a vertex as illustrated in FIG. 22. The fact that a new path which has not been detected in the wild type is detected in the G12C mutation type is consistent with the fact that the allosteric control of a KRAS has changed due to the G12C mutation. This result teaches that an amino acid residue which is important in allosteric control of a protein can be extracted from the allosteric path.

(Third embodiment)

A third embodiment of the present invention will be described below in detail with reference to the drawings.

In the first embodiment and the second embodiment, an allosteric path is predicted with a start point and an end point of a path as predetermined vertices. In the present embodiment, only one of the start point and the end point is set as a predetermined vertex, and the other vertex is changed. In the present embodiment, by extracting a vertex which contributes strongly to a reactivity of the predetermined vertex out of the changed other vertices on the basis of evaluation values of allosteric paths, it is assumed that an allosteric control region or an allosteric control target region is searched for. In the following description, an allosteric control region or an allosteric control target region which is a search target may be referred to as an allosteric region.

The same elements as in the first embodiment or the second embodiment will be referred to by the same reference signs, and description of the same elements and operations may be omitted.

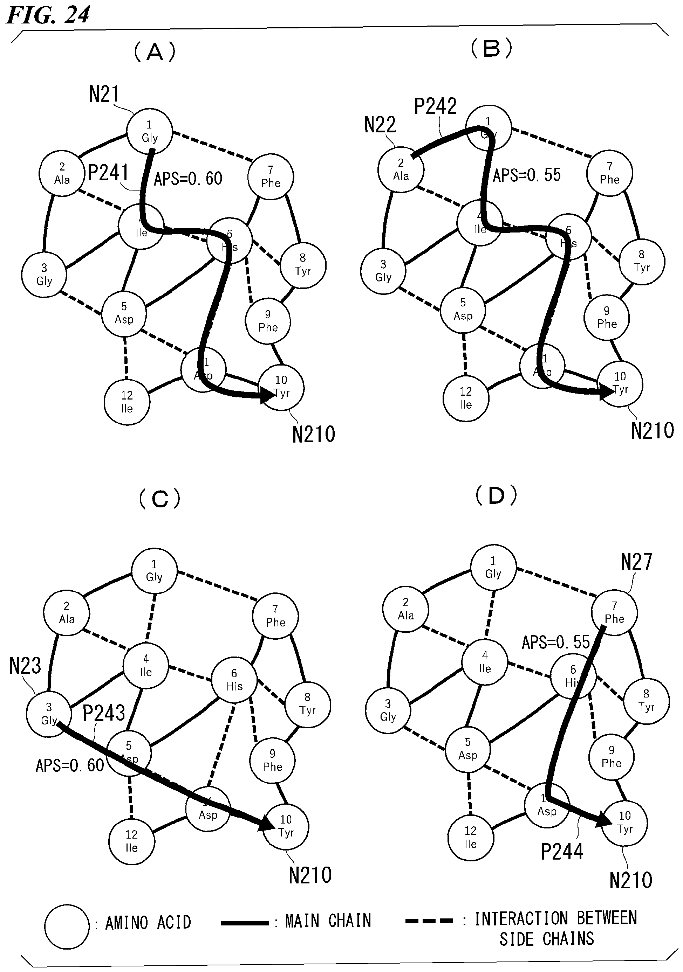

FIG. 24 is a diagram illustrating an example of the outline of an allosteric region search method according to the present embodiment. In the search method according to the present embodiment, a start point is set to a predetermined vertex and an end point is changed, or an end point is set to a predetermined vertex and a start point is changed. It is thought that an amino acid residue corresponding to an end point which is connected to a specific start point by a path with a high allosteric path score $APS_{s,e}^{n}$ greatly affects a reactivity of an amino acid residue corresponding to the start point. Similarly, it is thought that an amino acid residue corresponding to a start point which is connected to a specific end point by a path with a high allosteric path score $APS_{s,e}^{n}$ greatly affects a reactivity of an amino acid residue corresponding to the end point.

In the present embodiment, an index indicating such a degree of influence is referred to as an allosteric score. The allosteric score is an index indicating a degree of influence of each amino acid residue on a reactivity of a specific region. The allosteric score indicates a likelihood that each amino acid residue will serve as an allosteric control region when an allosteric control target region is designated as the specific region and indicates a likelihood that each amino acid residue will serve as an allosteric control target region when an allosteric control region is designated as the specific region. An example of the allosteric score is expressed by Expression (10). The aforementioned allosteric path score may be used as the allosteric score without any change.

[Math. 10]

$$AS_{s,e} = \sum_{k=1}^{n} w_{(k)} \times (corrected\ APS_{s,e}^{k}) \quad \cdots (10)$$

An allosteric score $AS_{s,e}$ expressed by Expression (10) represents the degree of influence of a start point s on an end point e. In Expression (10), corrected $APS_{s,e}^{k}$ is a value which is obtained by correcting the allosteric path score $APS_{s,e}^{n}$ using the length of a path. Examples of corrected $APS_{s,e}^{k}$ are expressed by Expression (11), Expression (12), and Expression (13).

[Math. 11]

$$corrected\ APS_{s,e}^{k} = \frac{\log(APS_{s,e}^{k})}{l_{s,e}^{k}} \quad \cdots (11)$$

[Math. 12]

$$corrected\ APS_{s,e}^{k} = \frac{APS_{s,e}^{k} - 1}{l_{s,e}^{k}} \quad \cdots (12)$$

[Math. 13]

$$corrected\ APS_{s,e}^{k} = (APS_{s,e}^{l})^{-l_{s,e}^{k}} \quad \cdots (13)$$

The allosteric path score $APS_{s,e}^{k}$ is an allosteric path score of a k-th path with a start point s and an end point e and is a product of signal transmission probabilities between each vertex of the paths similarly to the first embodiment. $l_{s,e}^{k}$ is a length of the k-th path with the start point s and the end point e. Here, a length of a path is the number of edges included in the path. The value of corrected $APS_{s,e}^{k}$ may be expressed by an expression other than Expression (11), Expression (12), and Expression (13) as long as $APS_{s,e}^{k}$ can be corrected on the basis of a length of a path.

In Expression (10), $w_{(k)}$ is a weighting function $w_{(k)}$ corresponding to the ranking (k) of a path. Accordingly, the allosteric score $AS_{s,e}$ is a weighted sum of corrected $APS_{s,e}^{k}$. In Expression (10), n is the number of paths which are used to calculate the allosteric score $AS_{s,e}$.

In FIG. 24, an example in which a start point is changed and an influence of the start point on a specific end point is illustrated. An allosteric path P241 illustrated in FIG. 24(A) is a path in which a vertex N21 corresponding to a first amino acid residue is used as a start point (start point "1") and a vertex N210 corresponding to a tenth amino acid residue is used as an end point (end point "10"). An allosteric path P242 illustrated in FIG. 24(B) is a path in which a vertex N22 corresponding to a second amino acid residue is used as a start point (start point "2") and the vertex N210 corresponding to the tenth amino acid residue is used as an end point (end point "10"). An allosteric path P243 illustrated in FIG. 24(C) is a path in which a vertex N21 corresponding to a third amino acid residue is used as a start point (start point "3") and the vertex N210 corresponding to the tenth amino acid residue is used as an end point (end point "10"). An allosteric path P244 illustrated in FIG. 24(D) is a path in which a vertex N21 corresponding to a seventh amino acid residue is used as a start point (start point "7") and the vertex N210 corresponding to the tenth amino acid residue is used as an end point (end point "10").

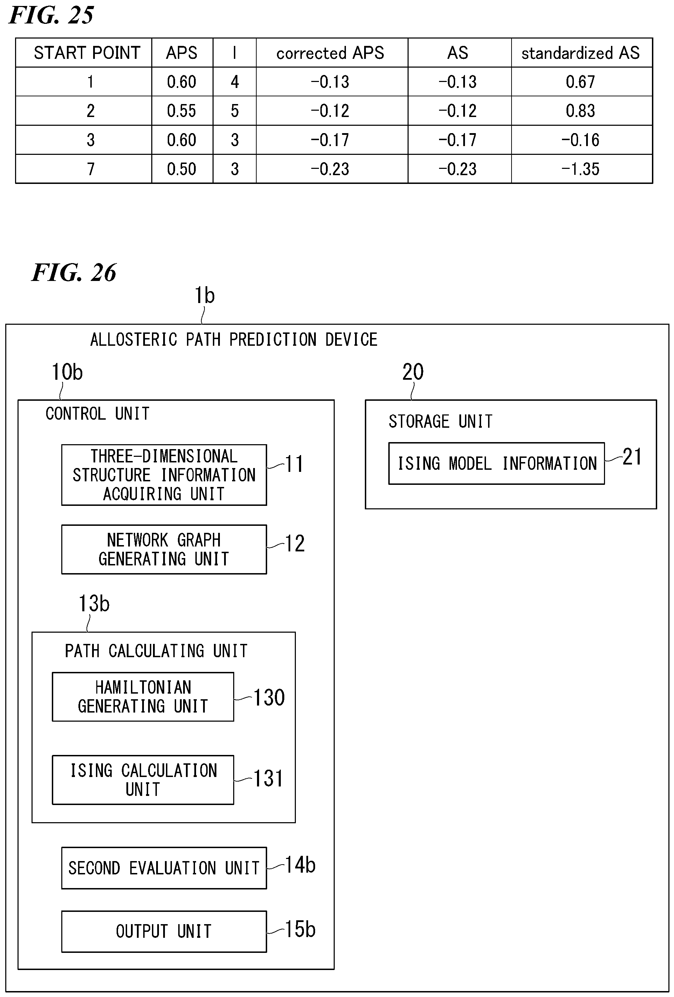

In FIG. 25, results of evaluation in which an influence of four start points of the start point "1," the start point "2," the start point "3," and the start point "7" on the end point "10" has been evaluated are illustrated. In the example illustrated in FIG. 25, one path is used to calculate the allosteric score $AS_{s,e}$ (that is, n=1). That is, in the example illustrated in FIG. 25, the start point and the end point correspond in a one-to-one manner. The value of the weighting function $w_{(k)}$ is 1 for all the paths. Expression (11) is used as corrected $APS_{s,e}^{k}$. In FIG. 25, a length of a path is illustrated as "1." In FIG. 25, a value obtained by standardizing the allosteric score $AS_{s,e}$ is illustrated along with the allosteric score $AS_{s,e}$. According to the evaluation results illustrated in FIG. 25, an influence of the second amino acid residue corresponding to the start point "2" on the amino acid residue corresponding to the end point "10" is determined to be the largest.

[Functional configuration of allosteric path prediction device 1b]

FIG. 26 is a diagram illustrating an example of the functional configuration of a allosteric path prediction device 1b according to the present embodiment. The allosteric path prediction device 1b includes a control unit 10b and a storage unit 20. The allosteric path prediction device 1b according to the present embodiment (FIG. 26) is different from the allosteric path prediction device 1 according to the first embodiment (FIG. 6) in the control unit 10b. Description of the same functions as in the first embodiment will be omitted, and differences from the first embodiment will be mainly described in the third embodiment.

The control unit 10b includes a three-dimensional structure information acquiring unit 11, a network graph generating unit 12, a path calculating unit 13b, a second evaluation unit 14b, and an output unit 15b. The control unit 10b (FIG. 26) is different from the control unit 10 (FIG. 6) in the path calculating unit 13b, the second evaluation unit 14b, and the output unit 15b). Here, the functions of the other elements (the three-dimensional structure information acquiring unit 11 and the network graph generating unit 12) are the same as in the first embodiment.

In the present embodiment, one of a start point and an end point of a path is a predetermined vertex in a network graph, and the other is an evaluation target vertex. The evaluation target vertex is a vertex to be evaluated by the second evaluation unit 14b. The evaluation target vertex is a vertex in which a degree of influence of an amino acid residue corresponding to the evaluation target vertex on the reactivity of an amino acid residue corresponding to a predetermined vertex is evaluated. In other words, the evaluation target vertex is a vertex corresponding to an allosteric region.

The path calculating unit 13b calculates a path connecting the predetermined vertex and the evaluation target vertex in the network graph for each combination of the predetermined vertex and the evaluation target vertex. In the present embodiment, the number of predetermined vertices is one. When one predetermined vertex is designated, a combination of the predetermined vertex and the evaluation target vertex is designated on the basis of the evaluation target vertex. Accordingly, in the present embodiment, the path calculating unit 13b calculates a path connecting the predetermined vertex and the evaluation target vertex out of the vertices in the network graph for each evaluation target vertex. A case in which the number of predetermined vertices is two or more will be described in a modified example of the third embodiment.

The second evaluation unit 14b calculates an evaluation value of each of a plurality of evaluation target vertices on the basis of the evaluation values of the path calculated by the path calculating unit 13b. The evaluation value calculated by the second evaluation unit 14b is the aforementioned allosteric score.

The output unit 15b outputs search results of an allosteric region. The search results include the evaluation values from the second evaluation unit 14b.

[Region search process]

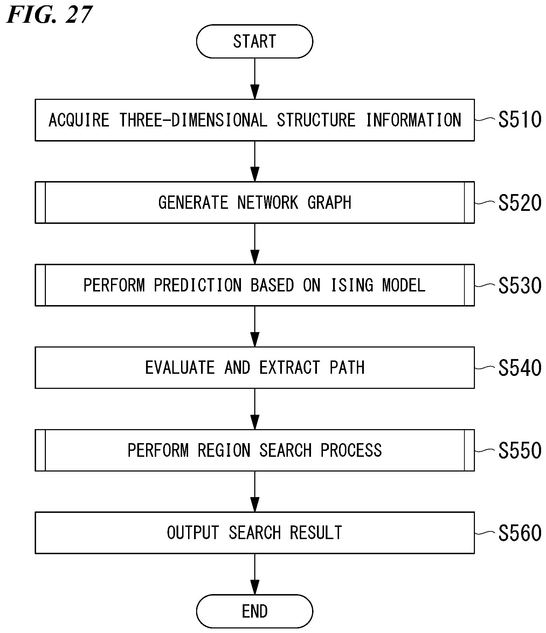

A region search process which is a process of searching for an allosteric region will be described below with reference to FIGS. 27 to 29. The region search process is performed as a part of the allosteric path predicting process. FIG. 27 is a diagram illustrating an example of the allosteric path predicting process according to the present embodiment. The processes of Steps S510, S520, and S540 are the same as the processes of Steps S10, S20, and S40 in FIG. 7, and thus a description thereof will be omitted. In the process of Step S540 illustrated in FIG. 27, evaluation of the rankings of the paths only needs to be performed, and extraction of one optimal path and one or more suboptimal paths may be skipped.

In the region search process illustrated in FIGS. 27 to 29, for example, it is assumed that an end point of a path is a predetermined vertex and a start point of the path is an evaluation target vertex. Even when the start point of the path is a predetermined vertex and the end point of the path is an evaluation target vertex, the region search process is the same as that when the end point of the path is a predetermined vertex and the start point of the path is an evaluation target vertex.

When the end point of a path is a predetermined vertex and the start point of the path is an evaluation target vertex, this case corresponds to evaluation of a degree of influence of an allosteric control site on a designated allosteric control target site with the allosteric control site as an evaluation target. On the other hand, when the start point of a path is a predetermined vertex and the end point of the path is an evaluation target vertex, this case corresponds to evaluation of a degree of influence of an allosteric control target site on a designated allosteric control site with the allosteric control target site as an evaluation target.

Step S530: The path calculating unit 13b performs a process of predicting an allosteric path on the basis of an Ising model. Thereafter, the control unit 10b performs the process of Step S540.

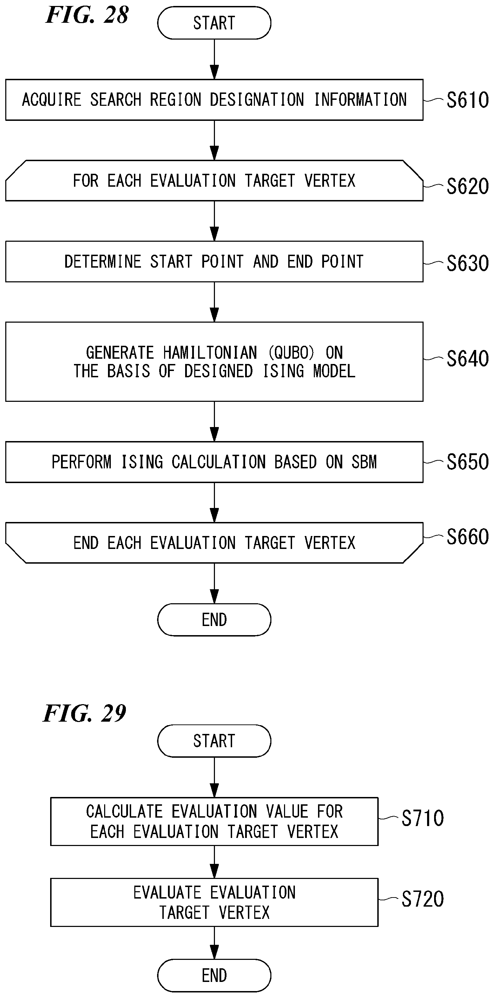

An Ising calculation process which is a process of causing the path calculating unit 13b to predict an allosteric path on the basis of an Ising model will be described below with reference to FIG. 28. FIG. 28 is a diagram illustrating an example of the Ising calculation process according to the present embodiment. The Ising calculation process illustrated in FIG. 28 is performed in Step S530 illustrated in FIG. 27. The processes of Steps S640 and S650 are the same as the processes of Steps S230 and S240 in FIG. 9, and thus description thereof will be omitted.

Step S610: The path calculating unit 13b acquires search region designation information. In the present embodiment, the search region designation information is information for designating an allosteric control target site and designating an allosteric control site as an evaluation target. The search region designation information is input, for example, by a user of the allosteric path prediction device 1b. Thereafter, the path calculating unit 13b performs the process of Step S620.

Step S620: The path calculating unit 13b starts a process of predicting an allosteric path for each evaluation target vertex. That is, the path calculating unit 13b designates each of one or more evaluation target vertices to one of a start point and an end point of a path and predicts an allosteric path for each evaluation target vertex. Thereafter, the path calculating unit 13b performs the process of Step S630.

Step S630: The path calculating unit 13b determines a start point and an end point of a path on the basis of the search region designation information. The path calculating unit 13b designates an evaluation target vertex indicated by the search region designation information as a start point of a path. The path calculating unit 13b determines a predetermined vertex indicated by the search region designation information as an end point of the path. Thereafter, the path calculating unit 13b performs the process of Step S640.

Step S660: The path calculating unit 13b ends the process of predicting an allosteric path for each evaluation target vertex.

In this way, the path calculating unit 13b ends the Ising calculation process.

Description of the allosteric path predicting process will be continued with reference back to FIG. 27.

Step S550: The second evaluation unit 14b performs a region search process. Thereafter, the control unit 10b performs the process of Step S560.

The region search process will be described below with reference to FIG. 29. FIG. 29 is a diagram illustrating an example of the region search process according to the present embodiment. The region search process illustrated in FIG. 29 is performed in Step S550 illustrated in FIG. 27.

Step S710: The second evaluation unit 14b calculates an evaluation value for each evaluation target vertex. The second evaluation unit 14b calculates an allosteric score as the evaluation value. Thereafter, the second evaluation unit 14b performs the process of Step S720.

Step S720: The second evaluation unit 14b evaluates the evaluation target vertex on the basis of the calculated evaluation value. The second evaluation unit 14b determines, for example, an evaluation target vertex in which the evaluation value is higher than a predetermined value. The second evaluation unit 14b may determine an evaluation target vertex with the highest evaluation value.

In this way, the second evaluation unit 14b ends the region search process.

Description of the allosteric path predicting process will be continued with reference back to FIG. 27.

Step S560: The output unit 15b outputs the evaluation result from the second evaluation unit 14b as a search result for an allosteric control site. The search result for an allosteric control site is a result obtained by searching for an allosteric control site of which a degree of influence on the designated allosteric control target site is evaluated to be high as described above.

Then, the control unit 10b ends the allosteric path predicting process.

(Example of third embodiment)

In the present example, an allosteric control region for controlling a reaction with an effector of a KRAS has been comprehensively searched for. A normal KRAS does not react with an effector protein in a state in which it is bound to a GDP, but can react with an effector protein by replacing the GDP is replaced with a GTP. In this way, a reaction of a KRAS with an effector protein is allosterically controlled by GDP/GTP. In the allosteric control, a GDP binding region is an allosteric control region, and an effector binding region is an allosteric control target region. In the present example, an allosteric path score between I36 located at the center of an RAF binding region which is an effector protein and each amino acid residue in a crystal structure with a PDB ID: 4obe of a normal KRAS is calculated, and an allosteric score is calculated. I36 corresponds to a predetermined vertex, and each amino acid residue corresponds to an evaluation target vertex.

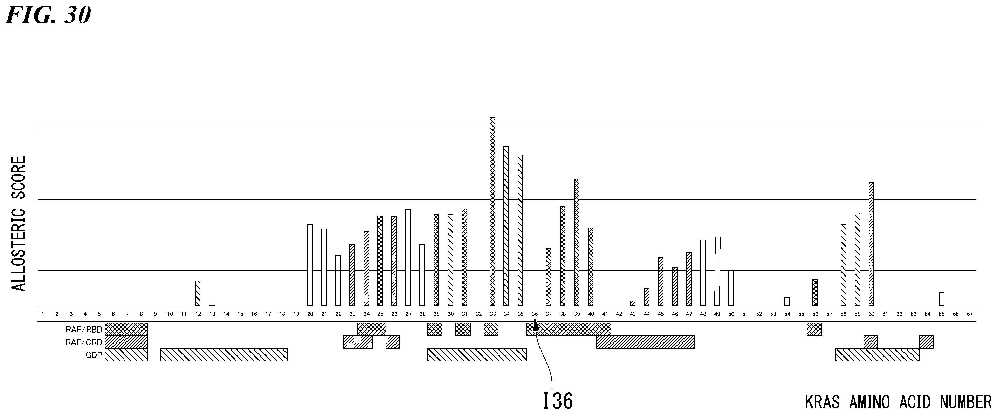

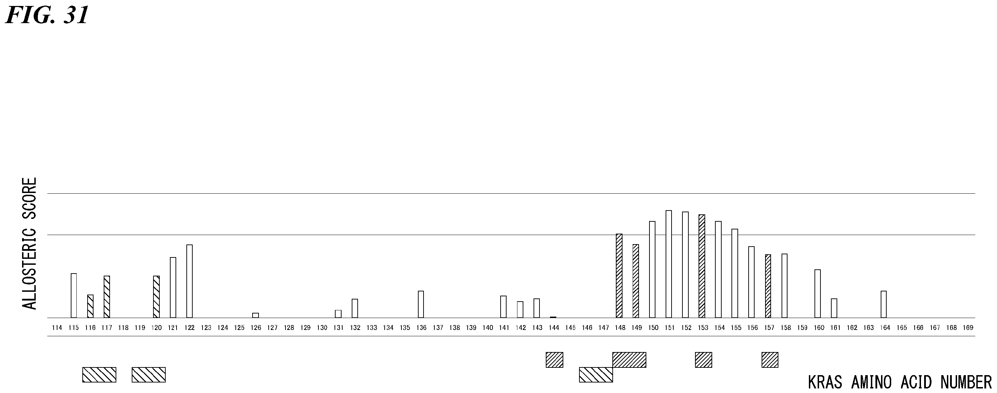

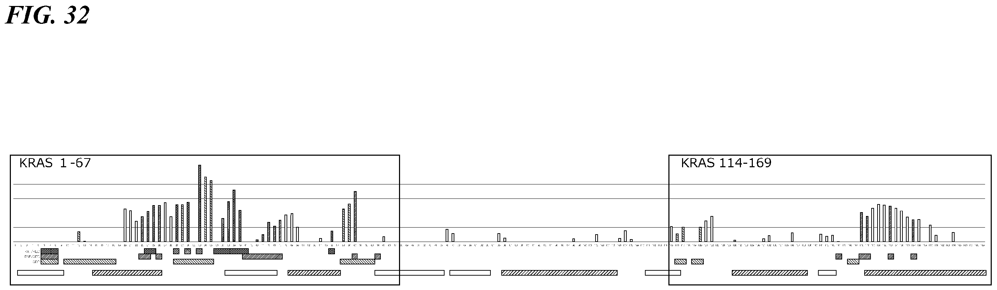

Search results of an allosteric control region according to the present example are illustrated in FIGS. 30 to 32. FIG. 30 is a diagram illustrating search results of an allosteric control region with respect to amino acid regions of which the amino acid number of a KRAS ranges from 1 to 67. FIG. 31 is a diagram illustrating search results of an allosteric control region with respect to amino acid regions of which the amino acid number of a KRAS ranges from 114 to 169. In FIGS. 30 and 31, an allosteric score for each amino acid number of the KRAS is indicated by a bar graph. FIG. 32 is a diagram illustrating search results of an allosteric control region with respect to the whole KRAS along with a position of an $\alpha$ helix chain.

As illustrated in FIG. 30, in regions in which the amino acid number ranges from 1 to 67, signals with high allosteric scores are observed in a GTP binding region, the vicinity of the GTP binding region, an RAF/RBD binding region, and an RAF/CRD binding region. As illustrated in FIG. 31, in regions in which the amino acid number ranges from 114 to 169, signals with high allosteric scores are observed in a GTP binding region and an RAF/CRD binding region. Regions in which signals with high allosteric scores are observed in the whole KRAS are concentrated on these interaction regions and the vicinity thereof. It has been confirmed that the allosteric score is an indicator which is effective to detect an allosteric control region for an interaction between a KRAS and an RAF.

As illustrated in FIG. 32, five $\alpha$ helixes are present in the KRAS. As illustrated in FIG. 32, signals with high allosteric scores are observed in only the interaction regions of the $\alpha$ helixes interacting with the RAF. When signals with high allosteric scores are observed in only the interaction regions of the $\alpha$ helixes, it means that the specificity of the allosteric region detected using the allosteric region search method according to the present embodiment is high.

(Modified example of third embodiment)

A modified example of the third embodiment will be described below in detail. In the present modified example, it is assumed that an allosteric control region or an allosteric control target region is searched for as a set of a plurality of start points or end points using the method of setting only one vertex of a start point and an end point to a predetermined vertex and changing the other vertex according to the third embodiment.

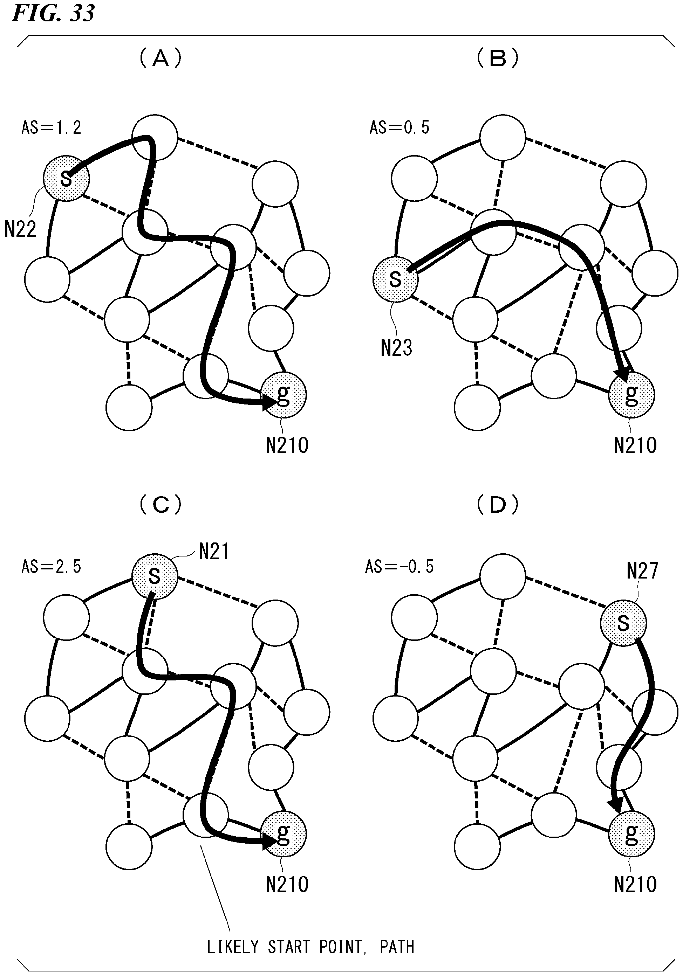

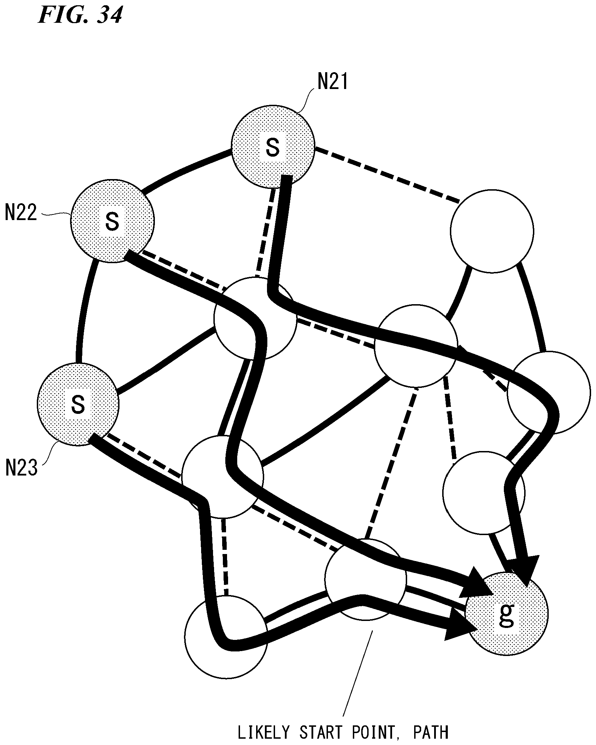

FIGS. 33 and 34 are diagrams illustrating an example of the outline of an allosteric region search method according to the present modified example. A plurality of residues included in an allosteric control region are close to each other in a three-dimensional structure. The allosteric region search method according to the present modified example is based on this study. First, as illustrated in FIG. 33, a specific end point is determined, and a plurality of start points connected to the specific end point via arbitrary paths are extracted. The start points may be exposed from a protein surface or may be buried in the protein, and is preferably exposed from the protein surface (which includes a substrate binding pocket). An allosteric score is calculated for each residue serving as the start points. As illustrated in FIG. 34, a score as a residue set including a plurality of residue close to each other is defined on the basis of the allosteric scores calculated for the residues. The score as a residue set based on the allosteric scores is referred to as a residue set score. An allosteric control region is searched for based on the premise that a residue set in which the residue set score is the highest is the allosteric control region.

In the example illustrated in FIG. 34, the allosteric scores for the residues and the residue set score in consideration of a degree of closeness between the residues in a three-dimensional structure are defined, and three vertices of a vertex N21, a vertex N22, and a vertex N23 are extracted as a residue set with the highest residue set score. Since the residue set score is defined to become higher when the allosteric scores of the residues included in the set are high and are three-dimensionally condensed, a residue such as a vertex N27 with a negative allosteric score is excluded from the allosteric control region.

[Functional configuration of allosteric path prediction device 1d]

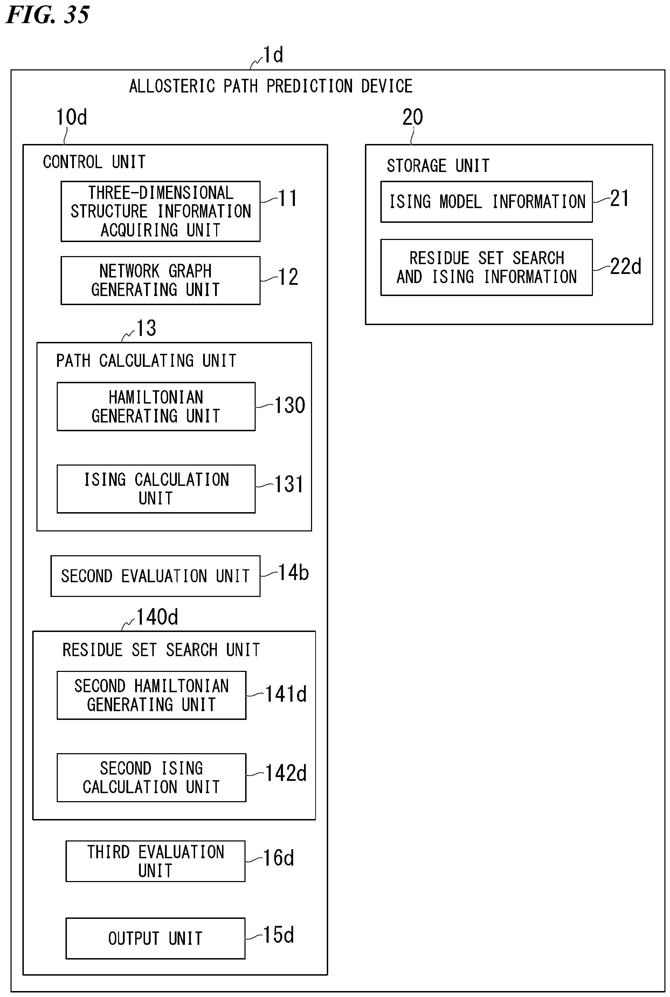

FIG. 35 is a diagram illustrating an example of the functional configuration of a allosteric path prediction device 1d according to the present modified example. The allosteric path prediction device 1d includes a control unit 10d and a storage unit 20. The allosteric path prediction device 1d according to the present modified example (FIG. 35) and the allosteric path prediction device 1b according to the third embodiment (FIG. 26) are different from each other in the control unit 10d and residue set search Ising information 22d stored in the storage unit 20. Description of the same functions as in the third embodiment will be omitted, and differences from the third embodiment will be mainly described in the present modified example.

The control unit 10d includes a three-dimensional structure information acquiring unit 11, a network graph generating unit 12, a path calculating unit 13, a second evaluation unit 14b, a residue set search unit 140d, a third evaluation unit 16d, and an output unit 15d. The control unit 10d (FIG. 35) and the control unit 10b (FIG. 26) are different from each other in the residue set search unit 140d, the third evaluation unit 16d, the output unit 15d, and residue set search Ising information 22d stored along with the Ising model information 21 in the storage unit 20. Here, the functions of the other elements (the three-dimensional structure information acquiring unit 11 and the network graph generating unit 12) are the same as those in the first embodiment.

The residue set search unit 140d includes a second Hamiltonian generating unit 141d and a second Ising calculation unit 142d.

The second Hamiltonian generating unit 141d generates a Hamiltonian for searching for a residue set. A objective function and constraint conditions which are used for the second Hamiltonian generating unit 141d to generate the Hamiltonian are stored in advance as residue set search Ising information 22d in the storage unit 20. A specific example of the Hamiltonian will be described later.

The second Ising calculation unit 142d performs Ising calculation based on a simulated bifurcation machine on the Hamiltonian generated by the second Hamiltonian generating unit 141d.

The third evaluation unit 16d calculates an evaluation value for a subset of a plurality of evaluation target vertices on the basis of the evaluation value calculated for each evaluation target vertex by the second evaluation unit 14b. A residue set which will be described below is an example of a subset of a plurality of evaluation target vertices. A residue set score which will be described below is an example of an evaluation value calculated by the third evaluation unit 16d.

The output unit 15d outputs a residue set search result. The search result includes the evaluation values from the third evaluation unit 16d.

[Residue set search process]

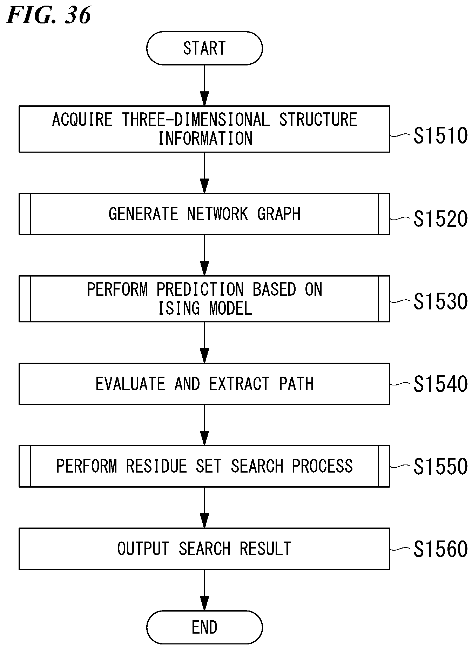

A residue set search process will be described below with reference to FIGS. 36 to 38. The residue set search process is performed as a part of the allosteric path predicting process. FIG. 36 is a diagram illustrating an example of an allosteric path predicting process according to the present modified example. The processes of Steps S1510, S1520, and S1540 are the same as the processes of Steps S510, S520, and S540 in FIG. 27, and thus a description thereof will be omitted.

In the residue set search process illustrated in FIGS. 36 to 38, for example, it is assumed that a set of end points of paths is an active center and a set of start points of paths is an allosteric control region. The residue set search process is performed in the same way even when the set of start points of paths is an active center and the set of end points of paths is an allosteric control region.

Step S1530: The path calculating unit 13 performs a process of predicting an allosteric path (an Ising calculation process) on the basis of an Ising model. Thereafter, the control unit 10d performs the process of Step S1540. The Ising calculation process according to the present modified example and the Ising calculation process according to the third embodiment (FIG. 28) are different from each other in a region designated by the search region designation information. In the Ising calculation process according to the present modified example, the search region designation information is information for designating an activie center (an allosteric control target site) and designating a residue set (an allosteric control site) as an evaluation target. The Ising calculation process according to the present modified example is the same as the Ising calculation process according to the third embodiment (FIG. 28) except the region designated by the search region designation information, and thus a description thereof will be omitted.

Step S1550: The residue set search unit 140d performs the residue set search process. Thereafter, the control unit 10d performs the process of Step S1560.

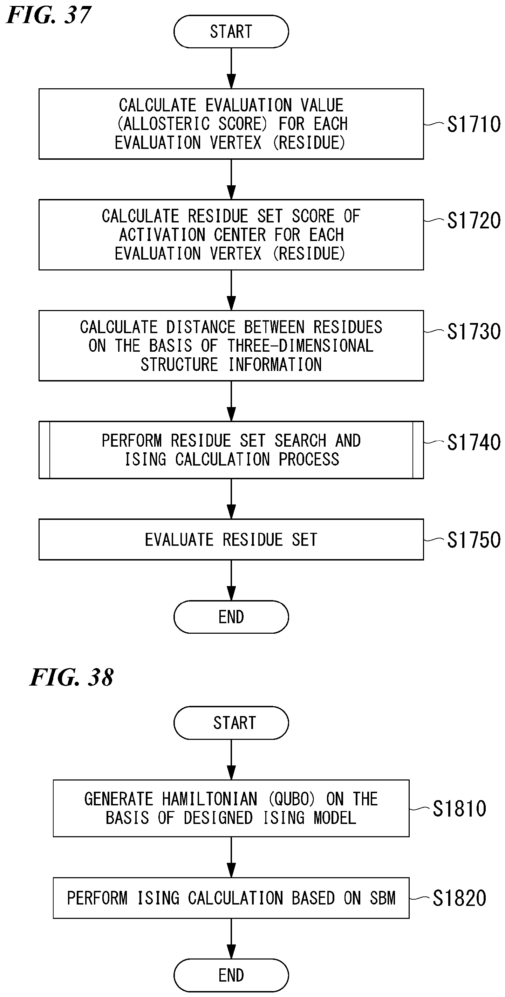

The residue set search process will be described below with reference to FIG. 37. FIG. 37 is a diagram illustrating an example of the residue set search process according to the present modified example. The residue set search process illustrated in FIG. 37 is performed in Step S1550 illustrated in FIG. 36.

Step S1710: The residue set search unit 140d causes the second evaluation unit 14b to calculate an evaluation value for each evaluation target vertex. Here, the evaluation target vertex is a residue included in the residue set which is an evaluation target as described above. The second evaluation unit 14b calculates an allosteric score as the evaluation value. An example of the allosteric score is expressed by Expression (14).

[Math. 14]

$$AS_{i,e_j} = \sum_{k=1}^{n} w(k) \times \left( corrected\ APS_{i,e_j}^k \right) \quad \cdots (1\,4)$$

The allosteric score expressed by Expression (14) is the same as the allosteric score (Expression (10)) described above in the third embodiment except that a residue $e_j$ included in the active center is designated as an end point. One of Expressions (11), (12), and (13) described above may be employed as corrected $APS_{s,e}^k$.

Thereafter, the third evaluation unit 16d performs the process of Step S1720.

Step S1720: The third evaluation unit 16d calculates an evaluation value for the active center for each evaluation target vertex (residue). The evaluation value is expressed, for example, by Expression (15).

[Math. 15]

$$AS_{i,e} = \sum_{j=1}^{|e|} standardized\ AS_{i,e_j} \quad \cdots (1\,5)$$

As illustrated in FIG. (15), the evaluation value for the active center for each evaluation target vertex (residue) calculated by the third evaluation unit 16d is a sum for the residues $e_j$ included in the active center of an amount obtained by standardizing the allosteric score expressed by Expression (14). Here, when the allosteric score is not standardized at the time of calculation of the evaluation value, the allosteric scores for paths with extremely low scores may become dominant. Accordingly, at the time of calculation of the evaluation value, it is preferable to standardize the allosteric scores as expressed by Expression (15). The allosteric scores may not be standardized at the time of calculation of the evaluation value.

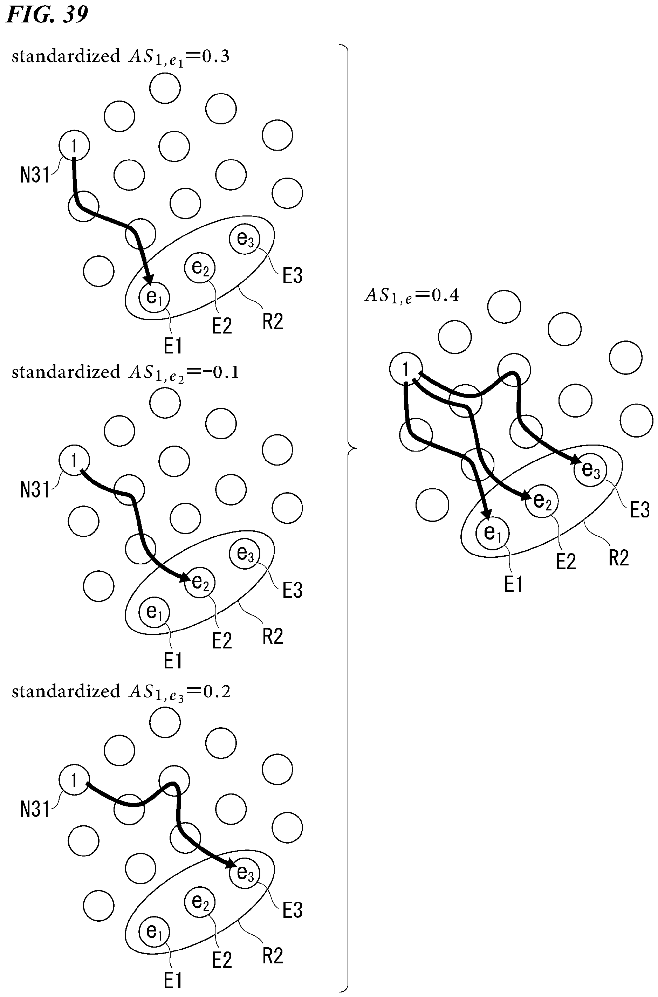

The outline of a process of causing the third evaluation unit 16d to calculate an evaluation value for the active center for each evaluation target vertex (residue) is illustrated in FIG. 39. In FIG. 39, an evaluation value for an active center R2 is calculated for a vertex N31. Three vertices of a vertex E1, a vertex E2, and a vertex E3 are included in the active center R2. The evaluation value of the vertex N31 for the active center R2 is calculated as a sum of an allosteric score of a path with the vertex N31 is a start point and with the vertex E1 as an end point, an allosteric score of a path with the vertex N31 is a start point and with the vertex E2 as an end point, and an allosteric score of a path with the vertex N31 is a start point and with the vertex E3 as an end point. The evaluation value for the active center R2 is calculated while changing the vertex N31 as a start point.

Step S1730: The residue set search unit 140d calculates a distance between residues on the basis of three-dimensional structure information. Thereafter, the control unit 10d performs the process of Step S1740.

The three-dimensional structure information is acquired by the three-dimensional structure information acquiring unit 11 as described above. The distance between residues is, for example, the distance between a representative atom of atoms constituting one residue and a representative atom of atoms constituting the other residue. Here, a representative atom is a predetermined atom of atoms constituting a residue. The representative atom is, for example, alpha carbon. The representative atom may be an atom other than alpha carbon. Information for designating a representative atom is included, for example, in the residue set search Ising information 22d.

The distance between residues may be determined on the basis of a signal transmission probability $P_{ij}$. For example, the distance between residues may be the reciprocal of the signal transmission probability $P_{ij}$. The distance between residues may be a value obtained by subtracting the value of the signal transmission probability $P_{ij}$ from 1.

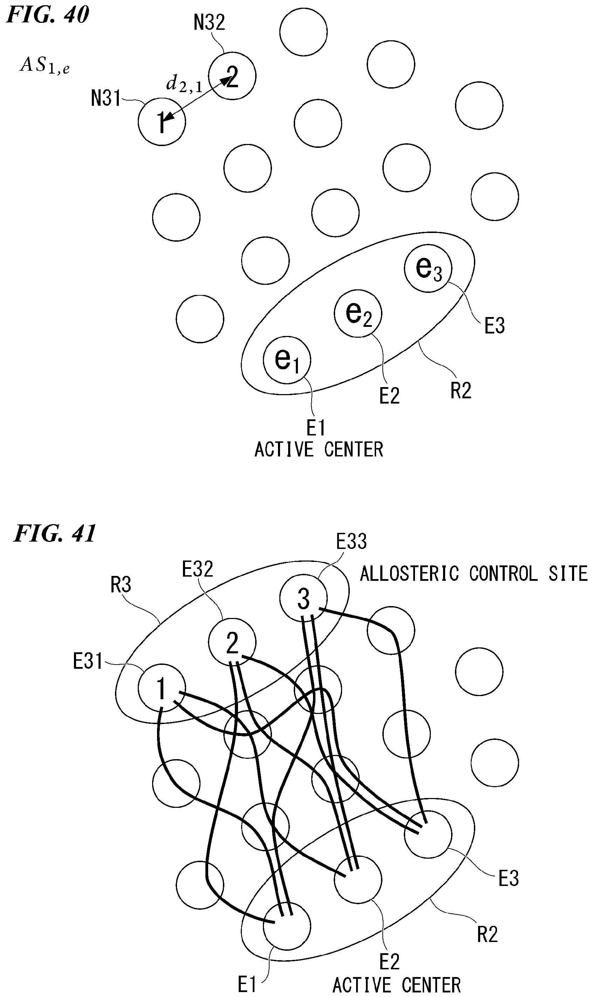

The distance between residues may be the minimum distance out of distances between two atoms constituting a group of an atom constituting one residue and an atom constituting the other residue. The distance between residues may be an average value in each group of distances between two atoms constituting the group of an atom constituting one residue and an atom constituting the other residue. The distance between residues may be the distance between the centers of the residues. FIG. 40 illustrates a distance $d_{2,1}$ between a vertex N31 and a vertex N32 as an example of the distance between residues.

Step S1740: The residue set search unit 140d performs a process of searching for a residue set on the basis of the Ising machine.

A residue set search and Ising calculation process which is a process of causing the residue set search unit 140d to search for a residue set on the basis of the Ising machine will be described below with reference to FIG. 38. FIG. 38 is a diagram illustrating an example of the residue set search and Ising calculation process according to the present modified example. The residue set search and Ising calculation process illustrated in FIG. 38 is performed in Step S1740 illustrated in FIG. 37.

Step S1810: The second Hamiltonian generating unit 141d generates a Hamiltonian on the basis of a designed mathematical model. In the residue set search and Ising calculation process, an objective function and constraint conditions included in the Hamiltonian generated by the second Hamiltonian generating unit 141d are stored in advance as residue set search Ising information 22d in the storage unit 20. The target function and the constraint conditions may be input by a user of the allosteric path prediction device 1. The mathematical model may be designed using the Ising model. The mathematical model is an extended model of the Ising model and may be designed using a model including a high-powered expression, a rational expression, or a general real-valued function.

An example of the Hamiltonian generated by the second Hamiltonian generating unit 141d is expressed by Expression (16).

[Math. 16]

$$H = -\sum_{i=0}^{|V|} AS_{i,e}q_i + A \sum_{i=0}^{|V|} \sum_{j=0}^{i} D_{i,j}q_iq_j \quad \cdots (16)$$

In Expression (16), $q_i$ denotes a spin corresponding to a residue. When the residue is selected as an element of a residue set, the value of $q_i$ is 1. When the residue is not selected as an element of a residue set, the value of $q_i$ is 0. The first term on the right side of Expression (16) represents the objective function, and the second term on the right side represents the constraint conditions.

The target function represented by the first term indicates conditions in which a certain residue set can be more easily selected as a sum of the allosteric scores for the residues included in the residue set becomes larger.

The constraint conditions represented by the second term indicate conditions in which residues between which the distance is smaller can be easily selected. $D_{ij}$ in the second term is expressed by Expression (17). A in the second term is a constant.

[Math. 17]

$$D_{i,j} = \begin{cases} 1 & if \ d_{i,j} > threshold \\ 0 & else \end{cases} \quad \cdots (17)$$

Accordingly, the constraint conditions represented by the second term on the right side of Expression (16) indicate that, when the distance $d_{ij}$ between one residue $q_i$ and the other residue $q_j$ is equal to or greater than a predetermined distance ("threshold"), the residues are not difficult to simultaneously select. That is, in the Hamiltonian expressed by Expression (16), the maximum distance between residues to be selected is limited.

A Hamiltonian expressed by Expression (18) may be provided instead of the Hamiltonian expressed by Expression (16).

[Math. 18]

$$H = -\sum_{i=0}^{|V|} AS_{i,e}q_i + A \sum_{i=0}^{|V|}\sum_{j=0}^{i} d_{i,j}q_iq_j \quad \cdots (18)$$

In the Hamiltonian expressed by Expression (18), the maximum distance between residues to be selected is not limited. The constraint conditions represented by the second term on the right side of Expression (18) indicate that a certain residue set can be more easily selected as the sum of the distances between residues of the residue set becomes smaller. Accordingly, with the Hamiltonian expressed by Expression (18), a certain residue set can be more easily selected as the sum of the allosteric scores of the residues included in the residue set becomes larger and as the sum of the distances between residues becomes smaller.

Thereafter, the residue set search unit 140d performs the process of Step S1820.

Step S1820: The second Ising calculation unit 142d performs Ising calculation based on a simulated bifurcation machine on the Hamiltonian generated by the second Hamiltonian generating unit 141d.

In this way, the residue set search unit 140d ends the residue set search and Ising calculation process.

Description of the residue set search process will be continued with reference back to FIG. 37.

Step S1750: The third evaluation unit 16d evaluates the residue set on the basis of the residue set score calculated by the residue set search unit 140d. The residue set score corresponds to energy indicated by the Hamiltonian expressed by Expression (16), and a lower value thereof means that the residue set is evaluated to be higher as a path set. For example, the third evaluation unit 16d determines a residue set of which the evaluation value based on the residue set score is the highest. The third evaluation unit 16d may determine a predetermined number of residue sets in the descending order of the evaluation values based on the residue set scores. The third evaluation unit 16d may determine residue sets of which the evaluation value based on the residue set score is higher than a predetermined value.

Here, a residue set corresponds to a path set which is a set of paths connecting a residue included in the residue set and a residue included in the active center. Accordingly, the evaluation function is a function of giving an evaluation value of the path set.

FIG. 41 illustrates an example of a residue set R3 which is determined as a residue set with the highest evaluation value as the outline of the residue set search result. The residue set R3 corresponds to an allosteric control region.

In this way, the control unit 10d ends the residue set search process.

Description of the allosteric path predicting process will be continued with reference back to FIG. 36.

Step S1560: The output unit 15d outputs a prediction result of the residue set search process. The output unit 15d outputs information indicating one or more residue sets determined by the third evaluation unit 16d and the evaluation result from the third evaluation unit 16d as the prediction result. The information indicating a residue set includes, for example, information indicating residues included in the residue set which are start points of an allosteric path and information indicating residues included in the active center which are end points of the allosteric path.

In this way, the control unit 10d ends the allosteric path predicting process.

(Example of modified example of third embodiment)

In an example of the third embodiment, an allosteric score of I36 located at the center of a binding region with RAF which is an effector protein is calculated using a crystal structure of PDB ID: 4obe of normal KRAS. In the present example, search for an allosteric control region for controlling a reaction of KRAS with an effector is performed using the allosteric score calculated in the example of the third embodiment.

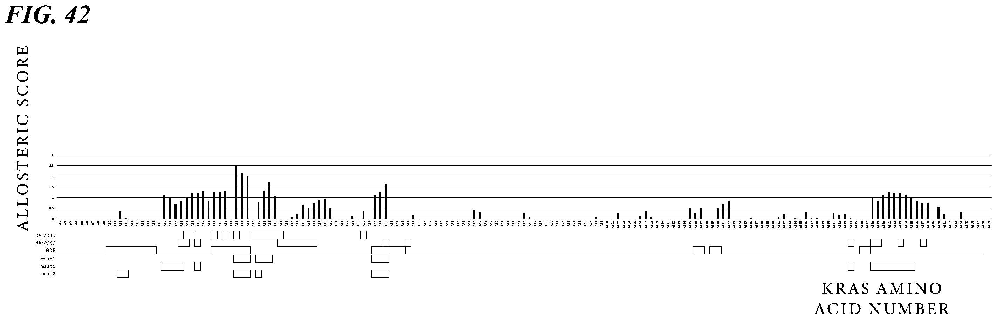

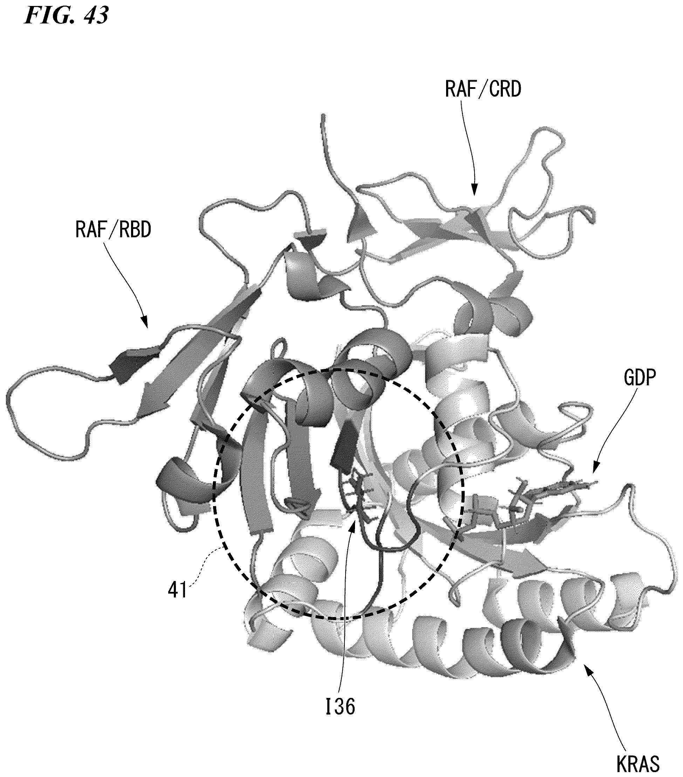

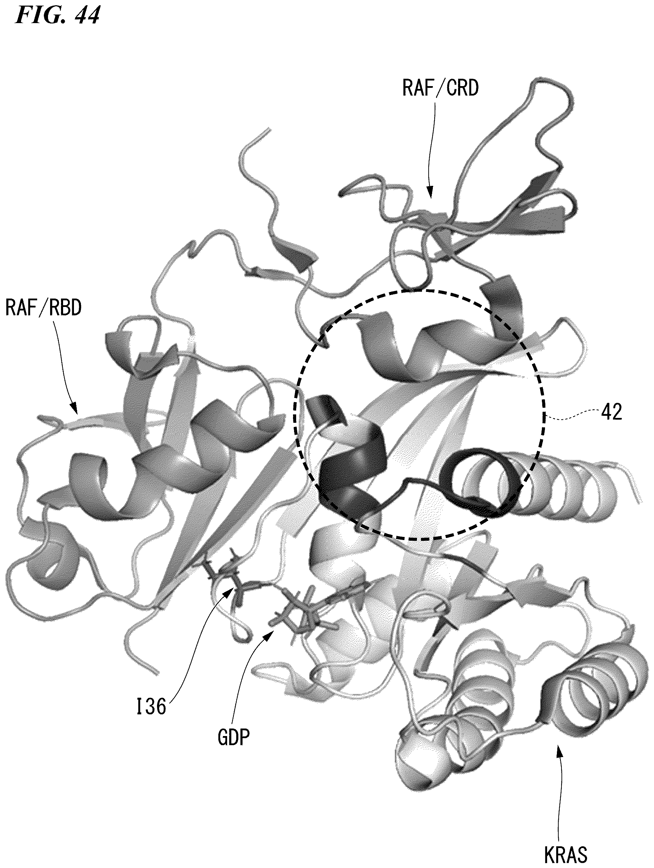

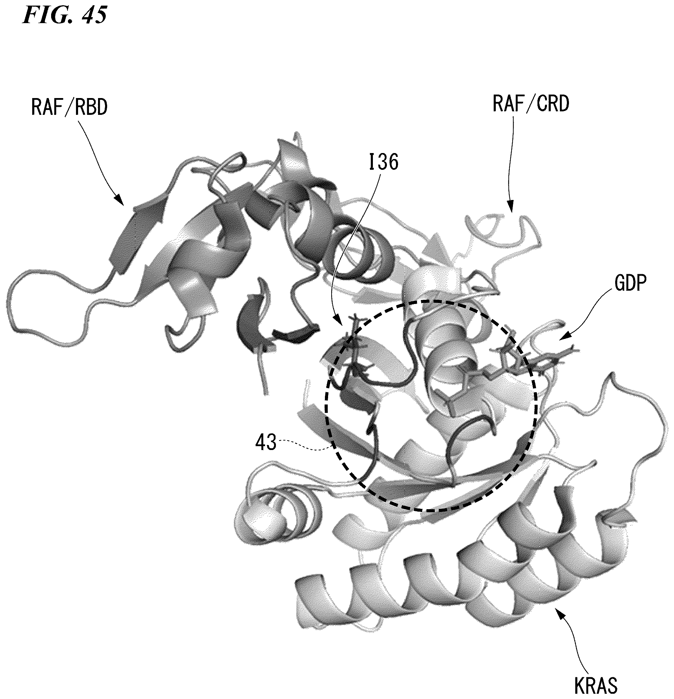

Search results of an allosteric control region according to the present example are illustrated in FIGS. 42 to 45. In FIG. 42, an allosteric score for each amino acid residue of KRAS is indicated by a bar graph. In FIG. 42, amino acid residues constituting an allosteric control region obtained in the present example are illustrated as "result 1," "result 2," and "result 3." FIGS. 43, 44, and 45 are diagrams illustrating ribbon models of KRAS/RAF composites corresponding to "result 1," "result 2," and "result 3." In FIGS. 43, 44, and 45, a searched region 41, amino acids constituting a searched region 42, and a searched region 43 are all filled.

KRAS and RAF are bound to each other in two domains (RBD and CRD), and a reaction between KRAS and RAF is controlled through replacement of GDP/GTP. In the present example, an allosteric control region which is a residue set including an I36 residue located at the center of a binding region between KRAS and RAF/RBD as an end point and a plurality of start points connected thereto through paths with a large influence on I36 is searched for. A search region in "result 1" is a region associated with binding between KRAS and RAF/RBD (in the vicinity of a predetermined vertex). A search region in "result 2" is a region associated with binding between KRAS and RAF/RRD. A search region in "result 3" is a binding region between GDP and KRAS. Three regions searched in "result 1," "result 2," and "result 3" are regions which are known to be regions important in a reaction between KRAS and RAF. Accordingly, it is confirmed that this allosteric control region search method is a method which is effective for identifying an allosteric control region for an interaction between KRAS and RAF.

(Fourth embodiment)

A fourth embodiment of the present invention will be described below in detail with reference to the drawings.

In the first to third embodiments, a case in which an allosteric path is a single path has been described. In the present embodiment, it is assumed that an allosteric path is a multiplex path. A multiplex path is conceptually included as a partial graph of a network graph along with a single path. In a multiplex path, the path may bifurcate or join. In a multiplex path, the path may include a plurality of start points or a plurality of end points.

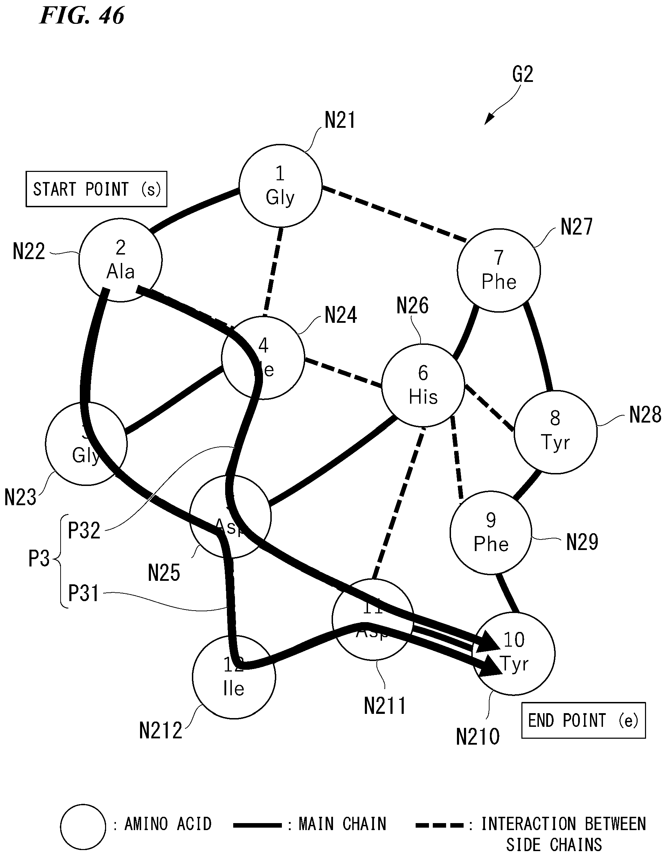

An example of a multiplex path is illustrated in FIG. 46. A path P3 is a multipilex path from a start point s to an end point e. The path P3 includes a path P31 and a path P32. The path P31 passes through a vertex N22 (start point s), a vertex N23, a vertex N25, a vertex N212, a vertex N211, and a vertex N210 (end point e) in this order. The path P32 passes through the vertex N22 (start point s), a vertex N24, the vertex N25, the vertex N211, and the vertex N210 (end point e) in this order. Accordingly, the path P3 is a multiplex path which bifurcates to the path P31 and the path P32 at the vertex N22 (start point s), joins at the vertex N25, bifurcates again, and joins again at the vertex N211. As described above, a multiplex path includes a bifurcation or a junction.

[Functional configuration of allosteric path prediction device 1c]

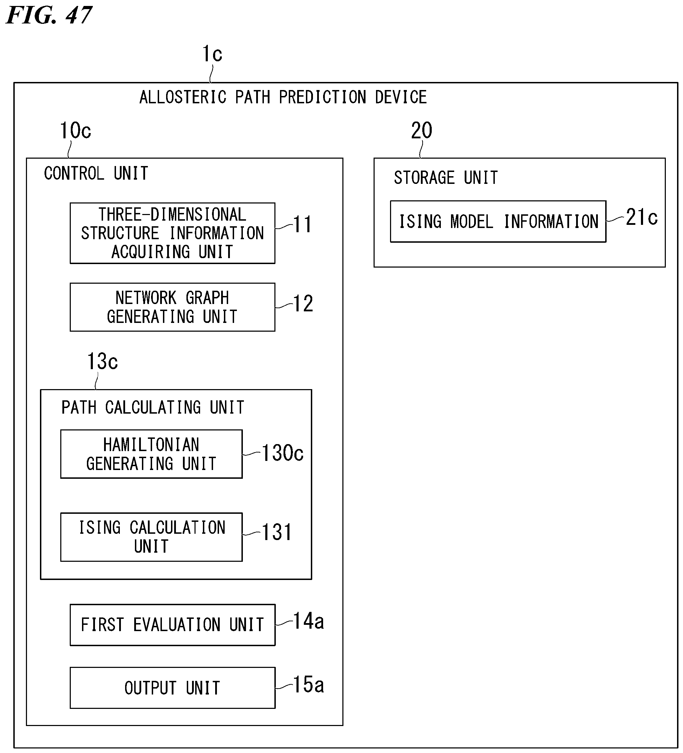

FIG. 47 is a diagram illustrating an example of the functional configuration of a allosteric path prediction device 1c according to the present embodiment. The allosteric path prediction device 1c includes a control unit 10c and a storage unit 20. The allosteric path prediction device 1c according to the present embodiment (FIG. 47) and the allosteric path prediction device 1a according to the second embodiment (FIG. 15) are different from each other in the control unit 10c and Ising model information 21c stored in the storage unit 20. A description of the same functions as in the second embodiment will be omitted, and differences from the second embodiment will be mainly described in the fourth embodiment.

The control unit 10c includes a three-dimensional structure information acquiring unit 11, a network graph generating unit 12, a path calculating unit 13c, a first evaluation unit 14a, and an output unit 15a. The control unit 10c (FIG. 47) and the control unit 10a (FIG. 15) are different from each other in the path calculating unit 13c. Here, the functions of the other elements (the three-dimensional structure information acquiring unit 11, the network graph generating unit 12, the first evaluation unit 14a, and the output unit 15a) are the same as those in the second embodiment.

The path calculating unit 13c includes a Hamiltonian generating unit 130c and an Ising calculation unit 131.

The function of the Hamiltonian generating unit 130c is the same as the function of the Hamiltonian generating unit 130 except that the Hamiltonian to be generated is a Hamiltonian for a multiplex path. An objective function and constraint conditions used for the Hamiltonian generating unit 130c to generate a Hamiltonian are stored as Ising model information 21c in the storage unit 20 in advance. The constraint conditions indicated by the Ising model information 21c are constraint conditions for a multiplex path.

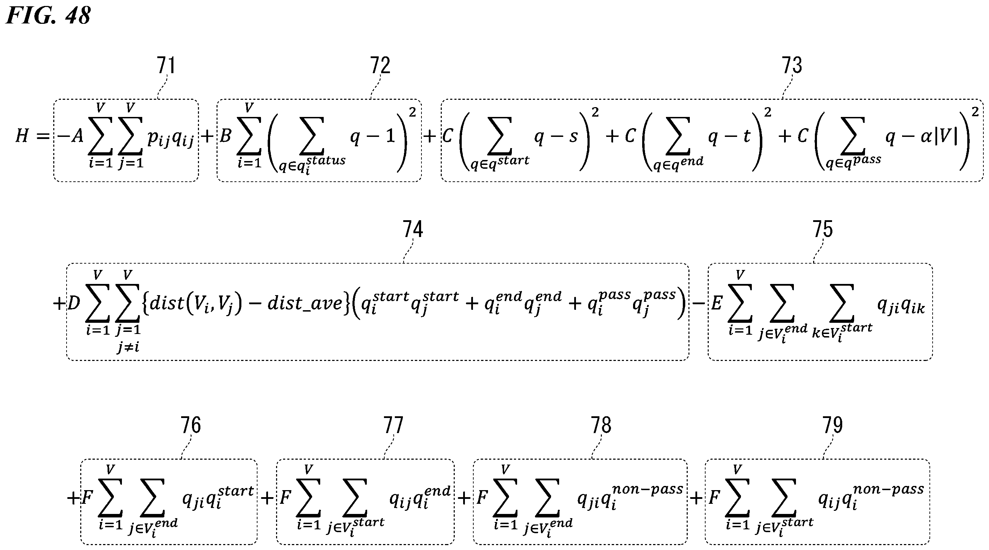

A Hamiltonian for a multiplex path which is generated by the Hamiltonian generating unit 130c will be described below with reference to FIG. 48. An example of the Hamiltonian generated by the Hamiltonian generating unit 130c is expressed by Expression (14). In Expression (14), A, B, C, D, E, and F are constants.

[Math. 19]

$$H = -A \sum_{i=1}^{V} \sum_{j=1}^{V} p_{ij} q_{ij} + B \sum_{i=1}^{V} \left( \sum_{q \in q_i^{status}} q - 1 \right)^2 + C \left( \sum_{q \in q^{start}} q - s \right)^2 + C \left( \sum_{q \in q^{end}} q - t \right)^2 + C \left( \sum_{q \in q^{pass}} q - \alpha|V| \right)^2$$

$$+ D \sum_{i=1}^{V} \sum_{\substack{j=1 \\ j \neq i}}^{V} \{dist(V_i, V_j) - dist\_ave\} \left( q_i^{start} q_j^{start} + q_i^{end} q_j^{end} + q_i^{pass} q_j^{pass} \right) - E \sum_{i=1}^{V} \sum_{j \in V_i^{end}} \sum_{k \in V_i^{start}} q_{ji} q_{ik}$$

$$+ F \sum_{i=1}^{V} \sum_{j \in V_i^{end}} q_{ji} q_i^{start} + F \sum_{i=1}^{V} \sum_{j \in V_i^{start}} q_{ij} q_i^{end} + F \sum_{i=1}^{V} \sum_{j \in V_i^{end}} q_{ji} q_i^{non-pass} + F \sum_{i=1}^{V} \sum_{j \in V_i^{start}} q_{ij} q_i^{non-pass}$$

$$\cdots (19)$$

In the Hamiltonian for a single path expressed by Expression (6), only a spin $q_{ij}$ corresponding to a edge connecting a vertex $V_i$ and a vertex $V_j$ is present as a spin. In the Hamiltonian for a multiplex path expressed by Expression (19), a spin indicating a status of a vertex as well as $q_{ij}$ is present as a spin. The spin indicating a status of a vertex includes four spins of $q_i^{start}$, $q_i^{end}$, $q_i^{pass}$, and $q_i^{non-pass}$. $q_i^{start}$ has a value of 1 when the vertex is a start point and a value of 0 when the vertex is not a start point. $q_i^{end}$ has a value of 1 when the vertex is an end point and a value of 0 when the vertex is not an end point. $q_i^{pass}$ has a value of 1 when the vertex is a pass point and a value of 0 when the vertex is not a pass point. $q_i^{non-pass}$ has a value of 1 when the vertex is a non-pass point and a value of 0 when the vertex is not a non-pass point.

As illustrated in FIG. 48, term 71 is an objective function. Term 72, term 73, term 74, term 75, term 76, term 77, term 78, and term 79 are constraint conditions. Term 72 and term 73 in the constraint conditions are constraint conditions required in graph theory. Term 74, term 75, term 76, term 77, term 78, and term 79 in the constraint conditions are constraint conditions required from biological aspects.

In Expression (19), the objective function is a sum of scores of edges employed by a path. In Expression (19), $p_{ij}$ is used as a weight assigned to the edge connecting the vertex $V_i$ and the vertex $V_j$.

Term 72 represents constraint conditions in which a status of each vertex is one of a start point, an end point, a pass point, and a non-pass point. $q_i^{status}$ is a set of spins (that is, $q_i^{start}$, $q_i^{end}$, $q_i^{pass}$, and $q_i^{non-pass}$) associated with statuses of the vertex $V_i$.

Term 73 represents constraint conditions for designating a multiplicity of a path. The multiplicity of a path is expressed by the number of start points, the number of end points, and the number of pass points. The multiplicity of a path is also referred to as a percentage of the number of start points, the number of end points, and the number of pass points. Parameter $\alpha$ is a parameter affecting the number of pass points to be designated and is a ratio of the total number of vertices to the number of pass points.

Term 74 represents constraint conditions for decreasing a distance between the same type of vertices as much as possible (also referred to as collection). Term 74 includes conditions for decreasing a distance between start points as much as possible, conditions for decreasing a distance between end points as much as possible, and conditions for decreasing a distance between pass points as much as possible. The sign of the constraint conditions represented by Term 74 is negative when the distance between vertices is smaller than an average value of the distances between vertices and is positive when the distance between vertices is larger than the average value of the distances between vertices. The absolute value of the constraint conditions represented by term 74 becomes lager as the number of vertices in the same status becomes larger.

Term 75 represents constraint conditions for causing a path to pass through a vertex with the larger number of inflows or outflows.

Term 76 represents constraint conditions in which a start point does not serve as an end point. Term 77 represents constraint conditions in which an end point does not serve as a start point. Term 78 represents constraint conditions in which a non-pass point does not serve as an end point. Term 79 represents constraint conditions in which a non-pass point does not serve as a start point.

One or more of term 74, term 75, term 76, term 77, term 78, and term 79 which are constraint conditions required from biological aspects may be omitted from the Hamiltonian expressed by Expression (19). Constraint conditions other than term 74, term 75, term 76, term 77, term 78, and term 79 may be added as constraint conditions required from biological aspects. That is, the Hamiltonian expressed by Expression (19) is an example, and the Hamiltonian may be changed (customized). Customization of a Hamiltonian will be described later in a fifth embodiment.

(First example of fourth embodiment)

As described above in the second example of the second embodiment, a reaction of KRAS with an effector protein is allosterically controlled by GDP/GTP. In such allosteric control, the amino acid residue search result using the Hamiltonian for a single path has been described in the second example of the second embodiment. In the present example, whether the same calculation as on a combination of single paths can be performed is ascertained using the Hamiltonian for a multiplex path.

In the present example, an allosteric path predicting process using A14 as a start point, using A36 as an end point, and using a Hamiltonian for a multiplex path is performed on KRAS structure information (PDB ID: 4obe). In the present example, a linear term in which a designated start point and a designated end point become advantageous is added to the Hamiltonian for a multiplex path expressed by Expression (19), and start points and end points are designated. The calculated path is a multiplex path having a bifurcation and a junction in the middle of the path. Amino acid residues included in the multiplex path are compared with results acquired from a combination of signal paths, and validity of the allosteric path predicting process using the Hamiltonian for a multiplex path is evaluated.

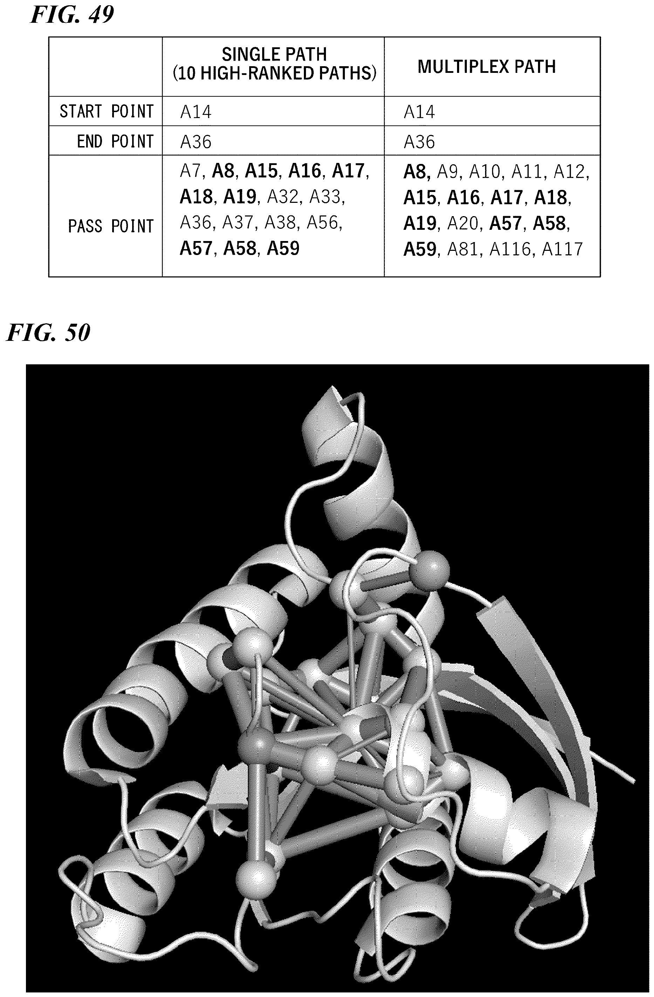

Search results of important amino acid residues according to the present example are illustrated in FIG. 49. In FIG. 49, search results of important amino acid residues based on a combination of single paths as a comparative example are illustrated along with the search results using the Hamiltonian for a multiplex path according to this example. An optimal path (in which a bifurcation and a junction are allowed) calculated using the Hamiltonian for a multiplex path passes through the amino acid residues searched for using the Hamiltonian for a signal path in the same way. The amino acid residues searched for using the Hamiltonian for a single path include A8, A15 to A18, and A57 to A59. In FIG. 50, allosteric paths which are calculated using the Hamiltonian for a multiplex path are illustrated. In FIG. 50, the allosteric paths are superimposed on a three-dimensional structure of a protein.

The search results according to the present example indicates that amino acid residues which are important in allosteric control can be identified using only an optimal solution to a Hamiltonian by employing the Hamiltonian for a multiplex path.

(Second example of fourth embodiment)

As described above in the example of the third embodiment, a reaction of KRAS with an effector protein is allosterically controlled by GDP/GTP. In such allosteric control, the allosteric control region search result using the Hamiltonian for a single path has been described in the example of the third embodiment. In the present example, it is ascertained that an allosteric control region can be searched for using the Hamiltonian for a multiplex path.

In the present example, the allosteric control region predicting process using I36 as an end point and using the Hamiltonian for a multiplex path is performed on KRAS structure information (PDB ID: 4obe). In the present example, a start point is not designated. The calculated path is a multiplex path having a bifurcation and a junction at a plurality of start points and in the middle of the path. By comparing the plurality of selected start points with an existing allosteric control region of KRAS, validity of the allosteric control region predicting process using the Hamiltonian for a multiplex path is evaluated.

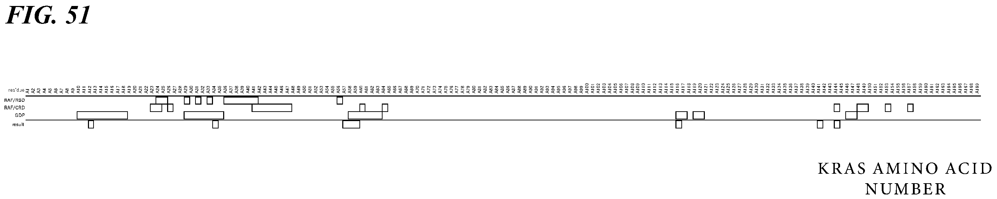

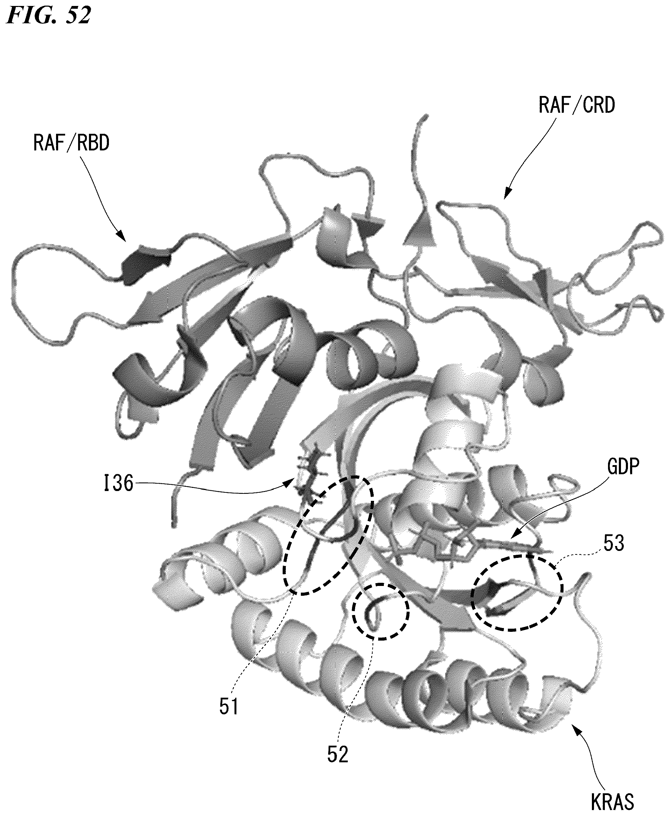

Search results of an allosteric control region according to the present example are illustrated in FIGS. 51 and 52. In FIG. 51, a region which is important in a reaction of KRAS with RAF which is one efffector protein and start points acquired in the present example are illustrated as "result." FIG. 52 is a diagram illustrating a ribbon model of KRAS/RAF composite corresponding to the "result." In FIG. 52, amino acids constituting the start points of the searched-out paths are all painted in a region 51, a region 52, and a region 53. The amino acids constituting the start points of the searched-for paths indicate binding regions between GDP and KRAS. This result matches that a reaction of KRAS with an effector protein is allosterically controlled by

GDP/GTP.

The search results in the present example indicate that an allosteric control region can be identified using only an optimal solution to a Hamiltonian by using the Hamiltonian for a multiplex path.

In the present example, a case in which the same processes as the important amino acid residue search process described in the second embodiment and the region search process described in the modified example of the third embodiment are performed has been described, but the present invention is not limited thereto. In the allosteric path predicting process described in the first embodiment or the region search process described in the third embodiment, the processes may be performed using the Hamiltonian for a multiplex path instead of the Hamiltonian for a single path.

In the allosteric path prediction devices 1, 1a, 1b, and 1c according to the aforementioned embodiments, since the Ising calculation is performed using a simulated bifurcation machine as described above, it is possible to faster perform the Ising calculation in comparison with a case in which the simulated bifurcation machine is not used. Since the calculation speed is high, it is possible to repeatedly perform the allosteric path predicting process in a practical time. In the allosteric path prediction devices 1, 1a, 1b, and 1c, it is possible to perform comprehensive search by repeatedly performing the allosteric path predicting process.

Comprehensive search is, for example, calculation of a set of candidates for an end point with respect to a certain start point (an end point candidate set) and a set of candidates for a start point with respect to a certain end point (a start point candidate set). For example, the end point candidate set with respect to all start points included in a network graph may be calculated while changing the start point variously. For example, the start point candidate set with respect to all end points included in the network graph may be calculated while changing the end point variously.

For example, the process of calculating a set of candidates for an important amino acid residue (an important amino acid residue candidate set) with respect to a predetermined start point and a predetermined end point has been described above in the second embodiment, but this process may be repeatedly performed on all sets of a start point and an end point included in the network graph, and the important amino acid residue candidate set may be correlated with all the sets of a start point and an end point included in the network graph. The important amino acid residue candidate set correlated with all the sets of a start point and an end point included in the network graph may be stored as whole information on allosteric control of a target protein in a database. Storage in the database will be described later in the fifth embodiment.

In this embodiment, an example in which a Hamiltonian for a multiplex path is used has been described above, but the Hamiltonian for a multiplex path may be repeatedly used in comprehensive search, or the Hamiltonian for a single path may be repeatedly used therein. A process using the Hamiltonian for a multiplex path and a process using the Hamiltonian for a single path may be combined and used.

In the allosteric path prediction devices 1, 1a, 1b, and 1c according to the aforementioned embodiments, a case in which the functional units of the control units 10, 10a, 10b, and 10c or the storage unit 20 are realized as a function of one computer has been described, but the present invention is not limited thereto. The functional units of the control units 10, 10a, 10b, and 10c or the storage unit 20 may be distributed and provided, for example, in a plurality of servers. The functional units of the control units 10, 10a, 10b, and 10c or the storage unit 20 may be realized by a cloud server. When the functional units of the control units 10, 10a, 10b, and 10c or the storage unit 20 are distributed and provided in a plurality of servers or when they are realized as a cloud server, the plurality of servers or the cloud server may be distributed and provided in different countries.

In the fifth embodiment which will be described below, it is assumed that the allosteric path prediction device is realized as a plurality of servers. In the fifth embodiment, details of a user interface will be described.

(Fifth embodiment)

[Configuration of allosteric path prediction system 3]

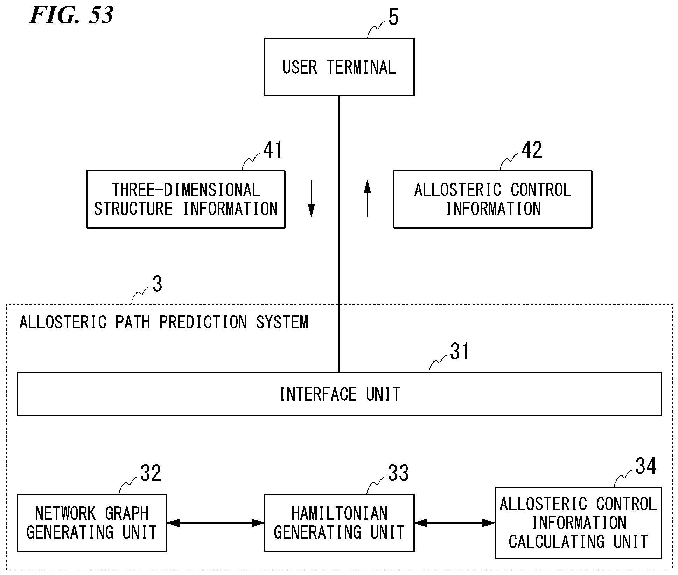

FIG. 53 is a diagram illustrating an example of a configuration of an allosteric path prediction system 3 according to the present embodiment. The allosteric path prediction system 3 includes an interface unit 31, a network graph generating unit 32, a Hamiltonian generating unit 33, and an allosteric control information calculating unit 34. For example, the interface unit 31, the network graph generating unit 32, the Hamiltonian generating unit 33, and the allosteric control information calculating unit 34 are servers.

The interface unit 31 performs inputting and outputting of various types of information, pre-processing, and post-processing.

The interface unit 31 acquires input information from a user terminal 5. The input information is information which is input to the user terminal 5 by a user. The input information includes, for example, three-dimensional structure information 41. The three-dimensional structure information 41 is information indicating a three-dimensional structure of a protein. The user terminal 5 is a terminal device such as a PC which is used by a user.

The interface unit 31 performs pre-processing of the input information. The pre-processing includes a process of converting information input from a user into a format which can be processed by the allosteric path prediction system 3.

The interface unit 31 performs post-processing of a prediction result from the allosteric path prediction system 3. The post-processing includes a process of converting the prediction result into a format desired by a user. The prediction result includes allosteric control information 42. The allosteric control information 42 includes allosteric path information and one or more of an allosteric path score, a residue score, and an allosteric score.

The interface unit 31 outputs the prediction result to the user terminal 5.

The network graph generating unit 32 generates a network graph on the basis of the three-dimensional structure information 41. The network graph generating unit 32 has the same function as the network graph generating unit 12.

The Hamiltonian generating unit 33 generates a Hamiltonian on the basis of the network graph generated by the network graph generating unit 32. The Hamiltonian generating unit 33 has the same function as the Hamiltonian generating unit 130.

The allosteric control information calculating unit 34 performs Ising calculation based on a simulated bifurcation machine on the Hamiltonian generated by the Hamiltonian generating unit 130. The allosteric control information calculating unit 34 has the same function as the Ising calculation unit 131. The allosteric control information calculating unit 34 generates the allosteric control information 42 on the basis of an execution result of the Ising calculation.

The user terminal 5 acquires the prediction result from the allosteric path prediction system 3 output from the interface unit 31. That is, the user terminal 5 acquires a calculation result of a path connecting vertices in the network graph. The user terminal 5 and the allosteric path prediction system 3 may be provided in different countries.

[Allosteric path predicting process in allosteric path prediction system 3]

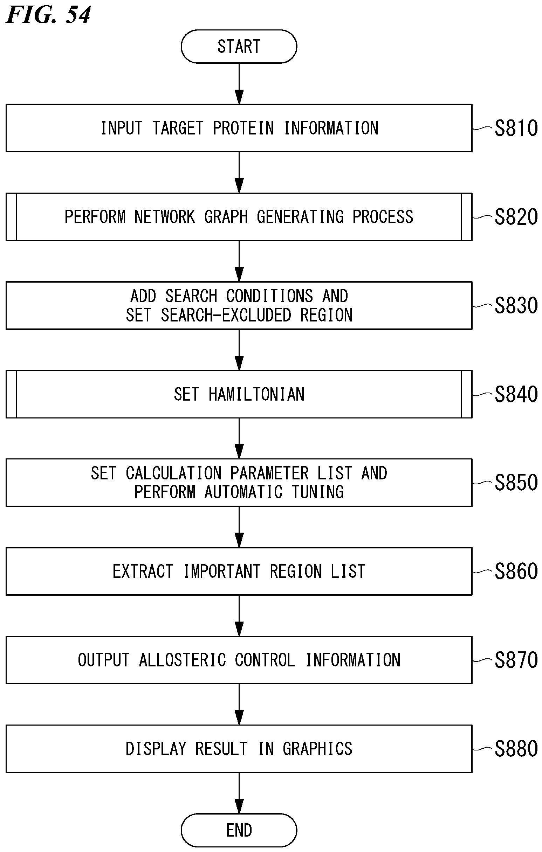

The allosteric path predicting process in the allosteric path prediction system 3 will be described below with reference to FIGS. 54 to 56. FIG. 54 is a diagram illustrating an example of the allosteric path predicting process according to the present embodiment.

Step S810: The interface unit 31 acquires the three-dimensional structure information 41 as input information from the user terminal 5. The three-dimensional structure information 41 is three-dimensional structure information on a protein in which an allosteric path is to be predicted. Thereafter, the allosteric path prediction system 3 performs the process of Step S820.

Step S820: The network graph generating unit 32 performs a network graph generating process. Thereafter, the network graph generating unit 32 performs the process of Step S830.

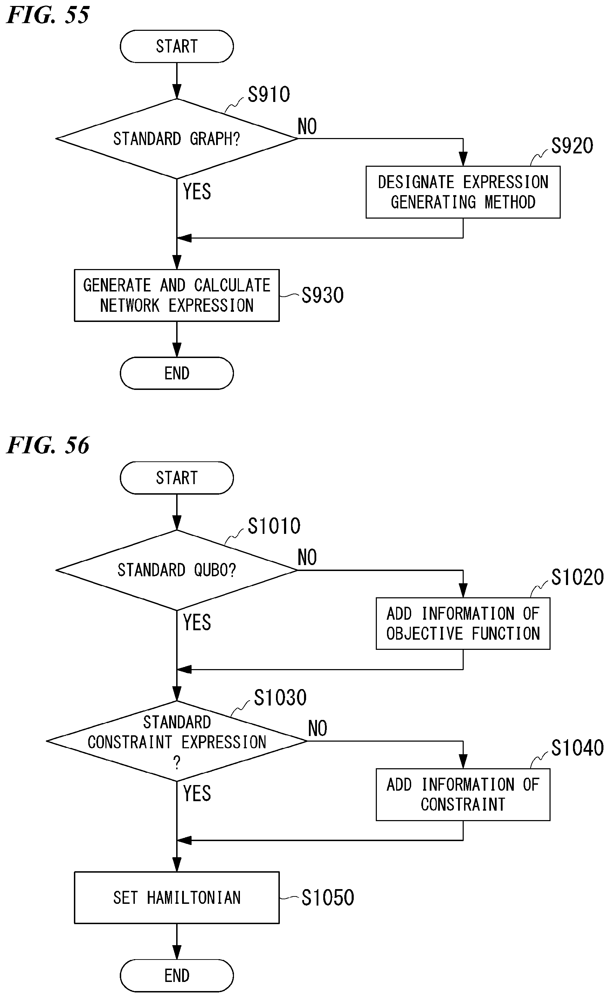

The network graph generating process will be described below with reference to FIG. 55. FIG. 55 is a diagram illustrating an example of the network graph generating process according to the present embodiment. The network graph generating process illustrated in FIG. 55 is performed in Step S820 illustrated in FIG. 54.

Step S910: The network graph generating unit 32 determines whether the generated network graph is a standard graph. The network graph generating unit 32 determines whether the network graph is a standard graph on the basis of graph setting information. The graph setting information includes information indicating the type of the network graph. The graph setting information may be acquired from the user terminal 5 by the interface unit 31 in Step S920 or may be acquired from the user terminal 5 along with the input information by the interface unit 31 in Step S810.

When it is determined that the graph setting information indicates a standard graph (Step S910: YES), the network graph generating unit 32 performs the process of Step S930. On the other hand, when it is determined that the graph setting information does not indicate a standard graph (Step S910: NO), the network graph generating unit 32 performs the process of Step S920.

Step S920: The network graph generating unit 32 designates an expression generation method of the generated network graph. Information indicating the expression generation method is included in the graph setting information. The network graph generating unit 32 designates the expression generation method on the basis of the graph setting information. The graph setting information includes, for example, information for setting weights other than weights indicated by a transmission probability matrix as weights assigned to sides of the network graph. The information is an expression for calculating weights other than the weights indicated by the transmission probability matrix. Thereafter, the network graph generating unit 32 performs the process of Step S930.

Step S930: The network graph generating unit 32 generates a network graph on the basis of the three-dimensional structure information 41. The process of causing the network graph generating unit 32 to generate a network graph includes processes of generating vertices corresponding to amino acid residues, an interaction matrix, and a signal transmission matrix. When the expression generation method is designated, the network graph generating unit 32 calculates the weights other than the weights indicated by the transmission probability matrix.

In this way, the network graph generating unit 32 ends the network graph generating process.

Description of the allosteric path predicting process will be continued with reference back to FIG. 54.

Step S830: The network graph generating unit 32 performs addition of search condition and setting of a search-excluded region. Search condition information includes the aforementioned site designation information (which is information indicating an allosteric control site and an allosteric control target site and thus information indicating a start point and an end point, where the number of start points and the number of end points does not have to be one and each of the start point and the end point may be a set of points including a plurality of points), information indicating additional search conditions, and information indicating the search-excluded region. The additional search conditions are search conditions which are designated by a user. The search-excluded region is a region which is excluded from search in a three-dimensional structure of a protein indicated by the three-dimensional structure information 41. The search-excluded region is designated by a user. The search condition information may be acquired from the user terminal 5 by the interface unit 31 in Step S830 or may be acquired from the user terminal 5 along with the input information by the interface unit 31 in Step S810.

When the information indicating additional search conditions is included in the search condition information, the network graph generating unit 32 adds the additional search conditions to the search conditions. When the information indicating a search-excluded region is included in the search condition information, the network graph generating unit 32 excludes the search-excluded region indicated by the information from search. Thereafter, the Hamiltonian generating unit 33 performs the process of Step S840.

The interface unit 31 may change an interface to a format desired by a user according to the user's settings. For example, the interface unit 31 may change the interface such that information indicating a domain is designated instead of the site designation information included in the search condition information. A domain is a unit indicating a part of a structure in a three-dimensional structure of a protein.

Step S840: The Hamiltonian generating unit 33 performs setting of a Hamiltonian. Thereafter, the allosteric control information calculating unit 34 performs the process of Step S850.

A Hamiltonian setting process will be described below with reference to FIG. 56. FIG. 56 is a diagram illustrating an example of the Hamiltonian setting process according to the present embodiment. The Hamiltonian setting process illustrated in FIG. 56 is performed in Step S840 illustrated in FIG. 54.

Step S1010: The Hamiltonian generating unit 33 determines whether use of a standard QUBO as a Hamiltonian to be generated is designated. The Hamiltonian generating unit 33 determines whether use of a standard QUBO as the Hamiltonian is designated on the basis of Hamiltonian designation information. The Hamiltonian designation information includes information for designating a Hamiltonian. The Hamiltonian designation information is designated by a user. The Hamiltonian designation information may be acquired from the user terminal 5 by the interface unit 31 in Step S1010 or may be acquired from the user terminal 5 along with the input information by the interface unit 31 in Step S810.

When it is determined that use of a standard QUBO as the Hamiltonian to be generated is designated (Step S1010: YES), the Hamiltonian generating unit 33 performs the process of Step S1030. On the other hand, when it is determined that a standard QUBO is not used as the Hamiltonian to be generated (Step S1010: NO), the Hamiltonian generating unit 33 performs the process of Step S1020.

Step S1020: The Hamiltonian generating unit 33 adds information for designating a calculation expression indicated by the Hamiltonian designation information to Hamiltonian information. The calculation expression may be a quadratic expression based on an Ising model or may be a high-powered expression to which the Ising model is extended, a rational expression, or a general real-valued function. The Hamiltonian information is information including an objective function and constraint conditions as described above and is stored in advance in the storage unit.

For example, when a calculation expression designated by a user is included in the Hamiltonian designation information, the Hamiltonian generating unit 33 adds the calculation expression to the Hamiltonian information. For example, when information for designating a calculation rule of weights assigned to sides of a network graph is included in the Hamiltonian designation information, the Hamiltonian generating unit 33 adds the information to the Hamiltonian information. The calculation rule of weights means whether to multiply a weight $p_{ij}$ by a plurality of edges (whether to include a logarithm of $p_{ij}$ in the objective function) or whether to add the weight $p_{ij}$ to the plurality of edges (whether to include $p_{ij}$ itself instead of the logarithm $p_{ij}$ in the objective function). Thereafter, the Hamiltonian generating unit 33 performs the process of Step S1030.

Step S1030: The Hamiltonian generating unit 33 determines whether use of a standard constraint expression as a term of a constraint expression of the Hamiltonian to be generated is designated. The Hamiltonian generating unit 33 determines whether the term of the constraint expression is a standard constraint expression on the basis of constraint expression designation information. The constraint expression designation information is designated by a user. The constraint expression designation information includes information for designating a constraint expression. A constraint expression may indicate constraint conditions on a shape of a partial graph of the network graph. The constraints on a shape of a partial graph of the network graph include a constraint in which a path without a bifurcation and an inflow is formed between one of the start point set to one of the end point set or a constraint on a distance between vertices associated with a partial graph. The constraint expression designation information may be acquired from the user terminal 5 by the interface unit 31 in Step S1030 or may be acquired along with the input information from the user terminal 5 by the interface unit 31 in Step S810.

When it is determined that use of a standard constraint expression as a term of a constraint expression of the Hamiltonian to be generated is designated (Step S1030: YES), the Hamiltonian generating unit 33 performs the process of Step S1050. On the other hand, when it is determined that a standard constraint expression is not used as a term of a constraint expression of the Hamiltonian to be generated is designated (Step S1030: NO), the Hamiltonian generating unit 33 performs the process of Step S1040.

Step S1040: The Hamiltonian generating unit 33 adds information for designating a constraint expression indicated by the constraint expression designation information to the Ising model information. Thereafter, the Hamiltonian generating unit 33 performs the process of Step S1050.

Step S1050: The Hamiltonian generating unit 33 generates a Hamiltonian on the basis of the site designation information, information indicating a search-excluded region, and the Hamiltonian information. When information for designating a calculation expression designated by a user is included in the Hamiltonian information, the Hamiltonian generating unit 33 generates the Hamiltonian on the basis of the information. When information for designating a constraint expression designated by a user is included in the Hamiltonian information, the Hamiltonian generating unit 33 generates a constraint expression on the basis of the information. When information designated by a user is not included in the Hamiltonian information, the Hamiltonian generating unit 33 generates the Hamiltonian on the basis of the standard QUBO indicated by the Hamiltonian information stored in advance and the standard constraint expression. The Hamiltonian may be expressed in the form of an inner product of a vector including one or more indicators which is preferable to have a small value and a vector for applying a weight to each indicator at the time of search for an important region in calculating allosteric control information. The Hamiltonian may be designed such that a large value is desirable, and a partial graph with a larger value of the Hamiltonian through optimization in Step S860 may be searched for. In this way, the Hamiltonian generating unit 33 ends the Hamiltonian setting process.

Description of the allosteric path predicting process will be continued with reference back to FIG. 54.

Step S850: The allosteric control information calculating unit 34 performs setting of a calculation parameter list and automatic tuning. The calculation parameter list includes hyperparameters used to perform optimization calculation using a simulated bifurcation machine and constants applied to terms included in the Hamiltonian set by the Hamiltonian generating unit 33. In the automatic tuning, a combination of values in which the optimization calculation using a simulated bifurcation machine can be appropriately performed is calculated and set for the plurality of parameters. The automatic tuning is performed in a conditional bifurcation or the like, for example, from characteristics of a target protein and the network graph. Alternatively, the automatic tuning may be performed according to an optimization technique such as Bayes optimization. Thereafter, the allosteric control information calculating unit 34 performs the process of Step S860.

Step S860: The allosteric control information calculating unit 34 performs the optimization calculation on the Hamiltonian generated by the Hamiltonian generating unit 33. The optimization calculation is calculation of correlating a partial graph of the network graph with the Hamiltonian indicating a degree of association of allosteric control with a designated allosteric control site and an allosteric control target site, searching for a partial graph in which the value of the Hamiltonian is small, and thus calculating a set of partial graphs which are likely to be associated with allosteric control.

The allosteric control information calculating unit 34 may use an optimization calculation means which is called an Ising machine as a means for searching for a partial graph in which the value of the Hamiltonian is small or may extend the Ising machine and use software which can handle a high-powered expression, a rational expression, or a general real-valued function. The calculation may be performed on the basis of suboptimal solutions which are given as a result with a probabilistic change. A simulated bifurcation machine may be used as the calculation means. The allosteric control information calculating unit 34 may calculate a plurality of candidates for a solution by repeatedly performing an Ising machine. The allosteric control information calculating unit 34 may calculate a plurality of candidates for a solution to the Hamiltonian representing optimization for a path set. The path set is a set of a plurality of paths including suboptimal paths in addition to an optimal path.

The allosteric control information calculating unit 34 may calculate a set of k partial graphs with the smallest values of the Hamiltonian using a natural number k as a threshold value or may rank multiplications of the Hamiltonian by a weight predetermined for each partial graph and calculate a set of partial graphs in the order of rankings. Another threshold value m may be determined, and a partial graph in which the Hamiltonian value is smaller than the threshold value m. The allosteric control information calculating unit 34 supplies the calculation result to the interface unit 31. Thereafter, the interface unit 31 performs the process of Step S870.

Step S870: The interface unit 31 outputs allosteric control information. For example, the interface unit 31 outputs the allosteric control information to the user terminal 5. The interface unit 31 may store the allosteric control information in a database (which is not illustrated in FIG. 53). Thereafter, the interface unit 31 performs the process of Step S880.

Here, the allosteric control information is generated by the interface unit 31 as post-processing. For example, the allosteric control information calculating unit 34 arranges the calculated partial graphs in the descending order of evaluations values calculated from the Hamiltonian (allosteric path scores) on the basis of the calculation result from the allosteric control information calculating unit 34 and generates the allosteric control information with a partial graph and an evaluation value of the corresponding path as a set.

Only partial graphs of which the type is a single graph may be selected. For example, the allosteric control information calculating unit 34 arranges the vertices included in a plurality of partial graph with high evaluation values in the descending order of evaluation values (residue scores) and generates the allosteric control information with a vertex and a corresponding evaluation value as a set.

For example, the allosteric control information calculating unit 34 arranges end point sets with high evaluation values (allosteric scores) indicating a degree of influence on a start point set designated by a user or start point sets with high evaluation values (allosteric score) indicating a degree of influence on an end point set designated by a user in the descending order of the evaluation values (allosteric scores) and generates the allosteric control information with a start point set or an end point set and an evaluation value of the start point set or the end point set as a set.

Step S880: The user terminal 5 displays the calculation result from the allosteric control information calculating unit 34 in graphics. The interface unit 31 outputs graphics information for graphic display to the user terminal 5. The graphics information is, for example, information indicating amino acid residues corresponding to vertices included in a predicted path in a three-dimensional structure of a protein. The graphics information may be customized by the interface unit 31 as post-processing before the graphics information is output to the user terminal 5. For example, the interface unit 31 changes the graphics information according to a user's settings such that the calculation result from the allosteric control information calculating unit 34 is graphically displayed in a format desired by the user.

In this way, the allosteric path prediction system 3 ends the allosteric path predicting process.

An example in which the allosteric path prediction system 3 operates will be described below as a modified example of the present embodiment.

(First modified example of fifth embodiment)

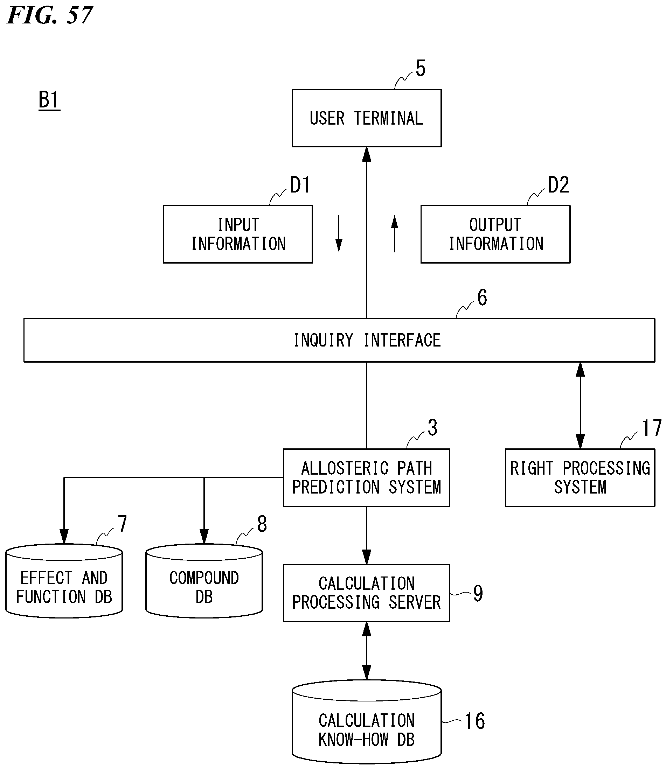

FIG. 57 is a diagram illustrating an example of a configuration of a system B1 according to a first modified example of the present embodiment. The system B1 operates as a calculation system. The system B1 includes an allosteric path prediction system 3, an inquiry interface 6, an effect and function database 7, a compound database 8, a calculation processing server 9, a calculation know-how database 16, and a right processing system 17. The allosteric path prediction system 3, the inquiry interface 6, the calculation processing server 9, and the right processing system 17 are, for example, servers. The effect and function database 7, the compound database 8, and the calculation know-how database 16 are, for example, database servers.

The allosteric path prediction system 3 acquires input information D1 from the user terminal 5 via the inquiry interface 6. The input information D1 includes, for example, information for designating one or more of a target protein, an allosteric control target site (an end point), an allosteric control site (a start point), a domain, an effect or function to be searched for, a candidate compound to work, and mutation information.

The allosteric path prediction system 3 performs the allosteric path predicting process on the basis of the input information D1. Here, the allosteric control information calculating unit 34 (which is not illustrated in FIG. 57) provided in the allosteric path prediction system 3 causes the calculation processing server 9 to perform optimization calculation. The calculation processing server 9 may use know-how information stored in the calculation know-how database 16 to perform the optimization calculation. The know-how information includes, for example, an effective Hamiltonian or information indicating effective parameter settings based on past analysis results. By using the know-how information, a user can try analysis using various conditions stored as the know-how information even when new conditions (such as a Hamiltonian or parameter settings) are not considered. That is, with the allosteric path prediction system 3, it is possible to reuse past analysis results by storing the past analysis results in the calculation know-how database 16.

The allosteric path prediction system 3 correlates an effect or function with the calculated allosteric control information with reference to the effect and function database 7. The allosteric path prediction system 3 correlates a compound with the calculated allosteric control information with reference to the compound database 8. The allosteric path prediction system 3 outputs the allosteric control information correlated with one or more of an effect, a function, and a compound to the inquiry interface 6. The allosteric path prediction system 3 may output only the allosteric control information to the inquiry interface 6.

Here, data which cannot be provided to a user may be included in data on effects or functions stored in the effect and function database 7 and data on compounds stored in the compound database 8. The data which cannot be provided to a user is, for example, data which cannot be provided to the user on the basis of security, know-how, a rights relation, or the like when the user cooperatively develops a drug with another user (such as a drug manufacturer). The right processing system 17 determines whether one or more of effects, functions, and compounds correlated with the allosteric control information output from the allosteric path prediction system 3 to the inquiry interface 6 is information which can be provided to the user.

The inquiry interface 6 outputs information determined to be provided to the user by the right processing system 17 out of the allosteric control information correlated with one or more of effects, functions, and compounds output from the allosteric path prediction system 3 as output information D2 to the user terminal 5. The output information D2 includes, for example, one or more of an allosteric control target site (end point) candidate list, an allosteric control site (start point) candidate list, an important amino acid residue list, a domain, an effect and function candidate list, and a candidate compound list. The effect and function candidate list is a list indicating candidates for effects or functions predicted (expected) on the basis of search results. The candidate compound list is a list indicating candidate for compounds predicted on the basis of the search results affecting the allosteric control.

When information for designating an effect or a function to be searched for is included in the input information D1, the inquiry interface 6 may add candidate compounds affecting the effect or the function to the output information D2. In this case, the inquiry interface 6 correlates the effect or the function correlated with the calculated allosteric control information and the compound correlated with the allosteric control information via the allosteric control information. The inquiry interface 6 adds candidate compounds corresponding to the information for designating the effect or the function to be searched for which is included in the input information D1 to the output information D2 on the basis of the correlation result.

Similarly, when information for designating a compound to be searched for is included in the input information D1, the inquiry interface 6 may add candidates for an effect or a function affected by the compound to the output information D2.

(Second modified example of fifth embodiment)

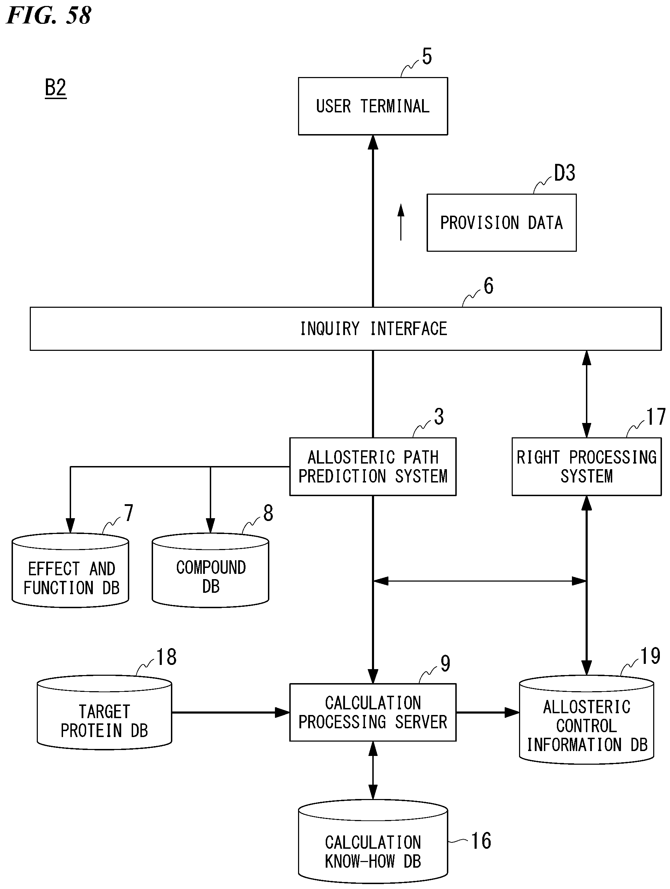

FIG. 58 is a diagram illustrating an example of a configuration of a system B2 according to a second modified example of the present embodiment. The system B2 operates as a database. The system B2 includes an allosteric path prediction system 3, an inquiry interface 6, an effect and function database 7, a compound database 8, a calculation processing server 9, a calculation know-how database 16, a right processing system 17, a target protein database 18, and an allosteric control information database 19. The system B2 according to the second modified example (FIG. 58) is different from the system B1 according to the first modified example (FIG. 57) in the target protein database 18 and the allosteric control information database 19. A description of the same functions as in the first modified example will be omitted, and differences from the first modified example will be mainly described in the second modified example.

The target protein database 18 and the allosteric control information database 19 are, for example, database servers.

The allosteric path prediction system 3 generates allosteric control information in advance. Here, the allosteric control information calculating unit 34 (which is not illustrated in FIG. 57) provided in the allosteric path prediction system 3 causes the calculation processing server 9 to perform optimization calculation. The calculation processing server 9 performs the allosteric path predicting process on the basis of three-dimensional structure information of a protein stored in the target protein database 18. The calculation processing server 9 generates allosteric control information as a result of the allosteric path predicting process.

The calculation processing server 9 stores the generated allosteric control information in the allosteric control information database 19. That is, in the system B2, a result of the allosteric path predicting process performed in advance is stored as a database.

When the allosteric path predicting process is newly performed, the calculation processing server 9 newly adds allosteric control information which is a result of the allosteric path predicting process to the allosteric control information database 19. For example, when three-dimensional structure information is newly added to the target protein database 18, the calculation processing server 9 performs an allosteric path predicting process on the basis of the added three-dimensional structure information.

The calculation processing server 9 may cause the right processing system 17 to determine whether allosteric control information is information which is releasable before the allosteric control information is newly added to the allosteric control information database 19. The calculation processing server 9 stores the allosteric control information determined to be information which is releasable by the right processing system 17 in the allosteric control information database 19. The calculation processing server 9 may discard the allosteric control information determined not to be information which is releasable by the right processing system 17 or may issue a non-releasable flag and store the allosteric control information in the allosteric control information database 19.

The calculation processing server 9 may use the allosteric control information stored in the allosteric control information database 19 as an existing result for the allosteric path predicting process.

When a request is received from the user terminal 5, the inquiry interface 6 outputs provision data D3 to the user terminal 5. The inquiry interface 6 outputs the allosteric control information stored in the allosteric control information database 19 as the provision data D3 to the user terminal 5 with reference to the allosteric control information database 19. When the inquiry interface 6 refers to the allosteric control information database 19, the right processing system 17 determines whether the allosteric control information to be referred to is releasable. The provision data D3 includes only releasable allosteric control information.

(Summaries of embodiments)

The functions of the allosteric path prediction devices and the allosteric path prediction systems according to the embodiments will be summarized below. The allosteric path prediction devices and the allosteric path prediction systems according to the embodiments have a grouping calculation function and a customization function along with a basic function of performing the allosteric path predicting process.

In the function of performing the allosteric path predicting process, an optimal path or an important amino acid residue in the path are searched for on the basis of a predetermined criterion. The predetermined criterion is minimization of energy indicated by a Hamiltonian.

In the grouping calculation function, a path set as well as an optimal path is searched for. The path set may be calculated by repeatedly performing the allosteric path predicting process on the basis of the Hamiltonian for a single path or may be calculated by performing the allosteric path predicting process on the basis of the Hamiltonian for a multiplex path.

In the grouping calculation function, a path set and a multiplex path under various conditions can be calculated according to settings of the Hamiltonian. Various conditions are obtained, for example, by designating various conditions as constraint conditions required from biological aspects in the Hamiltonian for a multiplex path.

In the grouping calculation function, various types of calculation are performed on the basis of a path set and a multiplex path set. The various types of calculation include an important amino acid residue search process of searching for a vertex with a high evaluation value (residue score) out of vertices included in a plurality of paths and a multiplex path set and a region search process of searching for a vertex with a high evaluation value (allosteric score) indicating the degree of influence on a certain vertex.

In the allosteric path prediction devices and the allosteric path prediction systems according to the embodiments, it is possible to fast perform calculation for a path set and a multiplex path set using Ising calculation, optimization calculation using extended Ising calculation, and a simulated bifurcation machine. Accordingly, a set of candidates for an end point and an end point set (allosteric control target sites) with respect to a start point and a start point set, a set of candidates for a start point set (allosteric control sites) with respect to an end point and an end point set, and the like can be comprehensively searched for. The search result can be stored as a database. Know-how on search for turned parameters or the like used in the search can be stored as a database. The search result may be combination information of an effect and function candidate list and a candidate compound list.

In the customization function, an evaluation function for evaluating a path can be changed. The evaluation function is a Hamiltonian. The customization function is realized by changing an objective function included in the Hamiltonian and changing or adding constraint conditions.

In the customization function, various criteria can be prepared according to a user's designation, and a path can be searched for. For example, a Hamiltonian for a multiplex path is changed according to a user's designation. In the customization function, an objective function can be independently designated by a user, a pass-avoided vertex set can be designated, or a pass-forced vertex set can be designated. The pass-avoided vertex set is a set of vertices not included in a path. The pass-forced vertex set is a set of vertices necessarily included in a path.

In the customization function, newly acquired biological knowledge can be reflected in the allosteric path predicting process by changing the Hamiltonian.

As described above, in the allosteric path prediction devices and the allosteric path prediction systems according to the embodiments, an evaluation function (Hamiltonian) or one or more of conditions for calculating a path set and a multiplex path set (designating a pass-avoided vertex set or designating a pass-forced vertex set) can be changed on the basis of designation from the outside. The outside is a user's operation, a file indicating instructions for customization, an allosteric path prediction device, an information processing device separate from the allosteric path prediction system, or the like.

In the aforementioned embodiments, an example in which a simulated bifurcation machine is used to calculate an Ising model in the allosteric path predicting process has been described, but the present invention is not limited thereto. An algorithm other than a simulated bifurcation machine may be used to calculate an Ising model. For example, an algorithm such as simulated annealing may be used to calculate an Ising model. As described above, the simulated bifurcation machine can be suitably used for an increase in speed and comprehensive search.

In the aforementioned embodiments, an example in which a solution to a combination optimization problem which is a QUBO is calculated on the basis of the Ising model in the allosteric path predicting process has been described above, but the present invention is not limited thereto. A solution to a combination optimization problem may be calculated on the basis of a technique other than the Ising model.

As described above, the allosteric path prediction devices according to the embodiments (the allosteric path prediction devices 1, 1a, 1b, and 1c or the allosteric path prediction system 3 according to the embodiments) include the network graph generating units 12 and 32 and the path calculating units 13, 13b, and 13c.

The network graph generating unit 12 generates a network graph which includes vertices corresponding to at least amino acid residues constituting a protein out of the amino acid residues and arbitrary binding substances bound to the protein and in which weights based on interactions between at least the amino acid residues out of the amino acid residues and the arbitrary binding substances are assigned to edges on the basis of three-dimensional structure information of the protein

The path calculating units 13, 13b, and 13c calculate a path and a multiplex path connecting the vertices on the network graph generated by the network graph generating unit 12 on the basis of an evaluation function based on the weights (a QUBO type Hamiltonian in the embodiments).

With this configuration, since the allosteric path prediction device according to the present embodiment can calculate the path and the multiplex path connecting the vertices in the network graph on the basis of the evaluation function based on weights, it is possible to predict amino acid residues contributing to allosteric control from a three-dimensional structure information of a protein.

In the related art, a prediction technique using some in-silico has been proposed as an allosteric path prediction method. The allosteric path prediction method in the related art roughly includes two methods of a molecular dynamics method (a dynamic method) and a prediction method using a network model (a static method)

In the molecular dynamics method, a structure change of a protein is ascertained through molecular dynamics simulation and an allosteric control site is predicted. On the other hand, in the prediction method using a network model, an allosteric control site is predicted in a network in which a specific structure of a protein is reflected. For example, a machine-learning prediction method from network information (Patent Document 1) and a prediction method using an information transmission model in a network (Non-Patent Documents 1 and 2) are known as the prediction method using a network model.

A motion of a molecule associated with a function of a protein may have a delay of from milliseconds to seconds from time to time. Accordingly, when molecular dynamics simulation is used, even a most advanced computer cannot calculate all from a first principle.

In prediction using a network model, prediction accuracy is not high, and there is a problem in selection of training data, adaptation (scalability) to a larger protein (such as a protein complex), and the like. Specifically, for example, in the prediction method described in Patent Document 1, a plurality of parameters such as evolutional characteristics and physical characteristics are provided, a protein is expressed using a network graph in which a protein structure is reflected, and amino acid residues important for allosteric control are calculated from the parameters in the network graph using a random forest method. However, in the prediction method described in Patent Document 1, many parameters are used, and applicability to all proteins is not clear. In the prediction method described in Patent Document 1, since the prediction accuracy depends on the quality of training data, it is thought that a prediction result for a protein not having a structure or materiality similar to a protein used to prepare the training data is lowered.

For example, in the prediction method described in Non-Patent Document 1 or Non-Patent Document 2, amino acid residues important for allosteric control are calculated by preparing a network graph in which structure information of a protein is reflected and predicting allosteric paths. However, in the prediction method described in Non-Patent Document 1 or Non-Patent Document 2, prediction accuracy is not high. In the prediction method described in Non-Patent Document 1 or Non-Patent Document 2, a solution is not likely to converge in a large-scale problem, and extendability to a large structure such as a protein complex is low.

On the other hand, in the allosteric path prediction device according to the present embodiment, since a path and a multiplex path connecting vertices in a network graph can be calculated on the basis of an evaluation function (a Hamiltonian of an Ising model) based on weights, it is possible to enhance prediction accuracy and to improve extendability to a large structure. The network graph which includes amino acid residues as vertices and in which weights based on interactions between amino acid residues are assigned to edges is a simplified model enabling practical calculation. In the allosteric path prediction device according to the present embodiment, since a plurality of paths including an optical path and suboptimal paths can be calculated on the basis of an evaluation function based on weights, it is possible to enhance prediction accuracy.

Some of the allosteric path prediction devices 1, 1a, 1b, 1c, and 1d or the allosteric path prediction system 3 according to the aforementioned embodiments, for example, the three-dimensional structure information acquiring unit 11, the network graph generating unit 12 or 32, the path calculating unit 13, 13b, 13c, or 13d, the Hamiltonian generating unit 33, the allosteric control information calculating unit 34, the evaluation unit 14, the first evaluation unit 14a, the second evaluation unit 14b, the third evaluation unit 16d, and the output unit 15, 15a, 15b, or 15d may be realized by a computer. In this case, these control functions may be realized by recording a program for realizing the control functions on a computer-readable recording medium and causing a computer system to read and execute the program recorded on the recording medium. The "computer system" mentioned herein is a computer system incorporated into the allosteric path prediction devices 1, 1a, 1b, 1c, and 1d or the allosteric path prediction system 3 and includes an operating system (OS) or hardware such as peripherals. The "computer-readable recording medium" is a portable medium such as a flexible disk, a magneto-optical disc, a ROM, or a CD-ROM or a storage device such as a hard disk incorporated into a computer system. The "computer-readable recording medium" may include a medium that dynamically holds a program for a short time such as a communication line when a program is transmitted via a network such as the Internet or a communication line such as a telephone line and a medium that holds a program for a predetermined time such as a volatile memory in a computer system serving as a server or a client in that case. The program may be a program for realizing some of the aforementioned functions or may be realize the aforementioned functions in combination with another program stored in advance in the computer system.

Some or all of the allosteric path prediction devices 1, 1a, 1b, 1c, and 1d or the allosteric path prediction system 3 may be realized as an integrated circuit such as a large scale integration (LSI) circuit. The functional blocks of the allosteric path prediction devices 1, 1a, 1b, 1c, and 1d or the allosteric path prediction system 3 may be individually formed as processors, or some or all thereof may be integrated as a processor. The integration technique is not limited to LSI, and the functional blocks may be realized by a dedicated circuit or a general-purpose processor. When integration technology replacing LSI appears as semiconductor technology develops, an integrated circuit based on the technology may be used.

While embodiments of the present invention have been described above in detail in conjunction with the drawings, a specific configuration thereof is not limited to the above description and can be subjected to various design modifications without departing from the gist of the present invention.

REFERENCE SIGNS LIST

1, 1a, 1b, 1c, 1d... Allosteric path prediction device
3... Allosteric path prediction system
12... Network graph generating unit
13, 13b, 13c, 13d... Path calculating unit

**Claims**

1.  An allosteric path prediction device comprising:

    a network graph generating unit configured to generate, on the basis of three-dimensional structure information of a protein, a network graph which includes vertices corresponding to at least amino acid residues constituting the protein out of the amino acid residues and arbitrary binding substances bound to the protein and in which weights based on interactions between at least the amino acid residues out of the amino acid residues and the arbitrary binding substances are assigned to edges; and
    a path calculating unit configured to calculate a path connecting the vertices on the network graph generated by the network graph generating unit on the basis of an evaluation function based on the weights.

2.  The allosteric path prediction device according to claim 1, wherein a start point and an end point of the path are predetermined vertices on the network graph.

3.  The allosteric path prediction device according to claim 1 or 2, wherein the path calculating unit calculates a plurality of the paths.

4.  The allosteric path prediction device according to claim 3, wherein the plurality of paths are ranked on the basis of evaluation values.

5.  The allosteric path prediction device according to claim 3, further comprising a first evaluation unit configured to evaluate the vertices on the basis of the number of times each vertex is included in the plurality of paths calculated by the path calculating unit.

6.  The allosteric path prediction device according to claim 3, further comprising a second evaluation unit,

    wherein one of a start point and an end point of each path is a predetermined vertex on the network graph and the other thereof is an evaluation target vertex which is a vertex to be evaluated by the second evaluation unit,
    wherein the path calculating unit calculates the path connecting the predetermined vertex and the evaluation target vertex for each combination of the predetermined vertex and the evaluation target vertex, and
    wherein the second evaluation unit calculates an evaluation value for each of a plurality of the evaluation target vertices on the basis of the evaluation values of the paths calculated by the path calculating unit.

7.  The allosteric path prediction device according to claim 6, further comprising a third evaluation unit configured to calculate an evaluation value for a subset of the plurality of evaluation target vertices on the basis of the evaluation value calculated for each of the evaluation target vertices by the second evaluation unit.

8.  The allosteric path prediction device according to claim 1, wherein each path includes a bifurcation or a junction.

9.  The allosteric path prediction device according to claim 1, wherein there are a plurality of start points or end points of the paths.

10. The allosteric path prediction device according to claim 1, wherein the evaluation function is a function for providing an evaluation value of a partial graph.

11. The allosteric path prediction device according to claim 1, wherein the evaluation function is a function for providing an evaluation value of a path set.

12. The allosteric path prediction device according to claim 1, wherein the evaluation function is a Hamiltonian.

13. The allosteric path prediction device according to claim 1, wherein the evaluation function is an Ising model.

14. The allosteric path prediction device according to claim 1, wherein the path calculating unit calculates the path by calculating a solution to the evaluation function on the basis of an optimization algorithm of a Hamiltonian.

15. The allosteric path prediction device according to claim 1, wherein the path calculating unit calculates the path by calculating a solution to the evaluation function using an Ising machine.

16. The allosteric path prediction device according to claim 1, wherein the path calculating unit calculates the path by calculating a solution to the evaluation function on the basis of a simulated bifurcation algorithm.

17. The allosteric path prediction device according to claim 1, further comprising a three-dimensional structure information acquiring unit configured to acquire the three-dimensional structure information from a database in which the three-dimensional structure information is stored in advance.

18. The allosteric path prediction device according to claim 1, wherein the three-dimensional structure information is calculated on the basis of three-dimensional structure simulation.

19. The allosteric path prediction device according to claim 1, wherein a prediction result of the path is stored in a database.

20. The allosteric path prediction device according to claim 1, wherein a group of one or more of an effect and function candidate list indicating candidates for an effect or a function which is predicted on the basis of a prediction result of the path and a candidate compound list indicating candidates for a compound which is predicted on the basis of the prediction result of the path when allosteric control is affected and the prediction result of the path is output.

21. The allosteric path prediction device according to claim 1, wherein the evaluation function or one or more of conditions for calculating the path are changeable on the basis of external designation.

22. An allosteric path prediction result acquisition method of acquiring calculation results of a path connecting vertices on a network graph, wherein

   a network graph includes vertices corresponding to at least amino acid residues constituting a protein out of the amino acid residues and arbitrary binding substances bound to the protein, weights based on interactions between at least the amino acid residues out of the amino acid residues and the arbitrary binding substances are assgned to edges, and is generated on the basis of three-dimensional structure information of the protein; and
   a path connecting the vertices on the network graph is calculated on the basis of an evaluation function based on the weights.

23. An allosteric path prediction method comprising:

   a network graph generating step of generating, on the basis of three-dimensional structure information of a protein, a network graph which includes vertices corresponding to at least amino acid residues constituting the protein out of the amino acid residues and arbitrary binding substances bound to the protein and in which weights based on interactions between at least the amino acid residues out of the amino acid residues and the arbitrary binding substances are assigned to edges; and
   a path calculating step of calculating a path connecting the vertices on the network graph generated in the network graph generating step on the basis of an evaluation function based on the weights.

24. A program causing a computer to perform:

a network graph generating step of generating, on the basis of three-dimensional structure information of a protein, a network graph which includes vertices corresponding to at least amino acid residues constituting the protein out of the amino acid residues and arbitrary binding substances bound to the protein and in which weights based on interactions between at least the amino acid residues out of the amino acid residues and the arbitrary binding substances are assigned to edges; and

a path calculating step of calculating a path connecting the vertices on the network graph generated in the network graph generating step on the basis of an evaluation function based on the weights.

FIG. 1

ALLOSTERIC
CONTROL SITE

T11

T1

T12

ACTIVE CENTER

## FIG. 2

FIG. 3

*FIG. 4*

$C_i = 9$, $C_i = 8$, $C_{ij} = 3$
$N_{ij} = 0.333$, $N_{ji} = 0.375$
$P_{ij} = 0.632$, $P_{ji} = 0.675$

**FIG. 5**

G2

N21

START POINT (s)

1
Gly

N22
2
Ala

P21

N27
7
Phe

N24
N26

P22
4
Ile

6
His

N28
8
Tyr

Gly
N23

5
Asp

N25

9
Phe
N29

11
Asp

10
Tyr

END POINT (e)

12
Ile

N211

N210

N212

◯ : AMINO ACID ───── : MAIN CHAIN ─ ─ ─ : INTERACTION BETWEEN SIDE CHAINS

## FIG. 6

1

ALLOSTERIC PATH PREDICTION DEVICE

10

CONTROL UNIT

THREE-DIMENSIONAL
STRUCTURE INFORMATION
ACQUIRING UNIT ─11

NETWORK GRAPH
GENERATING UNIT ─12

13

PATH CALCULATING UNIT

HAMILTONIAN
GENERATING UNIT ─130

ISING CALCULATION
UNIT ─131

EVALUATION
CALCULATING UNIT ─14

OUTPUT UNIT ─15

20

STORAGE UNIT

ISING MODEL
INFORMATION ─21

*FIG. 7*

START

↓

ACQUIRE THREE-DIMENSIONAL STRUCTURE INFORMATION ──S10

↓

GENERATE NETWORK GRAPH ──S20

↓

PERFORM PREDICTION BASED ON ISING MODEL ──S30

↓

EVALUATE AND EXTRACT PATH ──S40

↓

OUTPUT PREDICTION RESULT ──S50

↓

END

*FIG. 8*

START

↓

GENERATE VERTICES OF NETWORK GRAPH ──S110

↓

GENERATE NETWORK GRAPH OF INTERACTION ──S120

↓

GENERATE NETWORK GRAPH OF SIGNAL TRANSMISSION ──S130

↓

END

*FIG. 9*

```
            ┌─────────────┐
            │    START    │
            └──────┬──────┘
                   │
                   ▼
   ┌────────────────────────────────────┐
   │  ACQUIRE SITE DESIGNATION INFORMATION │──── S210
   └────────────────────┬───────────────┘
                   │
                   ▼
   ┌────────────────────────────────────┐
   │   DETERMINE START POINT AND END POINT │──── S220
   └────────────────────┬───────────────┘
                   │
                   ▼
   ┌────────────────────────────────────┐
   │       GENERATE HAMILTONIAN (QUBO)      │
   │  ON THE BASIS OF DESIGNED ISING MODEL  │──── S230
   └────────────────────┬───────────────┘
                   │
                   ▼
   ┌────────────────────────────────────┐
   │  PERFORM ISING CALCULATION BASED ON SBM │──── S240
   └────────────────────┬───────────────┘
                   │
                   ▼
            ┌─────────────┐
            │     END     │
            └─────────────┘
```

**FIG. 10**

s:DESIGNATED START POINT

pij

vj

e:DESIGNATED END POINT

FIG. 11

$$H = \underbrace{-A \sum_{i=1}^{V} \sum_{j=1}^{V} (\log p_{ij}) q_{ij}}_{61} + \underbrace{B \left( \sum_{i \in V_s^{start}} q_{si} - 1 \right)^2}_{62} + \underbrace{B \left( \sum_{i \in V_e^{end}} q_{ie} - 1 \right)^2}_{63}$$

$$+ \underbrace{C \sum_{i \in V_s^{end}} q_{is}}_{64} + \underbrace{C \sum_{i \in V_e^{start}} q_{ei}}_{65} + \underbrace{D \sum_{i=1}^{V} \sum_{j \in V_i^{start}} \sum_{\substack{J \neq j \\ J \in V_i^{start}}} q_{ij} q_{iJ}}_{66}$$

$$+ \underbrace{D \sum_{i=1}^{V} \sum_{j \in V_i^{end}} \sum_{\substack{J \neq j \\ J \in V_i^{end}}} q_{ji} q_{Ji}}_{67} + \underbrace{E \sum_{i=1}^{V} \left( \sum_{j \in V_i^{end}} q_{ji} - \sum_{J \in V_i^{start}} q_{iJ} \right)^2}_{68}$$

*FIG. 12*

| RANKING | PATH | EVALUATION VALUE |
|---|---|---|
| 1 | A14-A15-A18-A17-A32-A36 | 0. 759112 |
| 2 | A14-A15-A17-A32-A36 | 0. 779151 |
| 3 | A14-A16-A8-A58-A37-A36 | 0. 80354 |
| 4 | A14-A16-A8-A58-A59-A37-A36 | 0. 831245 |
| 5 | A14-A81-A114-A79-A8-A58-A37-A36 | 0. 887067 |
| 6 | A14-A81-A114-A79-A8-A58-A59-A37-A36 | 0. 914771 |
| 7 | A14-A15-A19-A146-A18-A17-A32-A36 | 0. 920042 |
| 8 | A14-A16-A17-A32-A36 | 0. 962545 |
| 9 | A14-A15-A18-A146-A22-A27-A25-A21-A17-A32-A36 | 0. 963685 |
| 10 | A14-A16-A58-A37-A36 | 0. 978772 |

## FIG. 13

（A）

P131

START POINT (s)

END POINT (e)

（B）

START POINT (s)

P132

END POINT (e)

（C）

START POINT (s)

P133

END POINT (e)

（D）

P134

START POINT (s)

END POINT (e)

◯ : AMINO ACID    ━━━ : MAIN CHAIN    ▬ ▬ ▬ : INTERACTION BETWEEN SIDE CHAINS

## *FIG. 14*

$$n = 4, w_{(k)} = 1$$

$$RS_{2,10}^{1} : 2 \qquad RS_{2,10}^{7} : 0$$

$$RS_{2,10}^{3} : 2 \qquad RS_{2,10}^{8} : 0$$

$$RS_{2,10}^{4} : 2 \qquad RS_{2,10}^{9} : 0$$

$$RS_{2,10}^{5} : 3 \qquad RS_{2,10}^{11} : 4$$

$$RS_{2,10}^{6} : 1 \qquad RS_{2,10}^{12} : 1$$

ELEVENTH AMINO ACID
RESIDUE IS IMPORTANT
IN ALLOSTERIC CONTROL

## *FIG. 15*

1a

ALLOSTERIC PATH PREDICTION DEVICE

10a

CONTROL UNIT

THREE-DIMENSIONAL STRUCTURE INFORMATION ACQUIRING UNIT ─11

NETWORK GRAPH GENERATING UNIT ─12

13

PATH CALCULATING UNIT

HAMILTONIAN GENERATING UNIT ─130

ISING CALCULATION UNIT ─131

FIRST EVALUATION UNIT ─14a

OUTPUT UNIT ─15a

20

STORAGE UNIT

ISING MODEL INFORMATION ─21

*FIG. 16*

```
                    ┌─────────────┐
                    │    START    │
                    └─────────────┘
                           │
                           ▼
    ┌─────────────────────────────────────────┐
    │  ACQUIRE THREE-DIMENSIONAL STRUCTURE     │── S310
    │               INFORMATION                │
    └─────────────────────────────────────────┘
                           │
                           ▼
    ┌─────────────────────────────────────────┐
    │         GENERATE NETWORK GRAPH           │── S320
    └─────────────────────────────────────────┘
                           │
                           ▼
    ┌─────────────────────────────────────────┐
    │  PERFORM PREDICTION BASED ON ISING MODEL │── S330
    └─────────────────────────────────────────┘
                           │
                           ▼
    ┌─────────────────────────────────────────┐
    │         EVALUATE AND EXTRACT PATH        │── S340
    └─────────────────────────────────────────┘
                           │
                           ▼
    ┌─────────────────────────────────────────┐
    │  PERFORM IMPORTANT AMINO ACID RESIDUE    │── S350
    │              SEARCH PROCESS              │
    └─────────────────────────────────────────┘
                           │
                           ▼
    ┌─────────────────────────────────────────┐
    │           OUTPUT SEARCH RESULT           │── S360
    └─────────────────────────────────────────┘
                           │
                           ▼
                    ┌─────────────┐
                    │     END     │
                    └─────────────┘
```

*FIG. 17*

```
                    ┌─────────────┐
                    │    START    │
                    └─────────────┘
                           │
                           ▼
    ┌─────────────────────────────────────────┐
    │         CALCULATE RESIDUE SCORE          │── S410
    └─────────────────────────────────────────┘
                           │
                           ▼
    ┌─────────────────────────────────────────┐
    │  DETERMINE IMPORTANT AMINO ACID RESIDUE  │── S420
    └─────────────────────────────────────────┘
                           │
                           ▼
                    ┌─────────────┐
                    │     END     │
                    └─────────────┘
```

**FIG. 18**

**FIG. 19**

| No. | Res. | RS |
|-----|------|------|
| 1 | <u>A97</u> | 1.00 |
| 2 | <u>A96</u> | 0.96 |
| 3 | <u>A10</u> | 0.96 |
| 4 | <u>A16</u> | 0.96 |
| 5 | A101 | 0.28 |
| 6 | A100 | 0.28 |
| 7 | <u>A17</u> | 0.17 |
| 8 | A98 | 0.12 |
| 9 | A94 | 0.12 |
| 10 | A14 | 0.09 |

*FIG. 20*

*FIG. 21*

| No. | Res. | RS* |
|-----|------|------|
| 1 | A15 | 1.00 |
| 2 | A17 | 0.80 |
| 3 | A18 | 0.60 |
| 4 | A37 | 0.60 |
| 5 | A32 | 0.40 |
| 6 | A58 | 0.40 |
| 7 | A59 | 0.40 |
| 8 | A16 | 0.40 |
| 9 | A57 | 0.40 |
| 10 | A8 | 0.20 |

*FIG. 22*

*FIG. 23*

| No. | Res. | RS* |
|-----|------|------|
| **1** | **A12** | **0.51** |
| 2 | A34 | 0.51 |
| 3 | A15 | 0.49 |
| 4 | A37 | 0.49 |
| 5 | A33 | 0.31 |
| 6 | A13 | 0.30 |
| 7 | A16 | 0.30 |
| 8 | A8 | 0.30 |
| 9 | A35 | 0.29 |
| 10 | A58 | 0.29 |

## FIG. 24

(A)

APS=0.60

(B)

APS=0.55

(C)

APS=0.60

(D)

APS=0.55

◯ : AMINO ACID ━━━ : MAIN CHAIN ━ ━ ━ : INTERACTION BETWEEN SIDE CHAINS

*FIG. 25*

| START POINT | APS | I | corrected APS | AS | standardized AS |
|---|---|---|---|---|---|
| 1 | 0.60 | 4 | −0.13 | −0.13 | 0.67 |
| 2 | 0.55 | 5 | −0.12 | −0.12 | 0.83 |
| 3 | 0.60 | 3 | −0.17 | −0.17 | −0.16 |
| 7 | 0.50 | 3 | −0.23 | −0.23 | −1.35 |

*FIG. 26*

1b

ALLOSTERIC PATH PREDICTION DEVICE

10b

CONTROL UNIT

THREE-DIMENSIONAL STRUCTURE INFORMATION ACQUIRING UNIT — 11

NETWORK GRAPH GENERATING UNIT — 12

13b

PATH CALCULATING UNIT

HAMILTONIAN GENERATING UNIT — 130

ISING CALCULATION UNIT — 131

SECOND EVALUATION UNIT — 14b

OUTPUT UNIT — 15b

20

STORAGE UNIT

ISING MODEL INFORMATION — 21

*FIG. 27*

START

↓

| ACQUIRE THREE-DIMENSIONAL STRUCTURE INFORMATION | ⌐ S510 |

↓

| GENERATE NETWORK GRAPH | ⌐ S520 |

↓

| PERFORM PREDICTION BASED ON ISING MODEL | ⌐ S530 |

↓

| EVALUATE AND EXTRACT PATH | ⌐ S540 |

↓

| PERFORM REGION SEARCH PROCESS | ⌐ S550 |

↓

| OUTPUT SEARCH RESULT | ⌐ S560 |

↓

END

*FIG. 28*

START

↓

ACQUIRE SEARCH REGION DESIGNATION INFORMATION — S610

↓

FOR EACH EVALUATION TARGET VERTEX — S620

↓

DETERMINE START POINT AND END POINT — S630

↓

GENERATE HAMILTONIAN (QUBO) ON
THE BASIS OF DESIGNED ISING MODEL — S640

↓

PERFORM ISING CALCULATION BASED ON SBM — S650

↓

END EACH EVALUATION TARGET VERTEX — S660

↓

END

*FIG. 29*

START

↓

CALCULATE EVALUATION VALUE FOR
EACH EVALUATION TARGET VERTEX — S710

↓

EVALUATE EVALUATION
TARGET VERTEX — S720

↓

END

*FIG. 30*

EP 4 546 346 A1

ALLOSTERIC SCORE

RAF/RBD
RAF/CRD
GDP

I36

KRAS AMINO ACID NUMBER

*FIG. 31*

*FIG. 32*

KRAS 1-67

KRAS 114-169

## FIG. 33

（A）

AS＝1.2

S

N22

g

N210

（B）

AS＝0.5

S

N23

g

N210

（C）

AS＝2.5

N21

S

g

N210

LIKELY START POINT, PATH

（D）

AS＝−0.5

N27

S

g

N210

*FIG. 34*

LIKELY START POINT, PATH

*FIG. 35*

1d

ALLOSTERIC PATH PREDICTION DEVICE

10d

CONTROL UNIT

THREE-DIMENSIONAL STRUCTURE INFORMATION ACQUIRING UNIT — 11

NETWORK GRAPH GENERATING UNIT — 12

13

PATH CALCULATING UNIT

HAMILTONIAN GENERATING UNIT — 130

ISING CALCULATION UNIT — 131

SECOND EVALUATION UNIT — 14b

140d

RESIDUE SET SEARCH UNIT

SECOND HAMILTONIAN GENERATING UNIT — 141d

SECOND ISING CALCULATION UNIT — 142d

THIRD EVALUATION UNIT — 16d

OUTPUT UNIT — 15d

20

STORAGE UNIT

ISING MODEL INFORMATION — 21

RESIDUE SET SEARCH AND ISING INFORMATION — 22d

*FIG. 36*

```
                    ┌──────────┐
                    │  START   │
                    └────┬─────┘
                         ↓
         ┌───────────────────────────────────┐
         │ ACQUIRE THREE-DIMENSIONAL STRUCTURE│ ～S1510
         │            INFORMATION             │
         └───────────────┬───────────────────┘
                         ↓
         ┌───────────────────────────────────┐
         │       GENERATE NETWORK GRAPH       │ ～S1520
         └───────────────┬───────────────────┘
                         ↓
         ┌───────────────────────────────────┐
         │       PERFORM PREDICTION BASED ON  │ ～S1530
         │            ISING MODEL             │
         └───────────────┬───────────────────┘
                         ↓
         ┌───────────────────────────────────┐
         │       EVALUATE AND EXTRACT PATH    │ ～S1540
         └───────────────┬───────────────────┘
                         ↓
         ┌───────────────────────────────────┐
         │   PERFORM RESIDUE SET SEARCH PROCESS│ ～S1550
         └───────────────┬───────────────────┘
                         ↓
         ┌───────────────────────────────────┐
         │        OUTPUT SEARCH RESULT        │ ～S1560
         └───────────────┬───────────────────┘
                         ↓
                    ┌──────────┐
                    │   END    │
                    └──────────┘
```

## FIG. 37

START

CALCULATE EVALUATION VALUE
(ALLOSTERIC SCORE) FOR EACH
EVALUATION VERTEX (RESIDUE) —— S1710

CALCULATE RESIDUE SET SCORE OF
ACTIVATION CENTER FOR EACH
EVALUATION VERTEX (RESIDUE) —— S1720

CALCULATE DISTANCE BETWEEN RESIDUES
ON THE BASIS OF THREE-DIMENSIONAL
STRUCTURE INFORMATION —— S1730

PERFORM RESIDUE SET SEARCH AND
ISING CALCULATION PROCESS —— S1740

EVALUATE RESIDUE SET —— S1750

END

## FIG. 38

START

GENERATE HAMILTONIAN (QUBO) ON THE
BASIS OF DESIGNED ISING MODEL —— S1810

PERFORM ISING CALCULATION BASED ON SBM —— S1820

END

*FIG. 39*

standardized $AS_{1,e_1}=0.3$

$AS_{1,e}=0.4$

standardized $AS_{1,e_2}=-0.1$

standardized $AS_{1,e_3}=0.2$

*FIG. 40*

*FIG. 41*

FIG. 42

KRAS AMINO
ACID NUMBER

**FIG. 43**

**FIG. 44**

# FIG. 45

**FIG. 46**

G2

N21

1
Gly

START POINT (s)

N27

7
Phe

N22

2
Ala

N24

N26

4
e

6
His

N28

8
Tyr

Gly

N23

9
Phe

N29

P32

P3

Asp

N25

P31

11
Asp

10
Tyr

1
Ile

N212

N211

END POINT (e)

N210

◯ : AMINO ACID ━━━ : MAIN CHAIN ━ ━ ━ : INTERACTION BETWEEN SIDE CHAINS

## FIG. 47

1c

ALLOSTERIC PATH PREDICTION DEVICE

10c

CONTROL UNIT

| THREE-DIMENSIONAL STRUCTURE INFORMATION ACQUIRING UNIT | 11 |

| NETWORK GRAPH GENERATING UNIT | 12 |

13c

PATH CALCULATING UNIT

| HAMILTONIAN GENERATING UNIT | 130c |

| ISING CALCULATION UNIT | 131 |

| FIRST EVALUATION UNIT | 14a |

| OUTPUT UNIT | 15a |

20

STORAGE UNIT

| ISING MODEL INFORMATION | 21c |

## FIG. 48

$$H = \underbrace{-A \sum_{i=1}^{V} \sum_{j=1}^{V} p_{ij} q_{ij}}_{71} + \underbrace{B \sum_{i=1}^{V} \left( \sum_{q \in q_i^{status}} q - 1 \right)^2}_{72} + \underbrace{C \left( \sum_{q \in q^{start}} q - s \right)^2 + C \left( \sum_{q \in q^{end}} q - t \right)^2 + C \left( \sum_{q \in q^{pass}} q - \alpha |V| \right)^2}_{73}$$

$$+ \underbrace{D \sum_{\substack{i=1 \\ j \neq i}}^{V} \sum_{j=1}^{V} \{ dist(V_i, V_j) - dist\_ave \} \left( q_i^{start} q_j^{start} + q_i^{end} q_j^{end} + q_i^{pass} q_j^{pass} \right)}_{74} - \underbrace{E \sum_{i=1}^{V} \sum_{j \in V_i^{end}} \sum_{k \in V_i^{start}} q_{ji} q_{ik}}_{75}$$

$$+ \underbrace{F \sum_{i=1}^{V} \sum_{j \in V_i^{end}} q_{ji} q_i^{start}}_{76} + \underbrace{F \sum_{i=1}^{V} \sum_{j \in V_i^{start}} q_{ij} q_i^{end}}_{77} + \underbrace{F \sum_{i=1}^{V} \sum_{j \in V_i^{end}} q_{ji} q_i^{non-pass}}_{78} + \underbrace{F \sum_{i=1}^{V} \sum_{j \in V_i^{start}} q_{ij} q_i^{non-pass}}_{79}$$

## FIG. 49

|  | SINGLE PATH (10 HIGH-RANKED PATHS) | MULTIPLEX PATH |
|---|---|---|
| START POINT | A14 | A14 |
| END POINT | A36 | A36 |
| PASS POINT | A7, **A8**, **A15**, **A16**, **A17**, **A18**, **A19**, A32, A33, A36, A37, A38, A56, **A57**, **A58**, **A59** | **A8,** A9, A10, A11, A12, **A15**, **A16**, **A17**, **A18**, **A19**, A20, **A57**, **A58**, **A59**, A81, A116, A117 |

## FIG. 50

*FIG. 51*

KRAS AMINO ACID
NUMBER

*FIG. 52*

*FIG. 53*

5
USER TERMINAL

41
THREE-DIMENSIONAL
STRUCTURE INFORMATION

42
ALLOSTERIC CONTROL
INFORMATION

3
ALLOSTERIC PATH PREDICTION SYSTEM

31
INTERFACE UNIT

32
NETWORK GRAPH
GENERATING UNIT

33
HAMILTONIAN
GENERATING UNIT

34
ALLOSTERIC CONTROL
INFORMATION
CALCULATING UNIT

## FIG. 54

```
           ┌─────────────┐
           │    START    │
           └──────┬──────┘
                  ▼
┌────────────────────────────────────────┐
│   INPUT TARGET PROTEIN INFORMATION      │──S810
└────────────────────┬───────────────────┘
                     ▼
┌────────────────────────────────────────┐
│  PERFORM NETWORK GRAPH GENERATING PROCESS │──S820
└────────────────────┬───────────────────┘
                     ▼
┌────────────────────────────────────────┐
│      ADD SEARCH CONDITIONS AND          │──S830
│     SET SEARCH-EXCLUDED REGION          │
└────────────────────┬───────────────────┘
                     ▼
┌────────────────────────────────────────┐
│          SET HAMILTONIAN                │──S840
└────────────────────┬───────────────────┘
                     ▼
┌────────────────────────────────────────┐
│   SET CALCULATION PARAMETER LIST AND    │──S850
│       PERFORM AUTOMATIC TUNING          │
└────────────────────┬───────────────────┘
                     ▼
┌────────────────────────────────────────┐
│      EXTRACT IMPORTANT REGION LIST      │──S860
└────────────────────┬───────────────────┘
                     ▼
┌────────────────────────────────────────┐
│   OUTPUT ALLOSTERIC CONTROL INFORMATION │──S870
└────────────────────┬───────────────────┘
                     ▼
┌────────────────────────────────────────┐
│      DISPLAY RESULT IN GRAPHICS         │──S880
└────────────────────┬───────────────────┘
                     ▼
           ┌─────────────┐
           │     END     │
           └─────────────┘
```

*FIG. 55*

```
        START
          |
          v
       S910
    ◇ STANDARD GRAPH? ◇ ──NO──────┐
          |                        |
         YES              S920     v
          |            ┌──────────────────────┐
          |            │ DESIGNATE EXPRESSION │
          |            │ GENERATING METHOD    │
          |            └──────────────────────┘
          |                        |
          v<───────────────────────┘
    ┌──────────────────────┐
    │ GENERATE AND CALCULATE│  S930
    │ NETWORK EXPRESSION    │
    └──────────────────────┘
          |
          v
        END
```

*FIG. 56*

```
        START
          |
          v
       S1010
    ◇ STANDARD QUBO? ◇ ──NO──────┐
          |                      S1020
         YES                      v
          |            ┌──────────────────────┐
          |            │ ADD INFORMATION OF   │
          |            │ OBJECTIVE FUNCTION   │
          |            └──────────────────────┘
          |                        |
          v<───────────────────────┘
       S1030
    ◇ STANDARD
      CONSTRAINT EXPRESSION ◇ ──NO──────┐
          ?                            S1040
         YES                            v
          |            ┌──────────────────────┐
          |            │ ADD INFORMATION OF   │
          |            │ CONSTRAINT           │
          |            └──────────────────────┘
          |                        |
          v<───────────────────────┘
    ┌──────────────────────┐
    │ SET HAMILTONIAN      │  S1050
    └──────────────────────┘
          |
          v
        END
```

## FIG. 57

<u>B1</u>

*FIG. 58*

B2

## INTERNATIONAL SEARCH REPORT

| | International application No. |
| --- | --- |
| | **PCT/JP2023/023405** |

| A. | CLASSIFICATION OF SUBJECT MATTER |
| --- | --- |

*G16B 15/00*(2019.01)i; *G06N 99/00*(2019.01)i
FI:  G16B15/00; G06N99/00 180

According to International Patent Classification (IPC) or to both national classification and IPC

| B. | FIELDS SEARCHED |
| --- | --- |

Minimum documentation searched (classification system followed by classification symbols)

  G06Q10/00-99/00; G16B5/00-99/00; G16C10/00-99/00; G06N99/00

Documentation searched other than minimum documentation to the extent that such documents are included in the fields searched

  Published examined utility model applications of Japan 1922-1996
  Published unexamined utility model applications of Japan 1971-2023
  Registered utility model specifications of Japan 1996-2023
  Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | PARK, Keun-Wan. KIM, Dong-Sup. Modeling allosteric signal propagation using protein structure networks. BMC Bioinformatics [online]. 15 February 2011, pp. 1-9, [retrieval date 19 July 2023], internet: <URL: https://bmcbioinformatics.biomedcentral.com/articles/10.1186/1471-2105-12-S1-S23>, <DOI:10.1186/1471-2105-12-S1-S23>, ISSN:1471-2105, pp. 1-3<br>  in particular, p. 1, "Introduction", pp. 2-3, "Network representation of protein structure", p. 3, "Average signal traffic of all pairwise signal transmissions" | 1-6, 8-10, 17-19, 21-24 |
| A | entire text, all drawings | 7, 11-16, 20 |
| A | DOKHOLYAN, Nikolay V. Controlling Allosteric Networks in Proteins. CHEMICAL REVIEWS [online]. 19 February 2016, pp. 6463-6487, [retrieval date 12 July 2023], internet <URL: https://pubs.acs.org/doi/full/10.1021/acs.chemrev.5b00544>, <DOI:10.1021/acs.chemrev.5b00544>, p. 6468<br>  entire text, all drawings | 1-24 |
| A | JP 2021-082165 A (FUJITSU LTD) 27 May 2021 (2021-05-27)<br>  entire text, all drawings | 1-24 |

☐ Further documents are listed in the continuation of Box C.  ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **14 July 2023** | **08 August 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/023405**

| Patent document cited in search report | | | Publication date (day/month/year) | Patent family member(s) | | | Publication date (day/month/year) |
|---|---|---|---|---|---|---|---|
| JP | 2021-082165 | A | 27 May 2021 | US | 2021/0158891 | A1 | |
| | | | | entire text, all drawings | | | |
| | | | | EP | 3826022 | A1 | |
| | | | | CN | 112837763 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022102155 A

- CN 108830043

**Non-patent literature cited in the description**

- *Biophysical Journal*, 09 February 2007, vol. 92, 3052-3062
- *NATURE COMMUNICATIONS*, 31 July 2020, vol. 11, 3862

- *Scientific Reports*, 22 February 2016, vol. 6, 21686